(19) **Europäisches Patentamt / European Patent Office / Office européen des brevets**

(11) **EP 4 067 373 A1**

(12) **EUROPEAN PATENT APPLICATION**
published in accordance with Art. 153(4) EPC

(43) Date of publication:
**05.10.2022 Bulletin 2022/40**

(21) Application number: **20893956.1**

(22) Date of filing: **27.11.2020**

(51) International Patent Classification (IPC):
**C07K 14/47** *(2006.01)*  **C07K 1/14** *(2006.01)*
**C12P 21/06** *(2006.01)*  **C12N 9/10** *(2006.01)*

(86) International application number:
**PCT/KR2020/017029**

(87) International publication number:
**WO 2021/107660 (03.06.2021 Gazette 2021/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.11.2019   KR 20190154945**

(71) Applicant: **Onegene Biotechnology Inc.**
**Suwon-si Gyeonggi-do 16229 (KR)**

(72) Inventors:
• **PARK, Sungjin**
**Seongnam-si Gyeonggi-do 13540 (KR)**
• **IM, Daeseong**
**Yongin-si Gyeonggi-do 17084 (KR)**
• **KIM, Ryuryun**
**Suwon-si Gyeonggi-do 16508 (KR)**
• **KIM, Minsum**
**Hwaseong-si Gyeonggi-do 18488 (KR)**
• **CHOI, Jaeyoung**
**Suwon-si Gyeonggi-do 16407 (KR)**

(74) Representative: **Callaghan, Dayle Anne et al**
**Kilburn & Strode LLP**
**Lacon London**
**84 Theobalds Road**
**London WC1X 8NL (GB)**

(54) **MULTIFUNCTIONAL MULTISPECIFIC MULTIMERIC BIOMOLECULE POLYMER HAVING PROLONGED IN-VIVO DURATION**

(57) The present invention provides a multifunctional multispecific multimeric biomolecule polymer which is formed by obtaining a biomolecule, to which a ubiquitin C-terminal tag is bound, by recombinantly expressing the biomolecule from a host cell, and polyubiquitinating, in vitro, the biomolecule along with a substrate, and proteins E1 (activation enzyme), E2 (conjugation enzyme) and E3 (ligase) which are involved in ubiquitination, and thus having the biomolecule bind to a polyubiquitin scaffold which is formed by covalently bonding two or more ubiquitins. The biomolecule of the present invention may be one or more selected from the group consisting of a protein, peptide, polypeptide, antibody, antibody fragment, DNA and RNA, and, for example, by using heterologous proteins, modularized functionality may be imparted to the multifunctional multispecific biomolecule polymer. In addition, according to the present invention, the multifunctional multispecific multimeric biomolecule polymer is provided in a form that is bound to a molecule capable of increasing the in vivo duration, and thus may be used for producing drugs requiring the increased in vivo duration of efficacy.

EP 4 067 373 A1

## Description

## Technical Field

The present invention relates to a method for preparing a biomolecule including a protein into a polymer in a multimeric form. Specifically, the present invention relates to a method for preparing a biomolecule recombinantly expressed from a host cell into a multifunctional multispecific biomolecule polymer having an increased in vivo duration using a ubiquitination system.

## Background Art

Preparing biomolecules and/or small molecule chemical compounds including proteins, peptides, polypeptides, antibodies, DNA and RNA in a multimeric form has various advantages. For example, the physicochemical properties of protein such as solubility, gelation, thermal stability and pH stability can be improved by linking two or more homogeneous or heterologous proteins using a fusion or a cross linker (or a cross-linking agent). For example, CLEA (cross-linked enzyme aggregate), laccase formed by multiple linking through a cross linker showed more enhanced stability and performance during starch oxidation, and CLEA of another enzyme, nitrile hydratase showed an excellent increase in activity in the conversion of acrylonitrile to acrylamide, and did not lose activity during 36 recycles.

In addition, many proteins form complexes in cells to perform complex functions, which are known to be due to the proximity effect of proteins. For example, the cellulase (Novozymes Cellic® CTec3), which is produced by preparing enzymes necessary for lignocellulose degradation, such as, cellulase, beta glucosidase (β-glucosidase), hemicellulase and the like in the form of a complex mixture using a scaffold, is known to exhibit a 3.5-fold or more increased effect in the degradation of lignocellulose. In addition, such a protein in a multimeric form exhibits a channeling effect. That is, if enzymes involved in a coupled reaction are present adjacent to each other, the transfer of the intermediate is efficient and the efficiency of the entire reaction is greatly increased. In addition, it is proposed to be desirable for an increase in its efficiency to use a homogeneous or heterologous protein in a multimeric form when analyzing any substance using a protein immobilized on a bead or a substrate, or separating and/or purifying a substance to be detected.

As described above, although the protein in a multimeric form provides various advantages in industrial and medical applications, it has been known that it is difficult to fabricate a protein having such a structure. For example, there is a method of developing and producing a multimeric protein as a new fusion enzyme by designing in-frame at the genetic stage. However, since a new protein must be designed and produced, it takes a long time to develop it and it is difficult to fuse two or more enzymes in reality. In addition, in the case of a method of fabricating a protein multimer construct (CLEA) using a chemical cross linker, the activity may be inhibited because a chemical bond does not occur at a specific site but can occur anywhere on the protein surface. Proteins that form a multimer construct must be capable of being prepared through synthesis or microbial expression, and the active sites of these proteins must not be disturbed.

A method of using ubiquitin has been proposed as a method for separating and/or purifying a protein of interest. It is the method in which first, a gene encoding a protein bound to ubiquitin is expressed in prokaryotic cells to prepare a fusion protein linked to ubiquitin, and then treated with ubiquitin cleaving enzyme to effectively separate and purify only the protein of interest from the ubiquitin fusion protein. U.S. Patent Application No. 10/504,785 relates to the expression of a recombinant gene and the purification of the expressed protein, and it describes that the fusion protein is prepared in which the nucleotide encoding the C-terminal domain of the ubiquitin-like protein (Ubl) is operatively bound to the nucleotide encoding the protein of interest, and it is expressed in a host cell. Korean Patent Application No. 10-2005-0050824 describes the use of ubiquitin as a fusion partner in expressing a recombinant protein. In addition, Korean Patent Application No. 10-2015-0120852 relates to the use of an ubiquitin column for purifying a protein, and describes that a polyubiquitin chain is loaded on the column, and the protein is purified using *in vitro* ubiquitination including E2. In addition, U.S. Patent Application No. 12/249,334 is to solve the problem of water solubility and folding, which is a problem in preparing by expressing a recombinant protein, and describes the use of SUMO having a cleavage site recognized by Ulp1 protease (Ubl-specific protease 1) for facilitating expression, separation and purification of the recombinant protein, and for increasing the activity of the protein. However, these methods only describe the use of ubiquitin for protein expression, and do not describe or suggest the production of a protein in a multimeric form, and since the protein to be separated and purified randomly binds to ubiquitin, these methods still have a limit to separation or analysis efficiency.

On the other hand, biomolecules such as proteins or peptides or recombinantly produced proteins or peptides are unstable molecules that exhibit a short serum half-life. In particular, these proteins or peptides are very unstable when prepared in aqueous solutions for diagnostic or therapeutic purposes. In addition, such protein or peptide drugs are disadvantageous because of their short serum half-life in vivo and must be administered at a high frequency or at a higher dose. However, frequent administration of the drug causes various side effects and causes discomfort to the patient. For example, there are many known problems that occur in patients requiring frequent administration of drugs, for example, diabetic patients or patients suffering from multiple sclerosis. Various methods for increasing the *in vivo* stability or half-life of such biomolecules have been studied. As an example, a component capable of increasing the half-life is covalently attached to a biomolecule such as a protein or peptide. For example, it is well known that attaching polymers such as polyethylene glycol or PEG to polypeptides can increase the serum half-life of these peptides.

Accordingly, the present inventors have made ceaseless efforts to develop a method for preparing a multifunctional multispecific biomolecule polymer having a high degree of integration and an increased in vivo duration or half-life without inhibiting the activity of the protein. As a result, a biomolecule bound to ubiquitin was recombinantly expressed from a host cell and was reacted *in vitro* with an enzyme related to ubiquitination to form a multifunctional multimeric biomolecule polymer bound to a polyubiquitin scaffold. Based on the above, the present inventors completed the present invention.

**Prior Art Document**

**Patent Document**

(Patent Document 1) Korean Patent Application No. 10-2005-0050824
(Patent Document 2) Korean Patent Application No. 10-2015-0120852
(Patent Document 3) U.S. Patent Application No. 12/249,334

**Detailed Description of the Invention**

**Technical Problem**

As described above, an object of the present invention is to provide a multifunctional multispecific biomolecule having an increased in vivo duration by binding a target biomolecule to a polyubiquitin scaffold.

Another object of the present invention is to provide a method for preparing a multifunctional multispecific biomolecule having an increased in vivo duration by binding a target biomolecule to a polyubiquitin scaffold.

Another object of the present invention is to provide a pharmaceutical composition comprising the multifunctional multispecific biomolecule.

Another object of the present invention is to provide a method for preparing a pharmaceutical composition comprising the multifunctional multispecific biomolecule.

**Solution to Problem**

In order to achieve the above objects, the present invention provides a multifunctional multispecific multimeric biomolecule polymer that is composed of a polyubiquitin scaffold which is formed by covalently bonding two or more ubiquitins, and 2 to 10 biomolecules comprising binding moieties, each specific for different binding sites, wherein the biomolecule comprises active sites that specifically bind to other biomolecules, small molecule chemical compounds or nanoparticles, and is directly bound to the N-terminus, the C-terminus, or both the N-terminus and the C-terminus of the ubiquitin or is bound by a linker; and a carrier that prolongs the in vivo stability and/or duration of the biomolecule is directly bound to the N-terminus or the C-terminus of the ubiquitin or is bound by a linker.

In one embodiment related thereto, the linker may be a combination of 1 to 30 repeats of GGGGS or EAAAK, but is not limited thereto. In another embodiment related thereto, the biomolecule bound to the N-terminus of the ubiquitin may be the distal end of the multimeric biomolecule polymer, and the biomolecule bound to the C-terminus, the N-terminus, or both the C-terminus and the N-terminus of the ubiquitin may be the proximal end of the multimeric biomolecule polymer.

As used herein, "polymer" refers to a group of monomers of a series of biomolecules linked together. The polymer may be linear or branched (branch form). When the polymer is branched, each polymer chain may be referred to as a "polymer arm." The end of the polymer arm linked to the initiator moiety is the proximal end, and the growing-chain end of the polymer arm is the distal end.

As used herein, "linker" refers to a chemical moiety that connects two groups together. The linker may be degradable or non-degradable. The degradable linker may be a hydrolysable, enzymatically degradable, pH sensitive, photolabile, or disulfide linker, among others. Other linkers include homobifunctional and heterobifunctional linkers.

In one embodiment of the present invention, the carrier has the function of increasing the in vivo duration of the biomolecule. The carrier may be one or more selected from the group consisting of albumin, antibody fragment, Fc domain, transferrin, XTEN (genetic fusion of non-exact repeat peptide sequence), CTP (carboxy-terminal peptide), PAS (proline-alanine-serine polymer), ELK (elastin-like peptide), HAP (homo-amino acid polymer), GLK (gelatin-like protein), PEG (polyethylene glycol), and fatty acid, but is not limited thereto.

In another embodiment of the present invention, the polyubiquitin scaffold may be formed by covalently bonding a donor ubiquitin in which one or more lysines of the ubiquitin are substituted with other amino acids including arginine or alanine, and an acceptor ubiquitin in which the 6th, 11th, 27th, 29th, 33rd, 48th, or 63rd lysine from the N-terminus is substituted with other amino acids including arginine or alanine. In addition, in another embodiment of the present invention, the 73rd leucine from the N-terminus of the ubiquitin may be substituted with other amino acids including proline.

As used herein, "biomolecule" refers to molecules having biological activity in a living body. In one embodiment of the present invention, the biomolecule may be selected from the group consisting of insulin, insulin analogue, glucagon, glucagon-like peptides, GLP-1 and glucagon dual agonist, GLP-1 and GIP dual agonist, GLP-1 and glucagon and GIP triple agonist, exendin-4, exendin-4 analogue, insulin secreting peptide and an analogue thereof, human growth hormone, growth hormone releasing hormone (GHRH), growth hormone releasing peptide, granulocyte colony stimulating factor (G-CSF), anti-obesity peptide, G-protein-coulped receptor, leptin, GIP (gastric inhibitory polypeptide), interleukins, interleukin receptors, interleukin binding proteins, interferons, interferon receptors, cytokine binding proteins, macrophage activator, macrophage peptide, B cell factor, Tcell factor, suppressive factor of allergy, cell necrosis glycoprotein, immunotoxin, lymphotoxin, tumor necrosis factor (TNF), tumor inhibitory factor, metastasis growth factor, alpha-1 antitrypsin, albumin, $\alpha$-lactalbumin, apolipoprotein-E, erythropoietin (EPO), high glycosylated erythropoietin, angiopoietins, hemoglobin, thrombin, thrombin receptor activating peptide, thrombomodulin, blood factors VII, VIIa, VIII, IX, and XIII, plasminogen activator, fibrin-binding peptide, urokinase, streptokinase, hirudin, protein C, C-reactive protein, renin inhibitor, collagenase inhibitor, superoxide dismutase, platelet derived growth factor, epithelial growth factor, epidermal growth factor, angiostatin, angiotensin, bone morphogenetic growth factor, bone morphogenetic protein, calcitonin, atriopeptin, cartilage inducing factor, elcatonin, connective tissue activator, tissue factor pathway inhibitor, follicle stimulating hormone (FSH), luteinizing hormone (LH), luteinizing hormone releasing hormone (LHRH), nerve growth factors, parathyroid hormone (PTH), relaxin, secretin, somatomedin, adrenal cortical hormone, cholecystokinin, pancreatic polypeptide, gastrin releasing peptide, corticotropin releasing factor, thyroid stimulating hormone (TSH), autotaxin, lactoferrin, myostatin, receptor, receptor antagonist, fibroblast growth factor, adiponectin, interleukin receptor antagonist, cell surface antigen, virus derived vaccine antigen, monoclonal antibody, polyclonal antibody and antibody fragments.

As used herein, "binding site" refers to a site that is bound to another material or component, and "binding moiety" refers to a component including a portion capable of binding to another material or component. In addition, as used herein, "active site" refers to a site that induces activity by reacting with a ligand or receptor.

In addition, UCT (ubiquitin C-terminal tag) refers to a specific sequence of the C-terminal site of the ubiquitin, and UCT is conjugated with a specific lysine of another ubiquitin through covalent bond.

In addition, the present invention provides a method for preparing a multifunctional multispecific multimeric biomolecule polymer, in which a polyubiquitin scaffold, two or more biomolecules comprising binding moieties, each specific for different binding sites, and a carrier that prolongs the in vivo duration are directly bound to the N-terminus or the C-terminus of the ubiquitin or are bound by a linker, wherein the method comprises (i) recombinantly expressing a biomolecule to which a ubiquitin C-terminal tag is fused or bound by a linker from a host cell including a procaryotic cell or a eukaryotic cell, and (ii) adding E1, E2 and E3 enzymes, or E1 and E2 enzymes for ubiquitination to the cell lysates or purified products of the host cell and reacting them, wherein the polyubiquitin scaffold is formed by covalently bonding two or more ubiquitins, and the biomolecule is composed of 2 to 10 biomolecules, has active sites that specifically bind to other biomolecules, small molecule chemical compounds or nanoparticles, and is bound to the N-terminus, the C-terminus, or both the N-terminus and the C-terminus of the ubiquitin by a linker.

In one embodiment related thereto, the E2 enzyme may bind to the 6th, 11th, 27th, 29th, 33rd, 48th or 63rd lysine from the N-terminus of the ubiquitin, or may be an E2-25K ubiquitin conjugating enzyme or Ucb13-MMS2, a ubiquitin conjugating enzyme complex, but is not limited thereto.

In another embodiment related thereto, the ubiquitin C-terminal tag may be one in which two or more ubiquitins are repeatedly linked in a head-to-tail form or in a branched form (branched type or iso-peptide branch type form), wherein the ubiquitin linked in a head-to-tail form or in a branched form may be one in which the 75th and 76th glycines from the N-terminus are substituted with other amino acids including valine.

## Effects of the Invention

According to the present invention, since the linkage between biomolecule polymers or complexes is made by a polyubiquitin scaffold, the polyubiquitin may act as a rigid scaffold or linker that maintains the spacing and orientation between biomolecules bound to the polyubiquitin. Therefore, a multifunctional multispecific multimeric biomolecule polymer can be prepared without interference of the active site.

In addition, according to the present invention, the multifunctional multispecific multimeric biomolecule polymer is provided in a form that is bound to a molecule capable of increasing the in vivo duration, and thus may be used for producing drugs requiring the increased in vivo stability and duration of efficacy.

In the present invention, the biomolecule may be one or more selected from the group consisting of a protein, peptide, polypeptide, antibody, antibody fragment, DNA and RNA, and, for example, by using heterologous proteins, modularized functionality may be imparted to the linear multifunctional multimeric polymer. In addition, according to the present invention, the multifunctional multispecific multimeric biomolecule polymer is provided in a form that is bound to a molecule capable of increasing the in vivo duration, and thus may provide the increased stability and duration of efficacy in vivo for the biomolecule.

**Brief Description of Drawings**

Fig. 1 shows the process of preparing the linear multifunctional multimeric fusion protein (UniStac) of the present invention.

Figs. 2 and 3 show results of confirming the UCT fusion protein in a multimeric form formed by the UniStac reaction of the present invention.

Fig. 4 shows various application forms of the linear multifunctional multimeric fusion protein of the present invention.

Fig. 5 shows the results of UniStac preparation using only E1-E2.

Fig. 6 schematically shows the preparation of the linear multifunctional multimeric fusion protein of the present invention and the use thereof by immobilization.

Fig. 7 schematically shows the head-to-tail UCT and UniStac method.

Fig. 8 is a result of confirming by SDS-PAGE after purification of xylose reductase (XR) prepared according to the present invention by GPC.

Fig. 9 is a result of confirming by SDS-PAGE after purification of oxaloacetate decarboxylase (OAC) prepared according to the present invention by GPC.

Fig. 10 is a result of confirming by SDS-PAGE after purification of xylitol dehydrogenase (XDH) prepared according to the present invention by GPC.

Fig. 11 is a result of confirming by SDS-PAGE after purification of triose-phosphate isomerase (TIM) prepared according to the present invention by GPC.

Fig. 12 is a result of confirming by SDS-PAGE after purification of aldolase (ALD) prepared according to the present invention by GPC.

Fig. 13 is a result of confirming by SDS-PAGE after purification of fructose 1,6-bisphosphatase (FBP) prepared according to the present invention by GPC.

Fig. 14 is a result of confirming by SDS-PAGE after purification of pyruvate oxidase (POPG) prepared according to the present invention by GPC.

Fig. 15 is a result of analysis of the activity of xylose reductase.

Fig. 16 is a result of analysis of the stability of xylose reductase.

Fig. 17 is a result of analysis of the activity of oxaloacetate decarboxylase.

Fig. 18 is a result of analysis of the stability of oxaloacetate decarboxylase.

Fig. 19 is a result of analysis of the activity of xylitol dehydrogenase.

Fig. 20 is a result of analysis of the stability of xylitol dehydrogenase.

Fig. 21 is a result of analysis of the activity of pyruvate oxidase.

Fig. 22 shows the UniStac polymer of the structure to which three enzymes, TIM, ALD and FBP are bound.

Fig. 23 shows the synergistic effect by TIM, ALD and FBP enzymes.

Fig. 24 is a result of preparing and confirming Protein A and Protein G linear multifunctional multimer complexes.

Fig. 25 is a result of preparing and confirming hGH in which aspartate is extended at the C-terminal portion of the 76th glycine of the ubiquitin C-terminal tag.

Fig. 26 is a result of preparing and confirming the polymer originated from E3.

Fig. 27 is a result of preparing and confirming the polymer of hGH according to the presence or absence of DUB.

Fig. 28 shows that the binding activity of human derived IgG to the beads on which the Protein A monomer is immobilized and the beads on which the Protein A polymer is immobilized.

Fig. 29 shows the structure of a linear multifunctional multimeric biomolecule polymer bound to the N-terminus, the C-terminus, or both the N-terminus and the C-terminus of the ubiquitin, respectively, and a result of the preparation thereof.

Fig. 30 is a PK profile result showing that the half-life in blood is at the same level and the bioabsorption rate (AUC) is more excellent when comparing a biomolecule polymer in which carrier is bound to double ubiquitin and a human serum albumin.

Fig. 31 shows a pcDNA3.1 (+) vector to which a gene expressing an Fc based acceptor protein is linked.

Fig. 32 shows a result of confirming the expression of the Fc based acceptor protein.

Fig. 33 shows a result of confirming the expression of the Fc based acceptor protein that specifically binds to an IgG Fc antibody.

Figs. 34 and 35 show results of purifying the Fc based acceptor protein.

Fig. 36 shows a result of confirming ubiquitin-IL-IRA through SDS-PAGE analysis method.

Fig. 37 shows the process of purifying ubiquitin-IL-1RA.

Figs. 38 and 39 show results of purifying the His-SUMO tagged ubiquitin-IL-IRA protein.

Fig. 40 shows a result of confirming the His-SUMO tagged ubiquitin-IL-IRA through SDS-PAGE analysis method.

Figs. 41 and 42 show results of confirming the His-SUMO detagged ubiquitin-IL-1RA.

Figs. 43 and 44 show results of purifying the ubiquitin-IL-1RA protein.

Fig. 45 shows a result of confirming the degree of conjugation between the acceptor and the donor through SDS-PAGE analysis method.

Fig. 46 shows a result of analysis of the conjugation yield using μCE-SDS analysis method.

Fig. 47 shows the process of purifying the conjugation.

Figs. 48 and 49 show results of confirming the conjugation purified with Ni-sepharose.

Figs. 50 and 51 show results of confirming the purified conjugation.

Figs. 52 and 53 are results of confirming the final UniStac polymer in SDS-PAGE (reducing and native conditions).

Fig. 54 shows a result of confirming the monomer using μCE-SDS analysis method.

Fig. 55 shows a result of confirming the monomer using SEC-HPLC.

Fig. 56 shows the structures of the donor protein (D-192), the UniStac protein (C-193), and the fusion protein (C-192; comparative group) using an acceptor protein using a human-serum albumin as a carrier.

Fig. 57 shows a graph of blood drug concentration over time after subcutaneous administration of the fusion proteins (C-192 and D-192).

Fig. 58 shows a graph of blood drug concentration over time after subcutaneous administration of the fusion proteins (C-193 and D-192).

## Best Mode for Carrying out the Invention

In one embodiment, the present invention provides a method for preparing a multifunctional multispecific multimeric biomolecule polymer, in which a polyubiquitin scaffold, two or more biomolecules comprising binding moieties, each specific for different binding sites, and a carrier that prolongs the in vivo duration are directly bound to the N-terminus or the C-terminus of the ubiquitin or are bound by a linker, wherein the method comprises (i) recombinantly expressing a biomolecule to which a ubiquitin C-terminal tag is fused or bound by a linker from a host cell including a procaryotic cell or a eukaryotic cell, and (ii) adding E1, E2 and E3 enzymes, or E1 and E2 enzymes for ubiquitination to the cell lysates or purified products of the host cell and reacting them, wherein the polyubiquitin scaffold is formed by covalently bonding two or more ubiquitins, and the biomolecule has active sites that specifically bind to other biomolecules, small molecule chemical compounds or nanoparticles, and is bound to the N-terminus, the C-terminus, or both the N-terminus and the C-terminus of the ubiquitin by a linker.

In the present invention, an initiator that initiates the formation of a multifunctional multispecific multimeric biomolecule polymer or complex may be E3, E2, E1, a free ubiquitin, or a target substrate of E3. Here, the E2 enzyme may bind to the 48th or 63rd lysine of the ubiquitin, and the E2 enzyme may be an E2-25K ubiquitin conjugating enzyme, or a ubiquitin conjugating enzyme complex Ucb13-MMS2.

In the present invention, each of the biomolecules preferably binds to the N-terminus of the ubiquitin. In addition, the multimeric biomolecule polymer may be composed of 2 to 30 biomolecules.

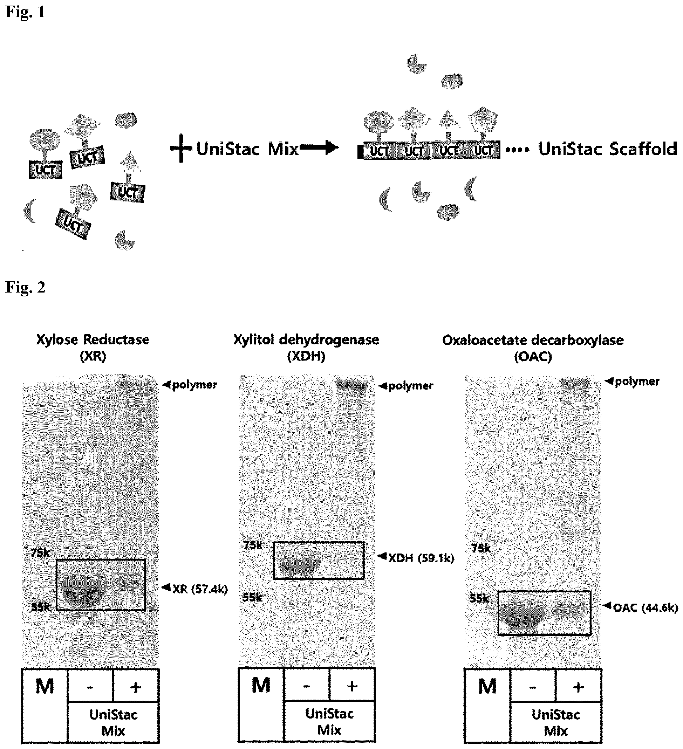

The UniStac reaction of the present invention is schematically shown in Fig. 1.

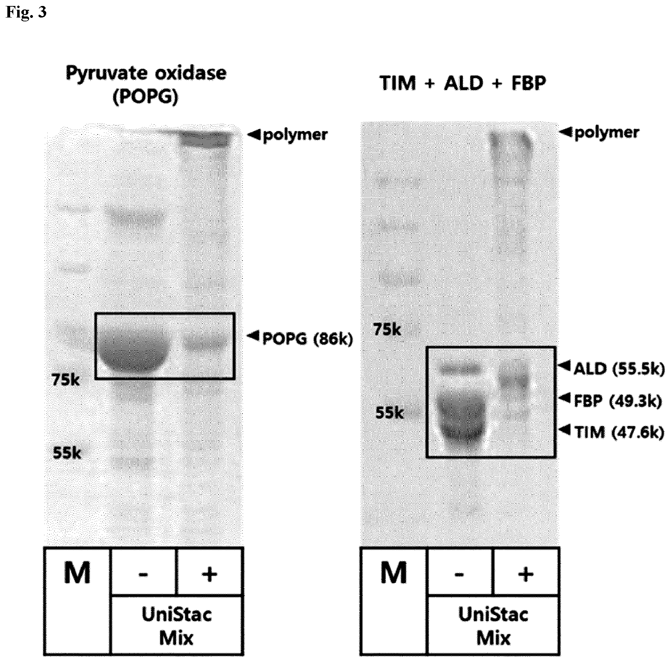

In addition, the results of confirming the UCT fusion protein in a multimeric form formed by the UniStac reaction are shown in Figs. 2 and 3.

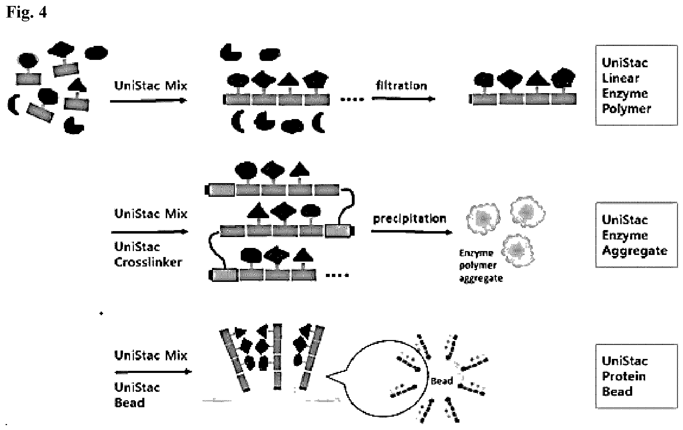

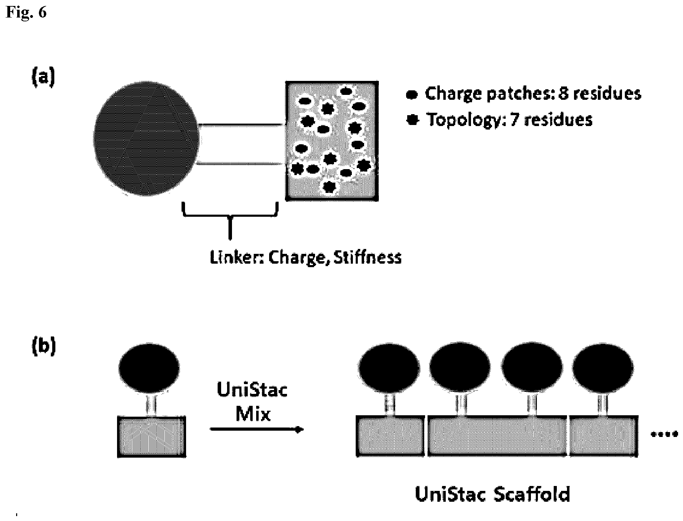

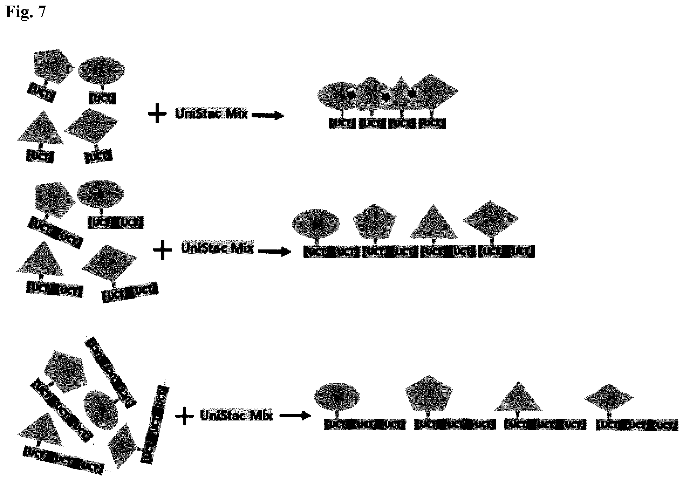

In addition, the multifunctional multispecific multimeric biomolecule polymer of the present invention may be fabricated in various forms. Specific examples are shown in Figs. 4, 6 and 7. That is, the first drawing schematically shows a process of preparing a UniStac linear enzyme polymer by reacting a UniStac mixture with a ubiquitin C-terminal tagged enzyme as shown in Fig. 1 followed by filtration. The second drawing shows a process of preparing a UniStac enzyme aggregate by reacting the UniStac mixture with a ubiquitin C-terminal tagged enzyme, followed by precipitation with a cross linker. The third drawing schematically shows a process of immobilizing the ubiquitin C-terminal tagged protein onto a substrate or a bead.

Hereinafter, the present invention is to be described in more detail through the following examples. These examples are only for describing the present invention in more detail, and it will be apparent to those of ordinary skill in the art that the scope of the present invention is not limited by these examples according to the gist of the present invention.

**[Preparation Example]**

**Preparation Example 1: Cloning, expression and purification of C-terminal fusion protein**

The gene encoding the UCT (ubiquitin C-terminal tag) (SEQ ID NO: 1) protein fusion used in the examples of the present invention was produced on request by Genscript Inc.

In order to prepare a Ub out gene construct that does not comprise a ubiquitin tag at the C-terminus, fast cloning system (Li C, Wen A, Shen B, Lu J, Huang Y, Chang Y (2011). Fast cloning: a highly simplified, purification-free, sequence- and ligation-independent PCR cloning method. BMC Biotechnol 11, 92.) was used. This method is a technology capable of linking genes (insertion, removal or substitution) in which if the PCR product is directly treated with only Dpn1 in the absence of a restriction enzyme and ligase, Dpn1 plays a role of a restriction enzyme and ligase through a mechanism that has not yet been identified along with polymerase. In this method, using a primer designed to overlap both terminus with Phusion polymerase (Thermo Fisher Scientific), PCR (95 °C for 3 minutes, 95 °C for 15 seconds - 55 °C for 1 minute - 72 °C for 1 minute/kb 18 times repeated, 72 °C for 5 minutes, 12 °C for 20 minutes) was carried out on all vectors except for the region to be deleted. Next, the resulting PCR product was subjected to Dpn1 treatment for 1 hour at 37 °C, and transformed into *E. coli* DH5α (Novagen), and then the plasmid of interest was obtained. All gene constructs were identified by commercial DNA sequencing.

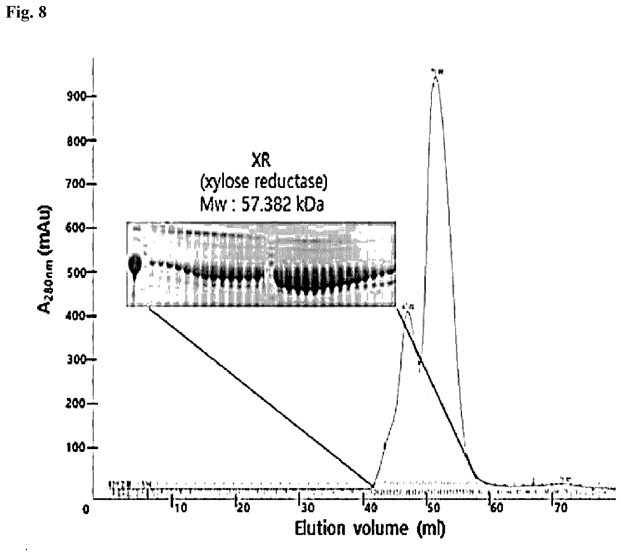

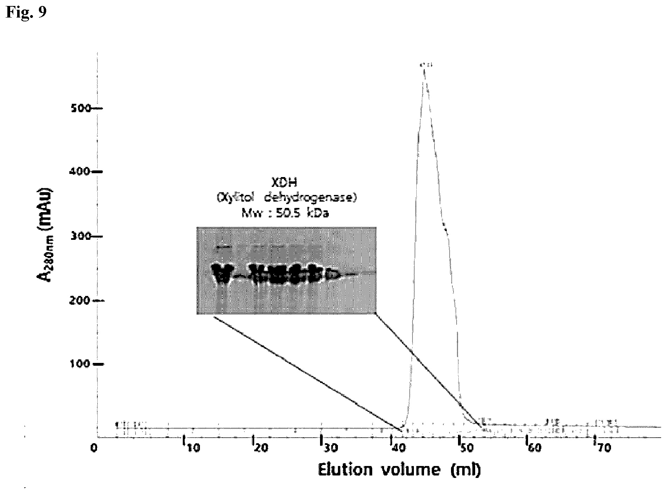

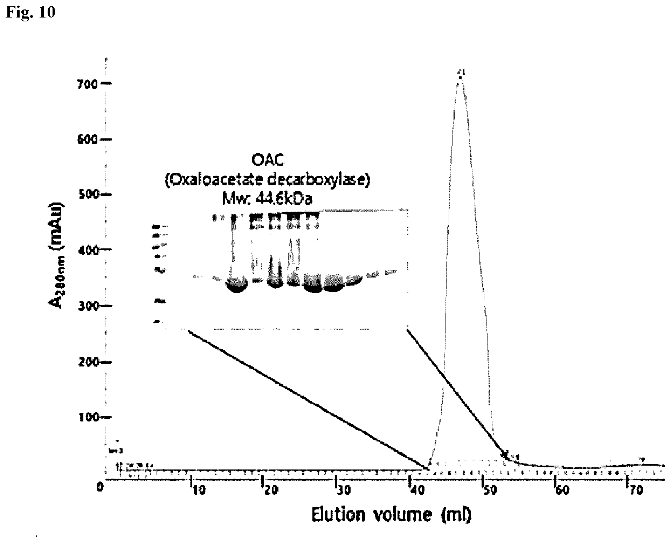

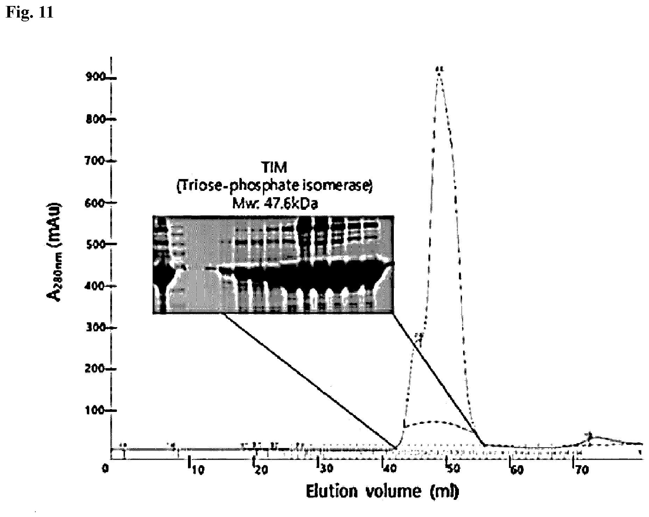

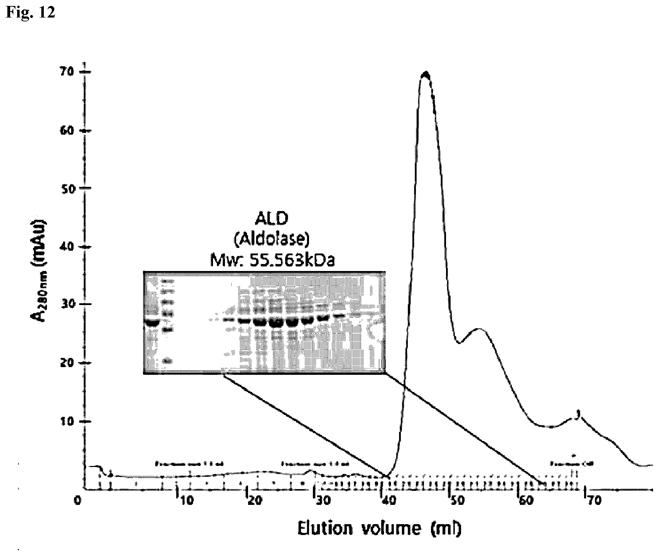

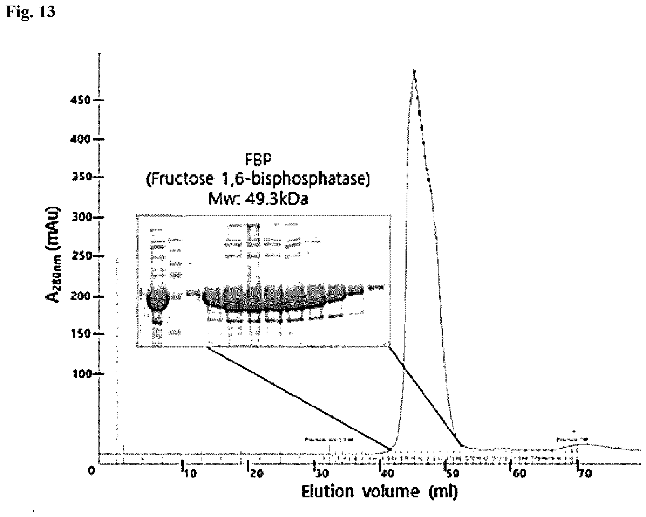

For overexpression of the UCT fusion protein, each gene construct was transformed into *E. coli* BL21 DE3 (Novagen) (XR, TIM, ALD), *Rosetta* pLysS DE3 (Novagen) (XDH, OAC, POPG), *Origami2* DE3 (Novagen) (FBP) strains. Cells comprising the protein expression plasmid (pET21a, Genscript) were incubated in LB medium (Miller) at 37 °C. When the $OD_{600}$ value reached about 0.6, the protein expression was induced with 250 $\mu$M of isopropyl β-D-1-thiogalactopyranoside (isopropyl-beta-D-thiogalactopyranoside) (IPTG) at 16 °C for 20 hours. Next, after centrifugation (at 3,500 rpm at 4 °C for 15 minutes), the cell pellet was resuspended in a lysis buffer (20 mM Tris-HCl pH 8.0, 500 mM $NaCl_2$, 20 mM imidazole) and lysed by sonication (50% amplitude, pluse on 3 seconds-off 5 seconds, final 15 minutes). Then, the lysate was further centrifuged at 14,000 rpm at 4 °C for 30 minutes. The water soluble fraction of the protein comprising the N-terminal His-tag was purified by gel filtration chromatography using Superdex 75 pg gel filtration column 16/600 (GE Healthcare) pre-equilibrated with nickel affinity and FPLC buffer (Ni-NTA Agarose, QIAGEN, 20 mM Tris-HCl pH 8.0, 150 mM $NaCl_2$). All UCT proteins were concentrated to 100 $\mu$M for analysis of the enzyme activity. All target proteins were evaluated by SDS-PAGE. Figs. 8 to 14 show the results of confirming the target proteins. The UCT fusion proteins used in the present invention are shown in Table 1 below.

[Table 1]

| UCT protein fusion | Molecular weight (kDa) | SEQ ID NO |
|---|---|---|
| Xylose reductase (XR) | 57.382 | SEQ ID NO: 2 |
| Xylitol dehydrogenase (XDH) | 59.1 | SEQ ID NO: 3 |
| Oxaloacetate decarboxylase (OAC) | 44.6 | SEQ ID NO: 4 |
| Triose-phosphate isomerase (TIM) | 47.6 | SEQ ID NO: 5 |
| Aldolase (ALD) | 55.563 | SEQ ID NO: 6 |
| Fructose 1,6-bisphosphatase (FBP) | 49.3 | SEQ ID NO: 7 |
| Pyruvate oxidase (POPG) | 86.032 | SEQ ID NO: 8 |

**Preparation Example 2: Preparation of UniStac linear construct**

In the present invention, the reaction for preparing the fusion protein in a linear multifunctional multimeric form was designated as UniStac reaction. The UniStac reaction (a total volume of 50 $\mu$L) was carried out in the UniStac buffer (25 mM HEPES (Sigma-aldrich), pH 7.5, 50 mM NaCl, 4 mM $MgCl_2$), and the UniStac mixture for the UniStac reaction (0.5 $\mu$M E1, 5 $\mu$M E2, 1 $\mu$M E3, 4 mM ATP) was added to the UCT protein fusion of the present invention to initiate the reaction. The UniStac reaction was carried out by shaking at room temperature for 1 hour.

The ratio of proteins used in the reaction was at a concentration of 10 $\mu$M to 20 $\mu$M UCT protein fusion per 1 $\mu$M E3 enzyme (a ratio of 1:10 to 1:20), which was a condition set for the purpose so that at least 10 fusion monomers form a linear multifunctional multimer within 1 hour through the UniStac reaction. The E1, E2 and E3 used in the present invention are as follows, respectively:

[Table 2]

| Category | Name | SEQ ID NO |
|---|---|---|
| E1 | Yeast UBE 1 | SEQ ID NO: 9 |
| E2 | Ubch5a [ *Homo sapiens*] (UniProtKB - P51668) | SEQ ID NO: 10 |
| | Ubch7 [ *Homo sapiens*] (UniProtKB - P68036) | SEQ ID NO: 11 |
| | E2-25K [ *Homo sapiens*] (UniProtKB - P61086) | SEQ ID NO: 12 |
| | Ubc13 [ *Saccharomyces cerevisiae*] (UniProtKB - P52490) | SEQ ID NO: 13 |
| | MMS2 (UEV - ubiquitin-conjugating enzyme variant) | SEQ ID NO: 14 |
| E3 | RSP5 (UniProt ID. P39940) | SEQ ID NO: 15 |
| | DOA10 (UniProt ID. P40318) | SEQ ID NO: 16 |
| | MARCH5 (UniProt ID. Q9NX47) | SEQ ID NO: 17 |

**Preparation Example 3: Preparation of UniStac using only E1-E2 (E2 platform)**

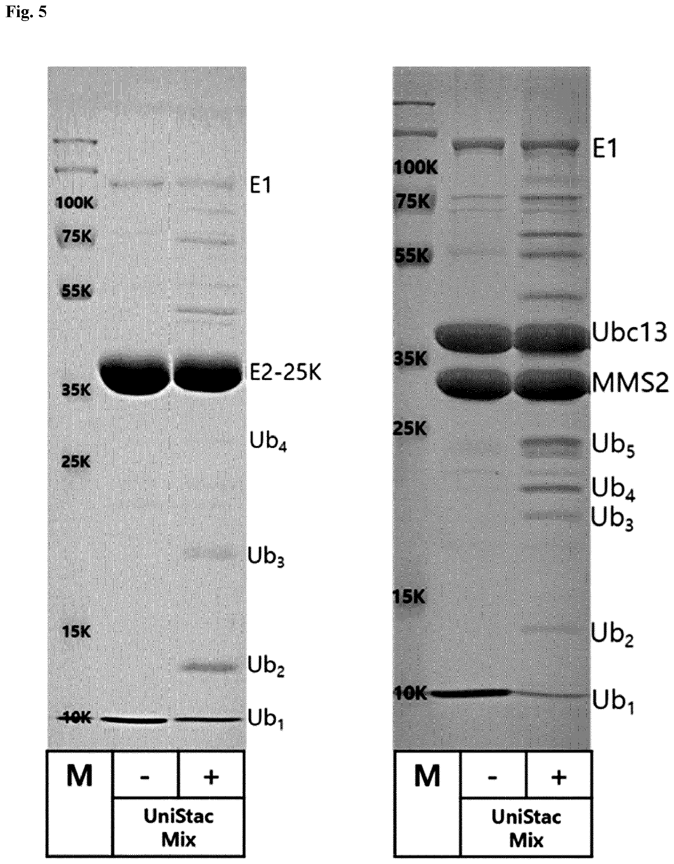

The E2-UniStac was prepared by using E2-25K (GenBank ID-U58522.1) (human E2), Ucb13 (yeast E2)-MMS2 (GenBank ID-U66724.1) (yeast ubiquitin-conjugating enzyme variant) (GenBank ID-U66724.1). The recombinant DNA plasmid synthesized by Genscript was used. The E2-UniStac reaction was carried out under a condition of the buffer (50 mM Tris pH 8.0, 5 mM $MgCl_2$), and the E2-UniStac mixture (1 $\mu$M E1, 10 $\mu$M E2, 4 mM ATP) was added to the free ubiquitin solution (20 $\mu$M) to initiate the reaction. The E2-UniStac reaction was carried out by shaking at room temperature for 1 hour. The results are shown in Fig. 5.

**[Example]**

**Example 1: Analysis of activity and stability of xylose reductase (XR)** <u>Analysis of activity of xylose reductase</u>

The UniStac reaction was carried out in the UniStac buffer (25 mM HEPES pH 7.5, 50 mM NaCl, 4 mM $MgCl_2$), and the UniStac mixture (0.5 $\mu$M E1, 5 $\mu$M E2, 1 $\mu$M E3, 4 mM ATP) was added to the XR protein solution to initiate the reaction. The UniStac reaction was carried out by shaking at room temperature for 1 hour, and then the catalytic activity was analyzed. The catalytic activity of XR was analyzed by measuring the change in absorbance at 340 nm induced by NADH oxidation.

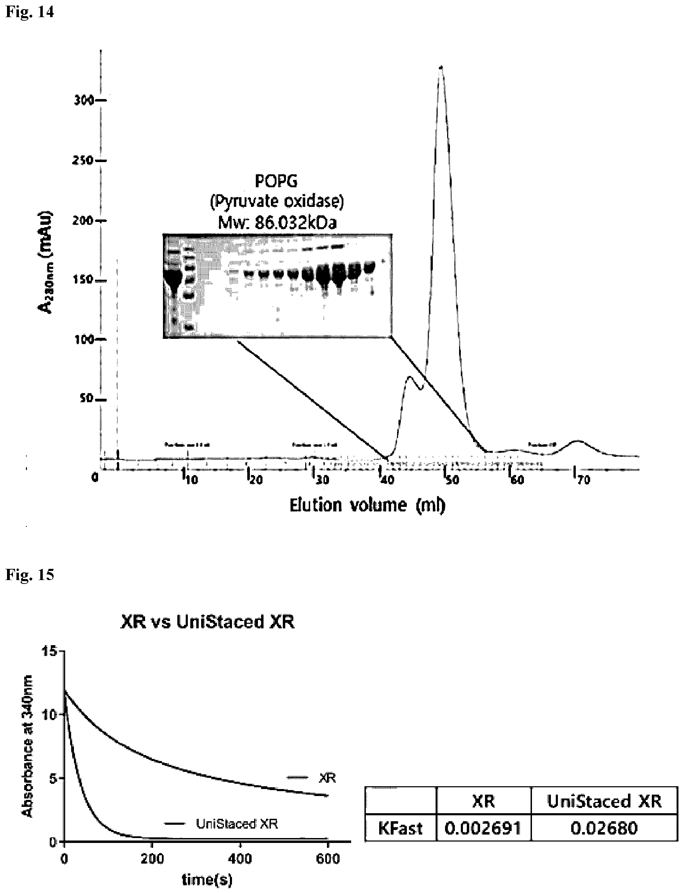

The reaction for analysis of the catalytic activity was initiated by adding NADH (2 mM) to a mixture of XR (10 $\mu$M) and xylose (200 mM) in a 100 mM NaCl buffer (pH 7.0) containing 1 mM $MgCl_2$ and 0.02% Tween-20. XR was a sample in the form of a monomer that did not comprise a ubiquitin tag at the C-terminus of the XR, and did not form a polymer under the same UniStac mixing condition. The statistical analysis was carried out using Prism 6 (GraphPad Software, Inc). The results are shown in Fig. 15.

As shown in Fig. 15, the XR according to the present invention promoted the reduction of D-xylose to xylitol by using NADH as a co-substrate. Absorbance represents the amount of NADH in solution. The UniStac polymer of the XR (lower curve) showed faster NADH consumption compared to the monomer form (upper curve). Both reactions contained an equal amount of the monomers. Therefore, the increased reaction rate is solely dependent on the covalent bonds between the monomers. In the end, it was confirmed that the activity of the XR UniStac polymer was increased by 10 times compared to the XR monomer without ubiquitin-tag.

<u>Analysis of pH stability of xylose reductase</u>

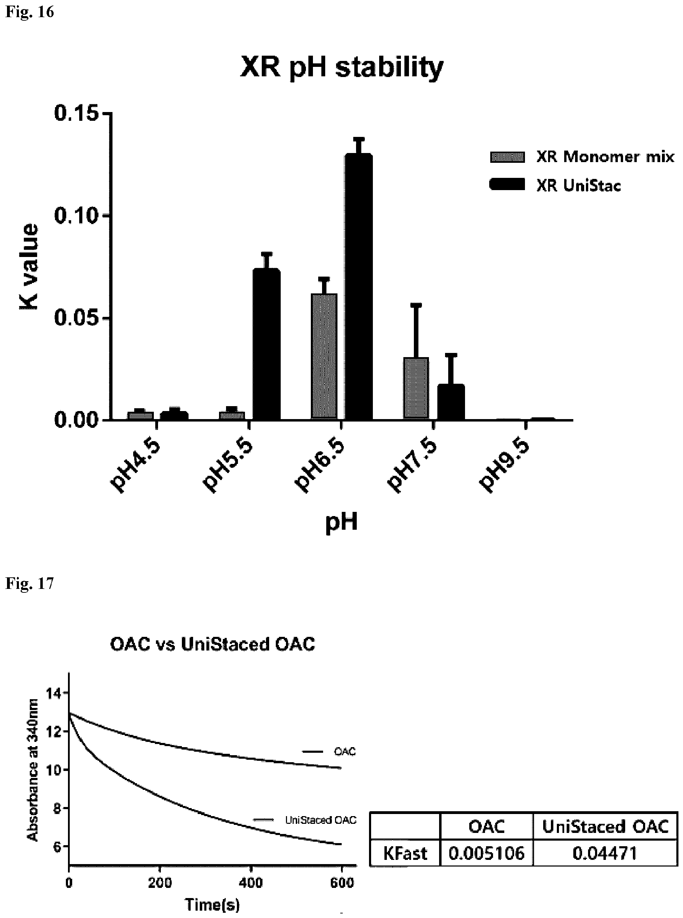

Both the XR monomer and the UniStac polymer were treated for 30 minutes at the indicated pH before initiating the reaction with the addition of NADH and xylose. As shown in Fig. 16, at pH 5.5 and 6.5, the XR UniStac polymer showed significantly enhanced stability compared to the XR monomer without ubiquitin-tag. The results represent the average value of the three experiments.

**Example 2: Analysis of activity and stability of oxaloacetate decarboxylase (OAC)** <u>Analysis of activity of OAC</u>

OAC involved in gluconeogenesis is used to investigate liver damage in conjunction with AST-ALT. The UniStac reaction was carried out in the UniStac buffer (25 mM HEPES pH 7.5, 50 mM NaCl, 4 mM MgCl$_2$), and the UniStac mixture (0.5 $\mu$M E1, 5 $\mu$M E2, 1 $\mu$M E3, 4 mM ATP) was added to the OAC protein solution to initiate the reaction. The UniStac reaction was carried out by shaking at room temperature for 1 hour, and then the catalytic activity was analyzed. The analysis of OAC activity was based on the decrease in absorbance (340 nm) as NADH consumption proceeded under the following conditions: 45 mM TEA buffer pH 8.0, 0.45 mM MnCl$_2$, 2 mM NADH, 11 U of LDH, 5 $\mu$M OAC, 2.5 mM.

The OAC was a sample in the form of a monomer that did not comprise a ubiquitin-tag at the C-terminus of the OAC, and did not form a polymer under the same UniStac mixing condition. The statistical analysis was carried out using Prism 6 (GraphPad Software, Inc). The results are shown in Fig. 17.

As shown in Fig. 17, as a result of comparing the activity of the monomer without Ub (OAC) at the C-terminus and the activity of the polymer (UniStaced OAC), the activity of the polymer was increased by 9 times. Absorbance represents the amount of NADH in solution. The UniStac polymer of the OAC (lower curve) showed faster NADH consumption compared to the monomer form (upper curve). Both reactions contained an equal amount of the monomers. Therefore, the increased reaction rate is solely dependent on the covalent bonds between the monomers. In the end, it was confirmed that the activity of the OAC UniStac polymer was increased by 9 times compared to the OAC monomer without ubiquitin-tag (OAC).

### Analysis of stability of OAC

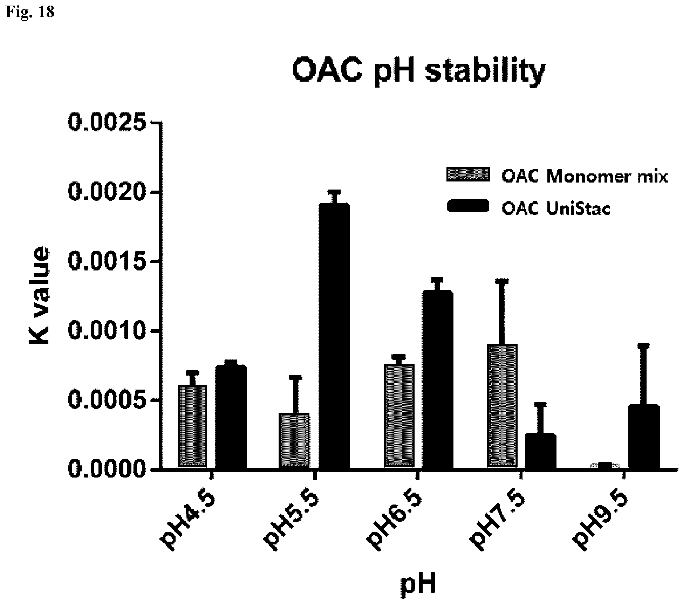

Both the OAC monomer and the UniStac polymer were treated for 30 minutes at the indicated pH before initiating the reaction with the addition of NADH and oxaloacetate. As shown in Fig. 18, at a low pH of pH 4.5 to 6.5, the OAC UniStac polymer showed significantly enhanced pH stability compared to the OAC monomer without ubiquitin-tag (OAC). The results represent the average value of the three experiments.

### Example 3: Analysis of activity and stability of xylitol dehydrogenase (XDH) <ins>Analysis of activity of XDH</ins>

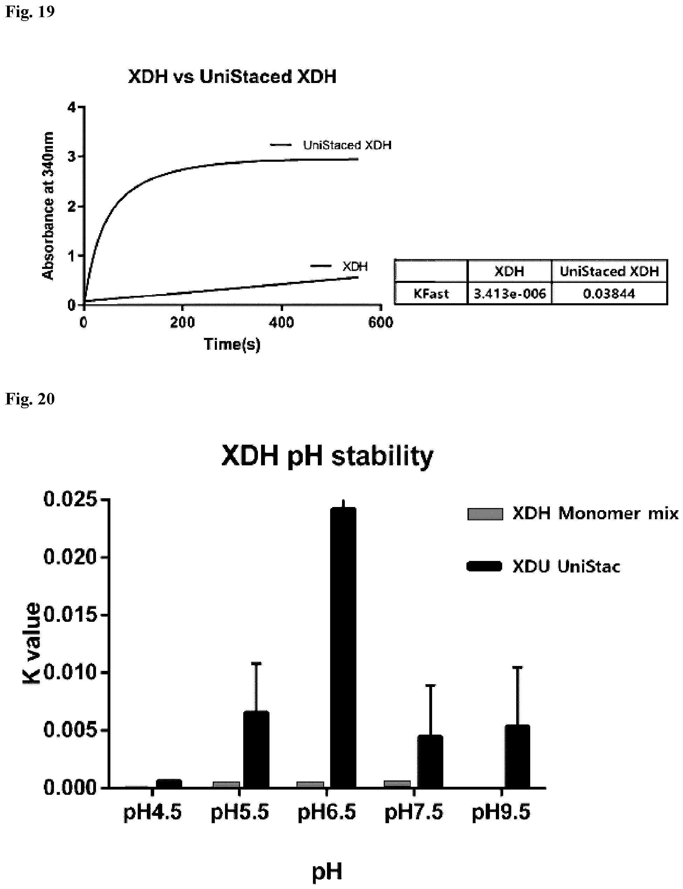

XDH is an enzyme belonging to the D-xylose catabolism pathway, and is known to convert xylitol, a product of XR, into xylulose using NAD$^+$. For analysis of activity of XDH, the UniStac reaction was first carried out in the UniStac buffer (25 mM HEPES pH 7.5, 50 mM NaCl, 4 mM MgCl$_2$), and the UniStac mixture (0.5 $\mu$M E1, 5$\mu$M E2, 1 $\mu$M E3, 4 mM ATP) was added to the XDH protein solution to initiate the reaction. The UniStac reaction was carried out by shaking at room temperature for 1 hour, and then the catalytic activity was analyzed. The activity of XDH was measured by monitoring NAD$^+$reduction at 340 nm. The reaction was initiated by adding NADH (2 mM) to a mixture of XDH (20 $\mu$M) and xylose (200 mM) in a 100 mM NaCl buffer (pH 7.0) containing 1 mM MgCl$_2$ and 0.02% Tween-20. The XDH was a sample in the form of a monomer that did not comprise a ubiquitin-tag at the C-terminus of the XDH, and did not form a polymer under the same UniStac mixing condition. The statistical analysis was carried out using Prism 6 (GraphPad Software, Inc). The results are shown in Fig. 19.

As shown in Fig. 19, at pH 5.5, the UniStac polymer of the XDH (upper curve) showed higher NADH+ consumption rate compared to its monomer form (lower curve). Both reactions contained an equal amount of the monomers. Therefore, the difference in activity is solely dependent on the covalent bonds between the monomers. In the end, it was confirmed that the activity of the XDH UniStac polymer was increased by 10 times compared to the XDH monomer without ubiquitin-tag (XDH).

### Analysis of stability of XDH

Both the XDH monomer and the UniStac polymer were treated for 30 minutes at the indicated pH before initiating the reaction with the addition of NAD$^+$ and xylitol. As shown in Fig. 20, at all measured pHs, the XR UniStac polymer showed significantly increased pH stability compared to the XDH. The results represent the average value of the three experiments.

### Example 4: Analysis of activity of pyruvate oxidase (POPG)

POPG is known to be used to investigate liver damage by detecting enzymes such as AST-ALT, an enzyme involved in the gluconeogenesis process. For analysis of activity of POPG, the UniStac reaction was first carried out in the UniStac buffer (25 mM HEPES pH 7.5, 50 mM NaCl, 4 mM MgCl$_2$), and the UniStac mixture (0.5 $\mu$M E1, 5 $\mu$M E2, 1 $\mu$M E3, 4 mM ATP) was added to the POPG protein solution to initiate the reaction. The UniStac reaction was carried out by shaking at room temperature for 1 hour, and then the catalytic activity was analyzed. In order to analyze the catalytic activity, the amount of H$_2$O$_2$ produced by the POPG oxidation process of pyruvate by ABTS was measured. The reaction was initiated by adding POPG (5 $\mu$M) to a mixture of pyruvate (100 mM), pyrophosphate (6 mM), ABTS (2,2'-azino-bis(3-ethylbenzothiazoline-6-sulfonic acid) (10 mM) and HRP (horseradish peroxidase) (0.2 U/mL) in a sodium phosphate buffer.

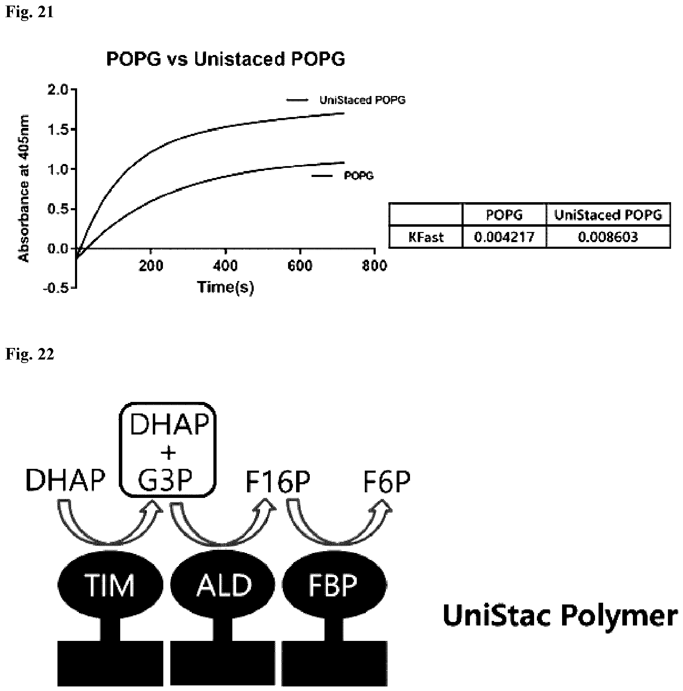

The POPG monomer (POPG) was a sample in the form of a monomer that did not comprise a ubiquitin tag at the C-terminus of the POPG, and did not form a polymer under the same UniStac mixing condition. The statistical analysis was carried out using Prism 6 (GraphPad Software, Inc). As shown in Fig. 21, at pH 5.5, the POPG (upper curve) showed higher activity compared to its monomer form (lower curve). Both reactions contained an equal amount of the monomers. Therefore, the difference in activity is solely dependent on the covalent bonds between the monomers. In the end, it was confirmed that the activity of the POPG UniStac polymer was increased by 2 times compared to the POPG monomer without ubiquitin-tag (POPG).

**Example 5: Analysis of synergistic effect of ubiquitin enzyme**

Triosephosphate isomerase (TIM), fructose bisphosphate aldolase (ALD) and fructose bisphosphatase (FBP) are known to form a cascade reaction for producing F6P as a final product from DHAP (dihydroxyacetone phosphate). The analysis of synergistic effect of the UniStac enzyme was carried out by measuring fructose- 6-Phosphate (F6P), a TIM product, ALD and FBP enzyme complex. F6P is isomerized to glucose-6-phosphate (G6P) by phosphoglucose isomerase (PGI), and an equal amount of $NAD^+$ as a substrate is modified by glucose-6-phosphate dehydrogenase (G6PDH). The present inventors determined the enzyme activity by measuring the amount of newly generated NADH at 340 nm by adding 2.5 mM enzyme complex (dihydroxyacetone phosphate, DHAP), 20 U/mL analysis enzyme (PGI and G6PDH) and 2.5 mM $NAD^+$ enzyme complex to a mixture of 4 $\mu$M TIM, ALD and FBP enzyme complex in a HEPES buffer condition (200 mM HEPES pH 7.5, 10 mM $MgCl_2$, 0.5 mM $MnCl_2$, 1 mM $CaCl_2$).

The enzyme complex mixture was a sample in the form of a monomer that did not comprise a ubiquitin tag at the C-terminus of the enzyme, and did not form a polymer under the same UniStac mixture condition. The statistical analysis was carried out using Prism 6 (GraphPad Software, Inc). At the indicated time point, the reaction was terminated, and the amount of F6P was measured using a phosphoglucose isomerase (PGI) that uses NAD+ to convert F6P into glucose-6-phosphate (G6P). Absorbance represents the amount of F6P.

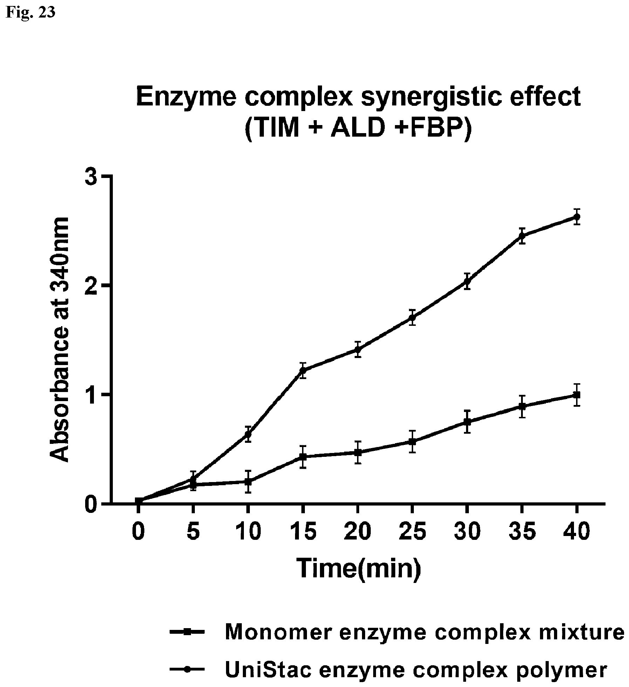

The results of the experiment are shown in Fig. 23. As shown in Fig. 23, the UniStac polymer of three different enzymes (upper curve) showed higher activity by five times than the monomeric enzyme mixture (lower curve), confirming the synergistic effect by the UniStac enzymes. Fig. 22 shows the resulting product of the structure (UniStac Polymer) in which three enzymes, TIM, ALD and FBP are bound.

**Example 6: Ubiquitin multistage labeling (prosthetics) method**

A ubiquitin C-terminal tagged biomolecule was synthesized according to the preparation examples of the present invention. Next, a polymer (polyethylene glycol) comprising hydroxylamine was reacted with the above biomolecule. As a result, it was confirmed that the polymer was labeled with ubiquitin by an oxime linkage. The oxime linkage can be used as a tool capable of allowing a polymeric drug delivery system.

**Example 7: Preparation of Protein A and Protein G linear multimeric polymer**

The UniStac reaction (a total volume of 50 $\mu$L) was carried out in the UniStac buffer (25 mM HEPES pH 7.5, 50 mM NaCl, 4 mM $MgCl_2$), and the UniStac mixture (0.5 $\mu$M E1, 5 $\mu$M E2, 1 $\mu$M E3, 4 mM ATP) was added to the Protein A or Protein G solution to initiate the reaction. Recombinant DNA plasmids comprising sequences corresponding to Protein A (GenBank ID-AAB05743.1) and Protein G (CAA27638.1) that were synthesized by Genscript were used. The UniStac reaction was carried out by shaking at room temperature for 1 hour, and then SDS-PAGE was carried out.

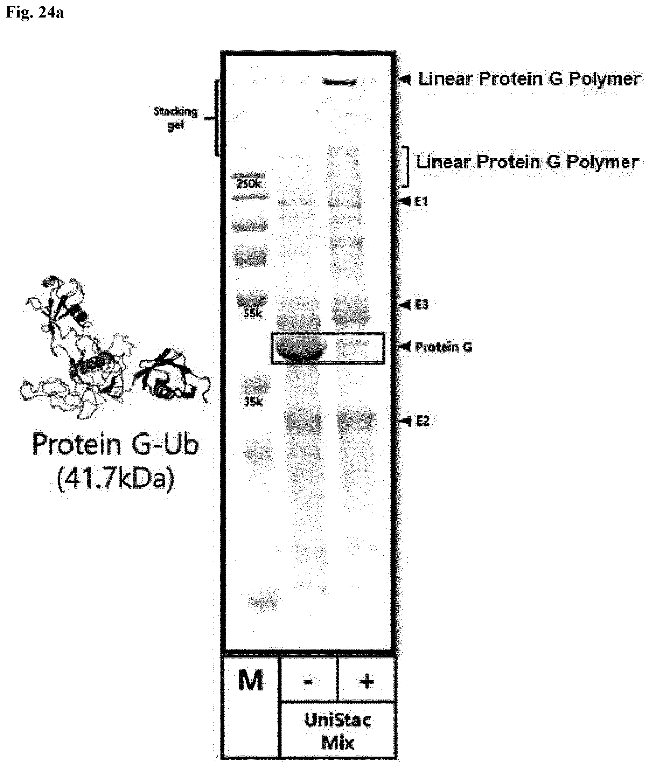

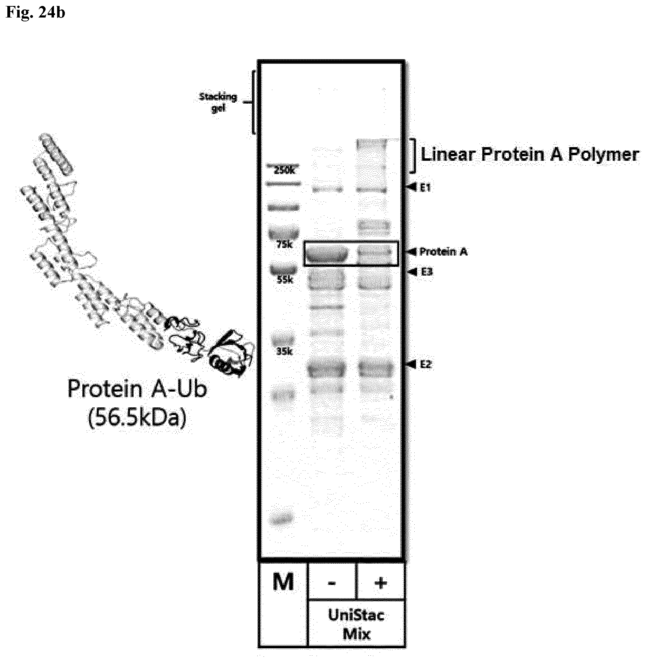

As shown in Fig. 24, compared to the sample without the UniStac mixture, it was confirmed that a monomer band of Protain A or Protein G was reduced in the sample to which the UniStac mixture was added, and a band of high molecular weight (linear multimeric polymer) newly appeared. In addition, it was confirmed that some linear multimeric polymers did not pass through a stacking gel due to an increase in molecular weight of up to several hundreds kDa.

**Example 8: hGH in which the C-terminus of the 76th glycine of the ubiquitin C-terminal tag is extended by aspartate**

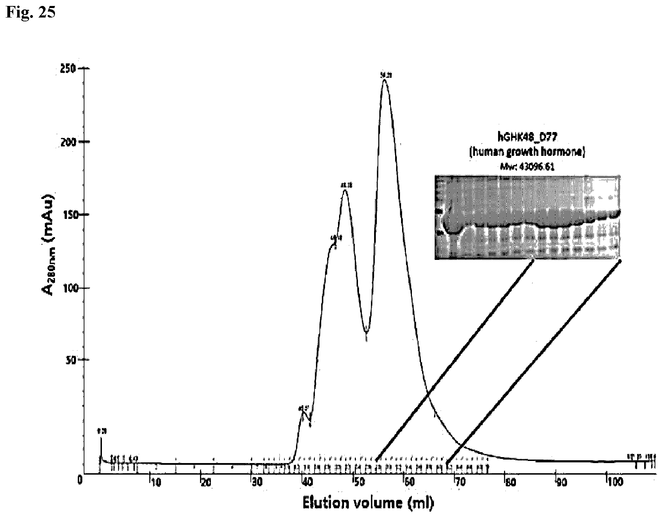

For overexpression of the UCT fusion protein, each gene construct was transformed into *E. coli* BL21 DE3 (Novagen) strain. In this example, hGH (SEQ ID NO: 18) was used as a protein. Cells comprising the protein expression plasmid (pET21a, Genscript) were incubated in LB medium (Miller) at 37 °C. When the $OD_{600}$ value reached about 0.6, the protein expression was induced with 250 $\mu$M isopropyl $\beta$-D-1-thiogalactopyranoside (isopropyl-beta-D-thiogalactopyranoside) (IPTG) at 16 °C for 20 hours. Next, after centrifugation (at 3,500 rpm at 4 °C for 15 minutes), the cell pellet was resuspended in a lysis buffer (20 mM Tris-HCl pH 8.0, 500 mM $NaCl_2$, 20 mM imidazole) and lysed by sonication (50% amplitude, pluse on 3 seconds-off 5 seconds, final 15 minutes). Then, the lysate was further centrifuged at 14,000 rpm at 4 °C for 30 minutes. The water soluble fraction of the protein comprising the N-terminal His-tag was purified by gel filtration chromatography using Superdex 75 pg gel filtration column 16/600 (GE Healthcare) pre-equilibrated with nickel affinity and FPLC buffer (Ni-NTA Agarose - QIAGEN, 20 mM Tris-HCl pH 8.0, 150 mM $NaCl_2$). The purified hGH was concentrated to 100 $\mu$M and evaluated by SDS-PAGE. The results are shown in Fig. 25.

**Example 9: Preparation of polyubiquitin scaffold originated from E3 (Rsp5)**

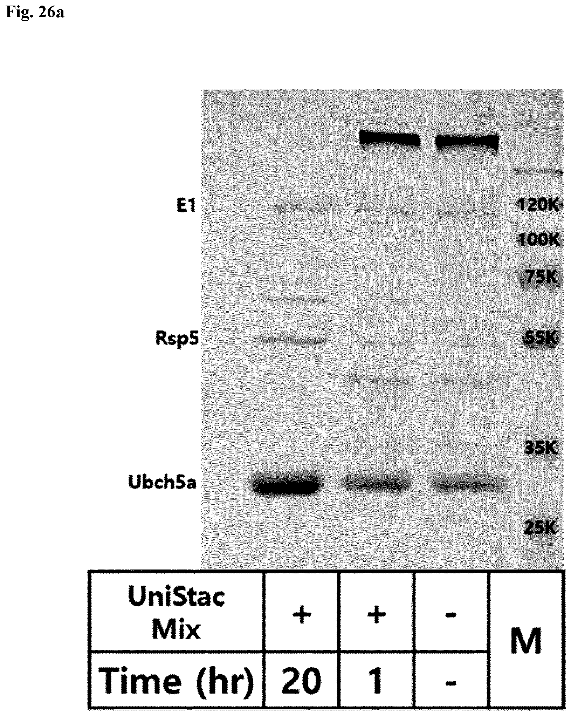

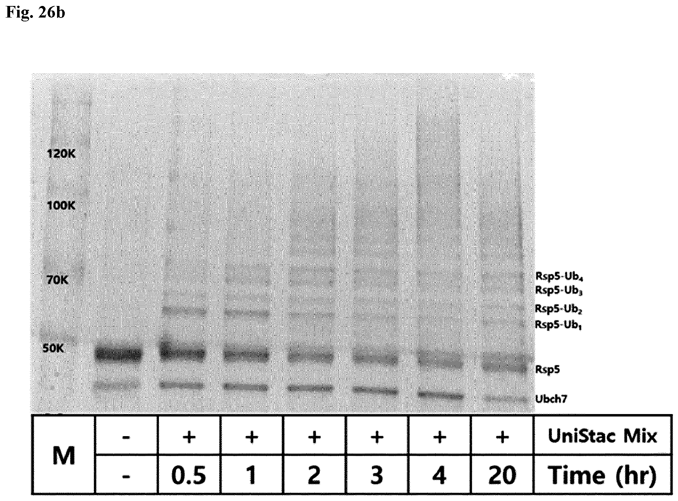

The UniStac reaction (a total volume of 50 $\mu$L) was carried out in the UniStac buffer (25 mM HEPES pH 7.5, 50 mM NaCl, 4 mM $MgCl_2$), and the UniStac mixture (0.5 $\mu$M E1, 5 $\mu$M E2 (Ubch5a or Ubch7), 1 $\mu$M E3, 4 mM ATP) was added to the protein solution to initiate the reaction. The UniStac reaction was carried out by shaking at room temperature for 1 hour, and then SDS-PAGE was carried out.
As shown in Fig. 26, it was confirmed that the amount of E3 was reduced in the sample to which the UniStac mixture was added compared to the sample without the UniStac mixture. This is because the band of E3 whose molecular mass was increased due to the formation of a polyubiquitin scaffold originated from E3 was shifted upwards. In addition, compared to the UniStac mixture comprising Ubch5a E2 (Fig. 26a), in the results of adding the UniStac mixture comprising Ubch7 E2 that has a weak reactivity (Fig. 26b), it was confirmed that the molecular weight was gradually increased by linking ubiquitin one by one to E3 (Rsp5) over time.

**Example 10: Preparation of polymer of hGH according to the presence or absence of DUB**

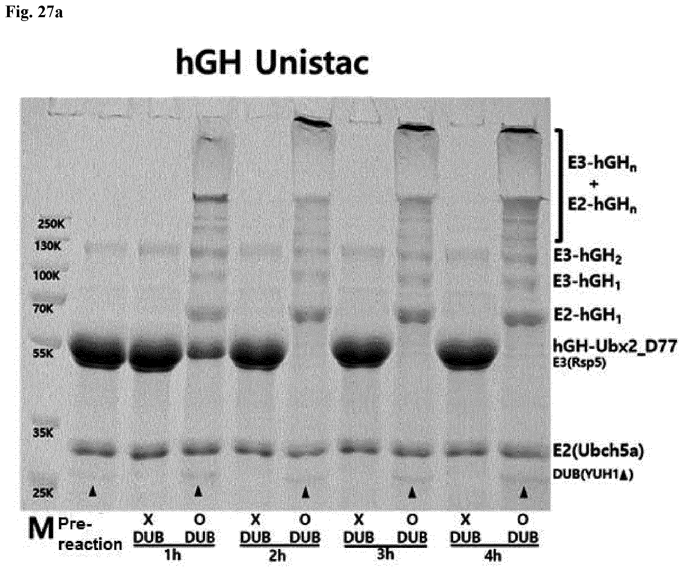

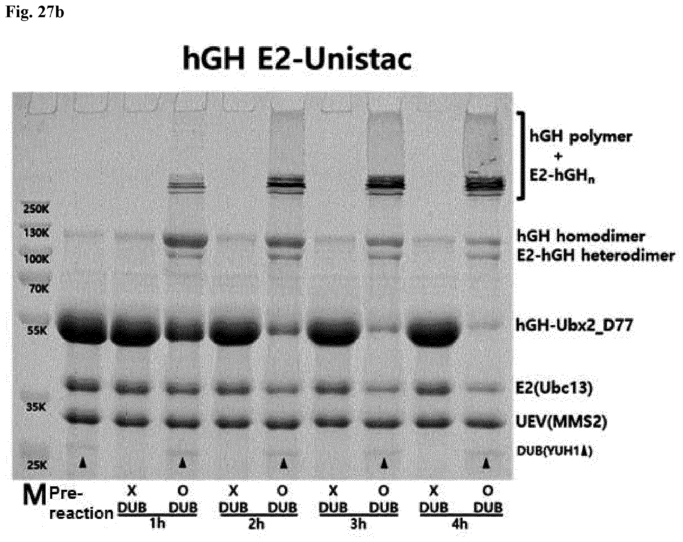

The UniStac reaction of hGH (SEQ ID NO: 18) was compared under the condition where DUB was present together and the condition where DUB was excluded. The hGH used at this time is one in which two ubiquitin tags are repeatedly connected at the C-terminus in a head-to-tail form, and the C-terminus of the ubiquitin tag is extended with aspartate. Therefore, if the aspartate at the C-terminus of the ubiquitin tag is not cleaved using DUB, the UniStac reaction does not occur.
The UniStac reaction to confirm the polymer formation of hGH, a biomolecule was carried out in the UniStac buffer (25 mM HEPES pH 7.5, 50 mM NaCl, 4 mM $MgCl_2$), and the UniStac mixture (1 $\mu$M E1, 5 $\mu$M E2 (ubch5a), 1 $\mu$M E3, 4 mM ATP) was added to 20 $\mu$M hGH protein solution to initiate the reaction.
In addition, the E2-UniStac reaction where E3 was excluded was carried out in the E2-UniStac buffer (50 mM Tris pH 8.0, 5 mM $MgCl_2$), and the E2-UniStac mixture (1 $\mu$M E1, 10 $\mu$M E2 (Ucb13-MMS2 complex), 4 mM ATP) was added to 20 $\mu$M hGH protein solution to initiate the reaction.
In order to confirm the activity of DUB together, the reaction was carried out simultaneously under the condition where DUB (YUH1) was absent and the condition where DUB (YUH1) was present at a concentration of 2 $\mu$M, respectively. All reactions were carried out by shaking at room temperature for 1 to 4 hours and then confirmed by SDS-PAGE.
As shown in Fig. 27, it was confirmed that the polymer of hGH was formed only under the condition where DUB was present, and it was confirmed that the polymer was not formed because the aspartate at the C-terminus of the hGH UCT was not cleaved under the condition where DUB was absent.

**Example 11: Binding activity of Protein A polymer immobilized on bead**

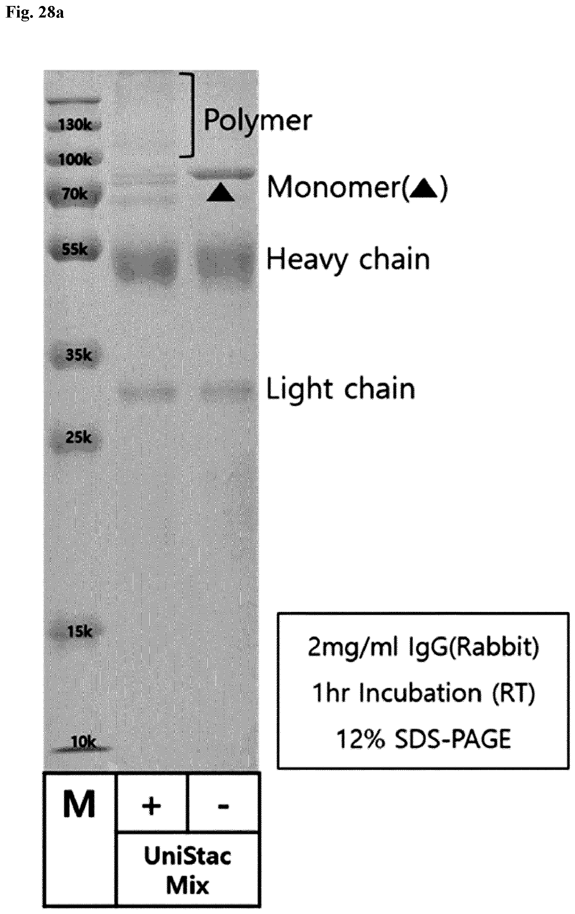

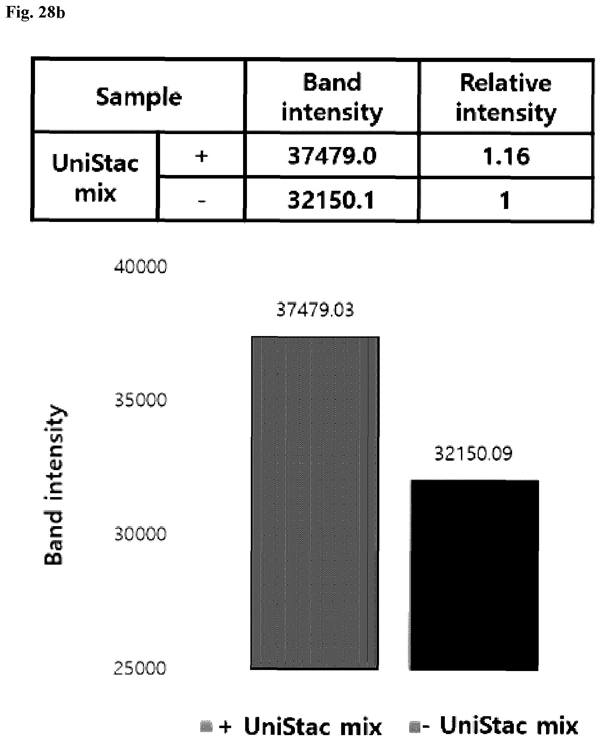

The UniStac reaction to prepare the Protein A polymer was carried out in the UniStac buffer (25 mM HEPES pH 7.5, 50 mM NaCl, 4 mM $MgCl_2$). The UniStac mixture (0.5 $\mu$M E1, 5 $\mu$M E2 (Ubch5a or Ubch7), 1 $\mu$M E3, 4 mM ATP) was added to the Protein A protein solution to initiate the reaction. The UniStac reaction was carried out by shaking at room temperature for 1 hour, and then mixing in a 1:1 ratio together with latex beads at 50% concentration, and then shaking at ambient temperature for 4 hours, and immobilizing the Protein A polymer on the beads. After the immobilization reaction, in order to remove the unimmobilized protein, washing was carried out three times with the PBS buffer (10 mM $Na_2HPO_4$ pH 7.4, 1.8 mM $KH_2PO_4$, 137 mM NaCl, 2.7 mM KCl). After washing, the immunoglobulin G (IgG) obtained from human serum was added to the beads at a concentration of 2 mg/mL to analyze the binding activity of the Protein A polymer immobilized on the beads. The binding reaction was carried out by shaking at ambient temperature for 1 hour, and then washing three times with the PBS buffer in the same manner as in the above washing method, and then confirmed by SDS-PAGE.
As shown in Fig. 28, it was confirmed that the binding activity of human derived IgG to the beads on which the Protein A polymer was immobilized was increased by 15% or more compared to the beads on which the Protein A monomer was immobilized in the same manner except that the UniStac mixture was not added.

**Example 12: Preparation of linear multivalent biomolecule polymer bound to N-terminus, C-terminus, or both N-terminus and C-terminus of ubiquitin, respectively**

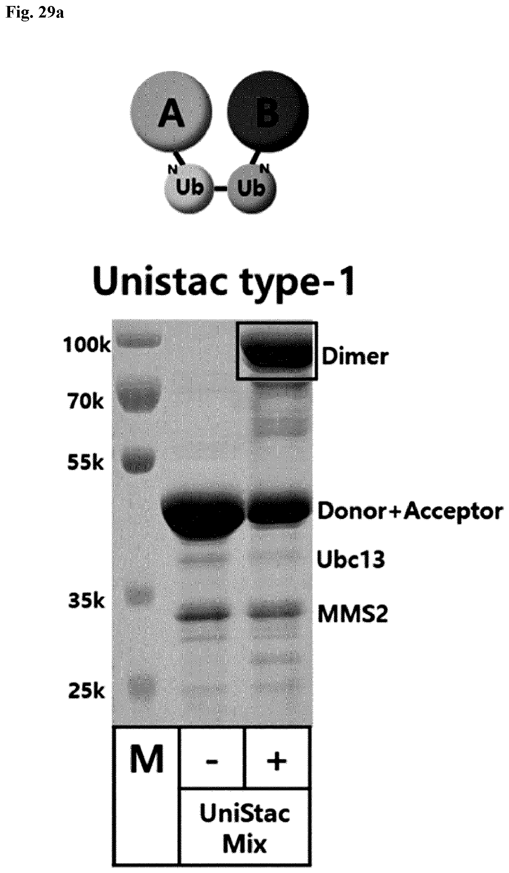

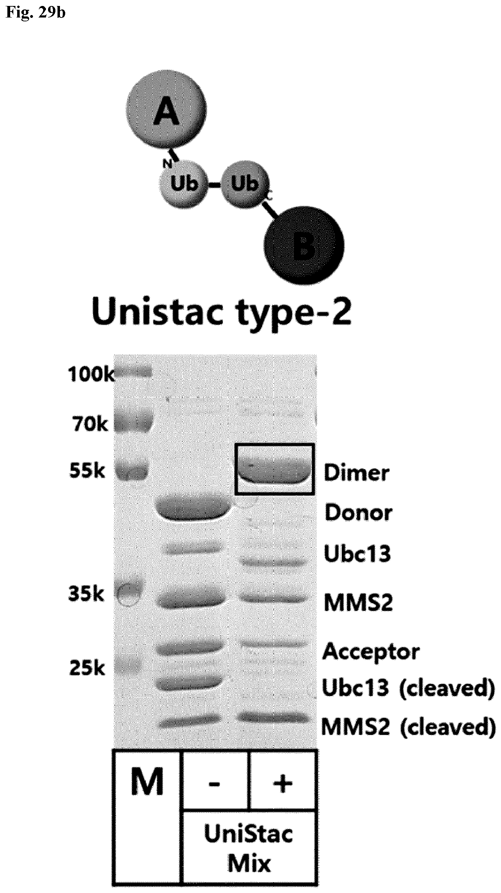

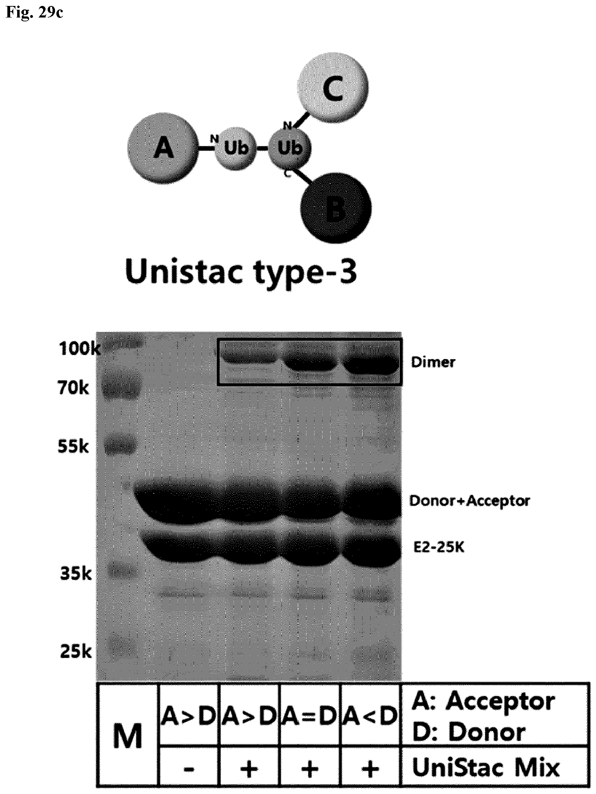

The formation of the UniStac dimer was confirmed by preparing the dimer of the donor ubiquitin in which hGH was bound to the N-terminus (SEQ ID NO: 18) and the acceptor ubiquitin in which hGH was bound to the N-terminus (SEQ ID NO: 19) (Fig. 29 (a)); the dimer of the donor ubiquitin in which hGH was bound to the N-terminus (SEQ ID NO: 18) and the acceptor ubiquitin in which hGH was bound to the C-terminus (SEQ ID NO: 20) (Fig. 29 (b)); and the dimer of the donor ubiquitin in which hGH was bound to the N-terminus (SEQ ID NO: 18) and the acceptor ubiquitin in which SUMO and hGH were bound to the N-terminus and the C-terminus, respectively (SEQ ID NO: 21) (Fig. 29 (c)), respectively.

The acceptor ubiquitin is a form in which the 73rd leucine is substituted with proline, a form in which other lysines except for the 48th lysine (Fig. 29 (c)) or the 63rd lysine (Fig. 29 (a) and (b)) of the acceptor ubiquitin are substituted with arginine, and a form in which the C-terminus is extended by aspartate or biomolecule (hGH).

The UniStac reaction (Fig. 29 (a) and (b)) was carried out in the UniStac buffer (25 mM HEPES pH 7.5, 50 mM NaCl, 4 mM $MgCl_2$), and the UniStac mixture (1 $\mu$M E1, 5 $\mu$M E2 (Ubc13-MMS2 complex), 4 mM ATP) was added to a solution (a total ubiquitin concentration of 20 $\mu$M) in which 10 $\mu$M acceptor ubiquitin protein and donor ubiquitin protein were mixed to initiate the reaction.

In addition, the UniStac reaction (Fig. 29 (c)) was initiated by replacing E2 with E2-25K other than Ubc13-MMS2 complex, and the acceptor ubiquitin with a protein having only the 48th Lys other than the 63rd Lys under the same conditions as the above reaction. The UniStac reaction was carried out by shaking at 27 °C for 4 hours and then confirmed by SDS-PAGE. In the UniStac reaction (Fig. 29 (b)), the acceptor ubiquitin in the Ub-hGH form in which His-sumo was cleaved using the SENP1 enzyme from a protein in the His-sumo-Ub-hGH form was used, and it was confirmed that at this time, the remaining SENP1 was included in the UniStac reaction, and thus the donor hGH, Ubc13 and His-sumo of MMS2 were also cleaved together, and the band of dimer and E2 (Ubc13, MMS2) after reaction was shifted.

As shown in Fig. 29, it was confirmed that the UniStac dimer was formed in all forms in which the biomolecule was bound to the N-terminus, the C-terminus, or both the N-terminus and the C-terminus, respectively. SEQ ID NOs of proteins and the like used in this example are as follows: the donor ubiquitin in which hGH was bound to the N-terminus (SEQ ID NO: 18); the acceptor ubiquitin in which hGH was bound to the N-terminus (SEQ ID NO: 19); the acceptor ubiquitin in which hGH was bound to the C-terminus (SEQ ID NO: 20); the acceptor ubiquitin in which SUMO and hGH were bound to the N-terminus and the C-terminus, respectively (SEQ ID NO: 21).

**Example 13: Confirmation of pharmacokinetics**

Diubiquitin-albumin (OGB1) and albumin (OGB3) were subcutaneously administered to 9-week-old male Spraque-Dawley rats once, and then blood was collected for each time period, and the drug concentration in the serum was analyzed. Diubiquitin-albumin was administered at 0.833 mg/kg, and albumin was administered at 1 mg/kg, and each group was composed of 12 male rats. Blood collection for analysis of blood drug concentration was carried out from 3 rats per group before administration (blank) and 0.5, 1, 2, 4, 6, 8, 12, 24, 36, 48 and 72 hours after administration (a total of 12 time points).

The control group was composed of 5 male rats, and blood collection was carried out 1 and 24 hours after administration. The serum was separated from the collected blood and was stored in a cryogenic frozen state at -70 $\pm$ 10 °C. Analysis was carried out by measuring drug concentrations from the blood samples collected for each time period by the human serum albumin ELISA kit. Serum for dilution was prepared by mixing a buffer for dilution (1x PBS, 1% BSA) and a rat blank serum in a 1:1 ratio and used for the ELISA analysis. Diubiquitin-albumin was diluted in the serum for dilution from 800 ng/mL to 15.625 ng/mL and dispensed into each well. Each sample was diluted using a rat blank serum and a buffer for dilution to obtain a serum for dilution in a final ratio of 1:1 and dispensed into each well. An antibody mixture solution was prepared by diluting the Capture and Detector antibodies of the human serum albumin kit in the antibody dilution CP solution. 100 $\mu$L of the antibody mixture solution was dispensed into each well and incubated at 400 rpm at room temperature for 1 hour. When the incubation was completed, 300 $\mu$L of the washing solution was dispensed into each well, and the process of shaking was repeated three times. 100 $\mu$L of the TMB substrate solution was dispensed into each well and incubated at 400 rpm at room temperature for 10 minutes, and 100 $\mu$L of the stop solution was dispensed into each well and put into the instrument, and absorbance (OD450) was measured. Albumin was diluted from 100 ng/mL to 2.5 ng/mL and dispensed into each well, and the rest of the sample dilution and experimental procedure were performed in the same manner to measure absorbance. A calibration curve was calculated with 4 parameters based on the absorbance values measured for each concentration, and the drug concentration in the serum was finally calculated based on the absorbance values measured from the sample compared to the calibration curve. Pharmacokinetic parameters were calculated using Phoenix WinNonlin (Ver. 8.1, Pharsight-A Certara company, U.S.A.) for the results of measuring the concentration of the test substance in serum to evaluate the pharmacokinetics.

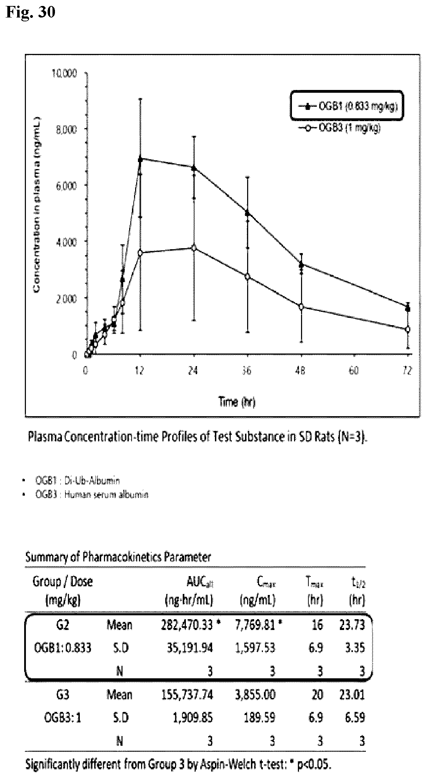

As shown in Fig. 30, it was confirmed that diubiquitin-albumin (OGB1) exhibited a higher plasma concentration. In addition, it was confirmed that AUC was increased by 1.8 times and Cmax was increased by more than 2 times in the group administered with diubiquitin-albumin (OGB1) compared to the group administered with albumin (OGB3). Therefore, it can be seen that the diubiquitin-albumin polymer of the present invention has a more excellent pharmacokinetic effect at a lower concentration.

In the end, the biomolecule polymer of the present invention is provided in a form that is bound to a molecule capable of increasing the in vivo duration, and thus may be used for producing a pharmaceutical composition requiring the increased in vivo duration of efficacy.

**Example 14: Preparation of recombinant expression plasmid DNA capable of expressing Fc based acceptor protein (acceptor protein)**

A fusion protein in which a carrier was directly bound to the C-terminus of the acceptor ubiquitin was prepared by the following method. An antibody fragment (IgG Fc domain) was used as a carrier protein of the fusion protein, and the fusion protein is referred to as an "Fc based acceptor protein."

A signal peptide that causes a protein to be secreted out of cells to express solubility in cells, an acceptor ubiquitin, a hinge and a Fc gene were designed to be inserted into the pcDNA3.1 (+) vector. The pcDNA3.1 (+) vector is an expression vector for animal cells having a CMV promoter, an ampicillin resistance gene, and the like.

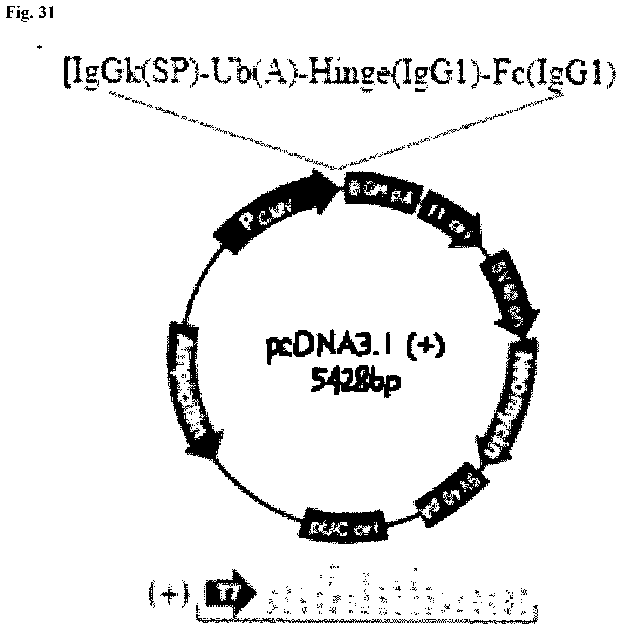

Fig. 31 shows the pcDNA3.1 (+) vector to which a gene expressing an Fc based acceptor protein is linked. The nucleotide sequence and amino acid sequence expressing the Fc based acceptor protein are shown in Tables 3 and 4 below.

[Table 3]

| Nucleotide sequence of IgGκ (SP)-Fc based acceptor protein | | | |
| --- | --- | --- | --- |
| Signal peptide (IgGκ) (SEQ ID NO: 22) | ATGGAAACTG ATACTCTGCT GCTGTGGGTG CTGCTGCTGT GGGTGCCCGG CTCAACTGGT | | |
| Ub (A) acceptor ubiquitin (SEQ ID NO: 23) | ATGCAGATCT | TCGTGAGGAC | CCTGACAGAT |
| | CGGACCATCA | CACTGGAGGT | GGAGCCAAGC |
| | GACACCATCG | AGAACGTGAG | GGCCAGAATC |
| | CAGGACCGGG | AGGGCATCCC | CCCTGATCAG |
| | CAGAGACTGA | TCTTCGCTGG | CCGCCAGCTG |
| | GAGGACGGAA | GGACCCTGAG | CGATTACAAT |
| | ATCCAGAAAG | AGTCTACACT | GCACCTGGTG |
| | CTGAGACCGC GCGTCGTGGA T | | |
| Hinge (IgG1) (SEQ ID NO: 24) | GAGCCAAAAT CTTGTGACAA AACTCATACA TGTCCC | | |

(continued)

| Nucleotide sequence of IgGκ (SP)-Fc based acceptor protein | |
|---|---|
| Fc (IgG1) (SEQ ID NO: 25) | CCATGTCCCG CACCTGAACT GCTGGGCGGA CCTAGCGTGT TTCTGTTCCC ACCTAAGCCA AAGGACACCC TGATGATCTC CAGGACCCCC GAGGTGACAT GCGTGGTGGT GGACGTGAGC CACGAGGACC CCGAGGTGAA GTTCAACTGG TACGTGGATG GCGTGGAGGT GCATAATGCC AAGACAAAGC CAAGGGAGGA GCAGTACAAC AGCACCTATC GGGTGGTGTC TGTGCTGACA GTGCTGCACC AGGACTGGCT GAACGGCAAG GAGTATAAGT GCAAGGTGTC TAATAAGGCC CTGCCCGCTC CTATCGAGAA GACCATCTCC AAGGCCAAGG GCCAGCCAAG AGAGCCCCAG GTGTACACAC TGCCCCCTAG CCGCGACGAG CTGACCAAGA ACCAGGTGTC TCTGACATGT CTGGTGAAGG GCTTCTATCC TTCTGATATC GCTGTGGAGT GGGAGTCCAA TGGCCAGCCA GAGAACAATT ACAAGACCAC ACCACCCGTG CTGGACTCTG ATGGCTCCTT CTTTCTGTAT TCCAAGCTGA CCGTGGATAA GAGCAGATGG CAGCAGGGCA ACGTGTTCTC CTGTAGCGTG ATGCATGAAG CACTGCATAA TCACTATACC CAGAAGTCAC TGTCACTGAG TCCCGGTAAA |

[Table 4]

| Amino acid sequence of IgGκ (SP)-Fc based acceptor protein | |
|---|---|
| Signal peptide (IgGκ) (SEQ ID NO: 26) | Met Glu Thr Asp Thr Leu Leu Leu Trp Val Leu Leu Leu Trp Val Pro Gly Ser Thr Gly |
| Ub (A) acceptor ubiquitin (SEQ ID NO: 27) | Met Gln Ile Phe Val Arg Thr Leu Thr Asp Arg Thr Ile Thr Leu Glu Val Glu Pro Ser Asp Thr Ile Glu Asn Val Arg Ala Arg Ile Gln Asp Arg Glu Gly Ile Pro Pro Asp Gln Gln Arg Leu Ile Phe Ala Gly Arg Gln Leu Glu Asp Gly Arg Thr Leu Ser Asp Tyr Asn IleGlnLysGluSerThrLeuHisLeuValLeuArgProArgValValAsp |
| | |
| Hinge (IgG1) (SEQ ID NO: 28) | Glu Pro Lys Ser Cys Asp Lys Thr His Thr Cys Pro |

(continued)

| Amino acid sequence of IgGκ (SP)-Fc based acceptor protein | |
|---|---|
| Fc (IgG1) (SEQ ID NO: 29) | Pro Cys Pro Ala Pro Glu Leu Leu Gly Gly Pro Ser Val Phe Leu Phe Pro Pro Lys Pro Lys Asp Thr Leu Met Ile Ser Arg Thr Pro Glu Val Thr Cys Val Val Val Asp Val Ser His Glu Asp Pro Glu Val Lys Phe Asn Trp Tyr Val Asp Gly Val Glu Val His Asn Ala Lys Thr Lys Pro Arg Glu Glu Gln Tyr Asn Ser Thr Tyr Arg Val Val Ser Val Leu Thr Val Leu His Gln Asp Trp Leu Asn Gly Lys Glu Tyr Lys Cys Lys Val Ser Asn Lys Ala Leu Pro Ala Pro Ile Glu Lys Thr Ile Ser Lys Ala Lys Gly Gln Pro Arg Glu Pro Gln Val Tyr Thr Leu Pro Pro Ser Arg Asp Glu Leu Thr Lys Asn Gln Val Ser Leu Thr Cys Leu Val Lys Gly Phe Tyr Pro Ser Asp Ile Ala Val Glu Trp Glu Ser Asn Gly Gln Pro Glu Asn Asn Tyr Lys Thr Thr Pro Pro Val Leu Asp Ser Asp Gly Ser Phe Phe Leu Tyr Ser Lys Leu Thr Val Asp Lys Ser Arg Trp Gln Gln Gly Asn Val Phe Ser Cys Ser Val Met His Glu Ala Leu His Asn His Tyr Thr Gln Lys Ser Leu Ser Leu Ser Pro Gly Lys |

The above Fc based acceptor protein recombinant expression vector was obtained.

Fc based acceptor protein plasmid DNA was put into DH5α competent cells, transformed by heat shock treatment at 42 °C for 1 minute, and plated on the LB solid medium containing 100 μg/mL ampicillin. The plated LB solid medium plate was stationary incubated at 37 °C for at least 16 hours to obtain colonies. A single colony was taken, inoculated into 5 mL of LB medium, and then incubated at 37 °C and 220 rpm for 16 hours. A part of the cultured solution was inoculated into LB medium containing ampicillin and then incubated at 37 °C and 220 rpm for 16 hours. The cultured solution was centrifuged at 3,500 rpm for 30 minutes to obtain an E. coli pellet, and then the PI, P2, and P3 solutions of the DNA extraction kit (QIAGEN) were added to break the cell wall, and the proteins were separated to obtain a DNA suspension. A plasmid DNA pellet was obtained from the DNA suspension obtained using a purification column of the DNA extraction kit (QIAGEN) and dried naturally. Water for cell culture (Sigma Aldrich) was added to the dried DNA pellet, dissolved, and then filtered by a 0.22 μm filter. The final extracted plasmid DNA was used for protein expression after measuring the DNA concentration and purity using a nano drop instrument (IMPLEN).

**Example 15: Expression and confirmation identification of Fc based acceptor protein (acceptor protein)**

**Expression of Fc based acceptor protein**

Expi293F human cells are derived from the human embryonic kidney 293 cell line and have high transfection and high protein expression efficiency.

24 hours prior to the transfection process, Expi293F (Gibco) cells were inoculated at 3 x $10^6$ viable cells/mL, placed on an Orbital shaker in an 8 % $CO_2$ incubator, and incubated for 24 hours at 37 °C, 80 % humidity or higher, 95 rpm (a shaking diameter of 50-mm) conditions. The cells were counted to determine the cell viability and the cell number, diluted with the Expi293 expression medium (Gibco) to a final concentration of 3 x $10^6$ viable cells/mL and a total volume of 200 mL, and inoculated into a 1 L Erlenmeyer flask.

200 μg of an Fc based acceptor protein recombinant expression vector DNA was diluted in 12 mL of the Opti-MEM I Reduced Serum Media (Gibco) medium, and 640 μL of the ExpiFectamineTM 293 Reagent (Gibco) was diluted in 11.2 mL of the Opti-MEM I Reduced Serum Media (Gibco). Then, the reaction was carried out at ambient temperature for 5 minutes. The solution containing the ExpiFectamineTM 293 Reagent was put into a solution containing an Fc based acceptor protein recombinant expression vector DNA, mixed, and reacted at ambient temperature for 12 minutes. Transfection was carried out by slowly dispensing it into a 1L flask inoculated to 3 × $10^6$ viable cells/mL. It was placed on an Orbital shaker in an 8 % $CO_2$ incubator and incubated for 18 hours at 37 °C, 80 % humidity or higher, 95 rpm (a shaking diameter of 50-mm) conditions. After 18 hours, 1.2 mL of Enhancer 1 (Gibco) and 12 mL of Enhancer 2 (Gibco) were added, respectively, placed on an Orbital shaker in an 8 % $CO_2$ incubator, and incubated for 7 days at 37 °C, 80 % humidity or higher, 95 rpm (a shaking diameter of 50-mm) conditions.

**Identification of expression of Fc based acceptor protein**

The cultured solution obtained above was centrifuged for at least 30 minutes at a 3,500 rpm condition to obtain only a cultured solution of Fc based acceptor protein expression except for the cell pellet. The obtained cultured solution was filtered through a filter to remove impurities. In order to determine the expression level of an Fc based acceptor protein from the cultured solution, 80 µL was taken, and 20 µL of 5X non-reducing sample loading dye was added thereto, mixed, and let stood at 95 °C for 10 minutes.

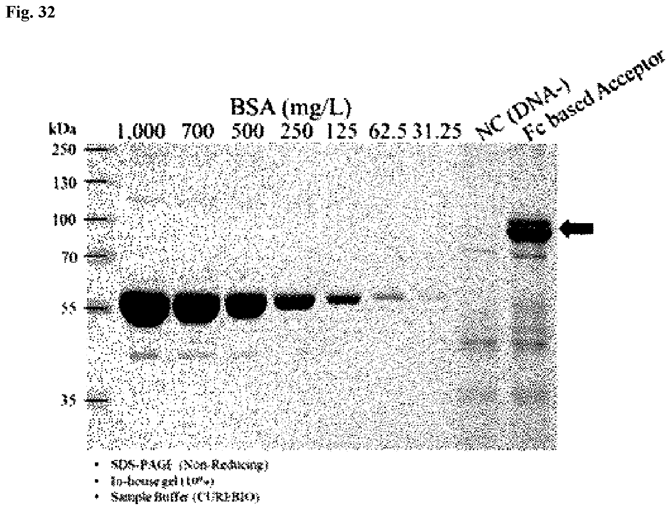

In order to qualitatively analyze the expression level, 80 µL of bovine serum albumin that was diluted to 31.25, 62.5, 125, 250, 500, 750, and 1,000 mg/mL was taken, and 20 µL of 5X non-reducing sample loading dye was added, mixed, and let stood at 95 °C for 10 minutes. Each sample and a marker protein for size check were loaded on a 10 % Tris-glycine gel, and the proteins were separated at 80 volts (V) for about 20 minutes and at 120 volts (V) for 90 minutes. When the gel running was completed, staining was performed with Coomassie brilliant blue R while gently shaking, and the staining reagent was removed from the stained gel using a buffer containing 10 % acetic acid while gently shaking. The decolorized gel was obtained as an image file, and the concentration of the Fc based acceptor protein compared to the amount of the bovine serum albumin protein band was analyzed qualitatively and quantified through the Image J program, and the results are shown in Fig. 32.

As shown in Fig. 32, it was confirmed that an Fc based acceptor protein having a size of 100 kDa was formed, and it was confirmed that 264.1 mg/mL of the protein was formed.

**Confirmation of target specificity of Fc based acceptor protein**

Western blotting was performed to confirm the target specific expression of an Fc based acceptor protein. 80 µL of the expressed cultured solution was taken, and 20 µL of 5X non-reducing sample loading dye was added, mixed, and let stood at 95 °C for 10 minutes. The prepared sample and a marker protein for size check were loaded on a 10 % Tris-glycine gel, and the proteins were separated at 80 volts (V) for about 20 minutes and at 120 volts (V) for 90 minutes. The gel transferred the protein on the polyvinylidene fluoride membrane (PVDF membrane) for about 2 hours at 0.3 amps (A) for electrophoresis. Blocking was performed on the membrane to remove non-specific reactions while gently shaking for 1 hour in 1X PBST (Phosphate Buffer Saline with Tween 20) containing 5% skim milk. Goat anti-rabbit IgG (H+L), which specifically binds to a human IgG Fc antibody, and HRP were used to confirm the Fc based acceptor protein specific expression. The results are shown in Fig. 3. A negative control is a sample that does not contain plasmid DNA.

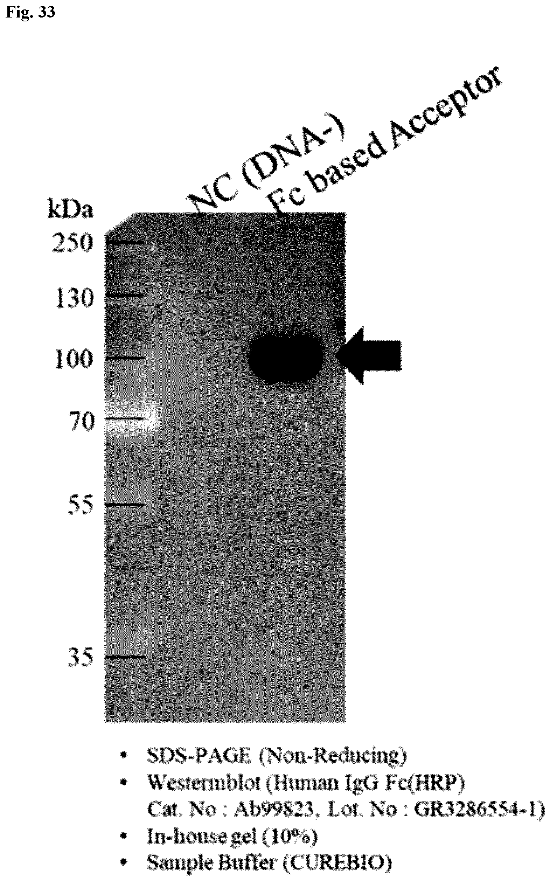

As shown in Fig. 33, the expression of an Fc based acceptor protein that specifically binds to an IgG Fc antibody and has a size of 100 kDa was confirmed.

**Purification of Fc based acceptor protein**

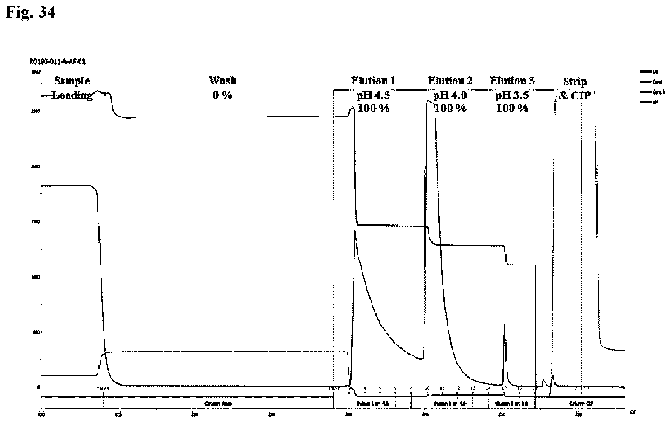

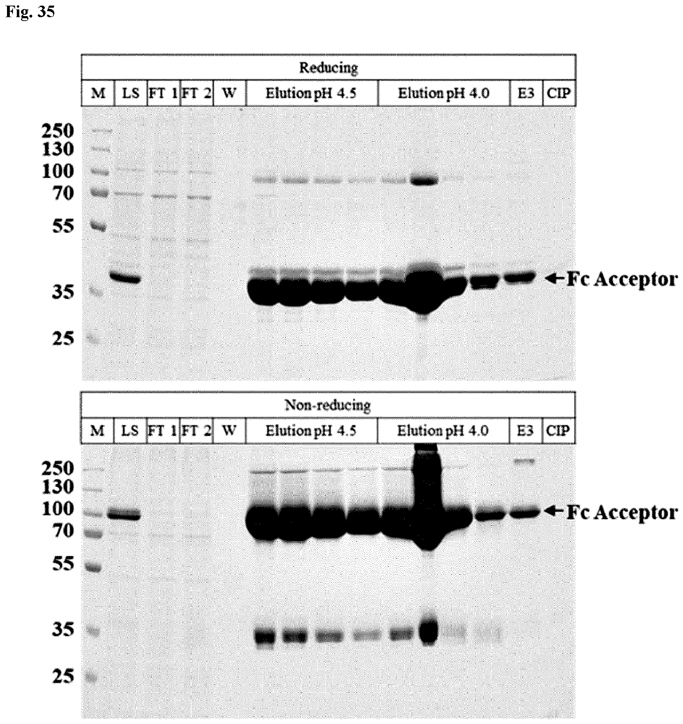

The cultured solution of the Fc based acceptor protein expressed above was loaded on a MabSelect Prism A (Cytiva) column equilibrated with an equilibrium buffer (20 mM sodium phosphate, 150 mM NaCl, pH 7.4). An equlibration buffer used to remove impurities that were not bound to the column, and Elution buffer 1 (50 mM sodium acetate at pH 4.5) and Elution buffer 2 (50 mM sodium acetate at pH 4.0) were used as a step elution to recover the acceptor protein. 1 M Tris was added to the recovered acceptor protein to allow the recovered protein to be at a pH of 7.5. The acceptor protein recovered after pH titration was subjected to dialysis with a 25 mM Tris, pH 7.5 buffer, followed by ultrafiltration. The results of the acceptor protein purification through SDS-PAGE were confirmed using a 10 % in-house gel. The results are shown in Figs. 34 and 35.

**Example 16: Expression and purification of ubiquitin-IL-1RA protein (donor protein)**

**Expression of ubiquitin-IL-1RA protein**

A ubiquitin-biomolecule protein in which a biomolecule is directly bound to the C-terminus of the donor ubiquitin was prepared as follows.

The gene sequence encoding the ubiquitin-biomolecule protein was transformed into a His-SUMO tagged pET21a vector, and then 0.5 µL of a plasmid into which the gene was inserted was put into an E-tube containing 50 µL of *E. coli* BL21 (DE3), a competent cell. Thereafter, it was mixed by tapping and then let stood in ice for 20 minutes. In order to apply a heat shock, the E-tube was let stood in a water bath at 42 °C for 50 seconds and then let stood in ice for 5 minutes. Thereafter, 300 μL of fresh LB medium was added to the E-tube and incubated in a 37 °C shaking incubator for 1 hour to complete transformation. The transformed cells were spread on an LB plate containing ampicillin at a concentration of 100 mg/ml in BSC in a 1/1000 ratio and incubated overnight in a 37 °C stationary incubator. Thereafter, the resulting single colony was taken and inoculated into 100 mL of TB medium containing ampicillin at a concentration of 100 mg/ml in a 1/1000 ratio, and then the seed was cultured at 37 °C and 220 rpm for 6 hours.

In the case of the ubiquitin-IL-IRA protein (Donor, D-192) containing IL-1RA as a biomolecule, the seed cultured solution was inoculated into 3 L of In-house TB medium containing in a 1/1000 ratio ampicillin at a concentration of 100 mg/ml in a 1:100 ratio, and main culture was carried out. Incubation was carried out at 37 °C, dissolved oxygen 40 % for 4 hours using the Biocanvas fermentor (Centrion), and induction of 1 M IPTG stock at a final concentration of 200 μM was carried out. In order to adjust the amount of dissolved oxygen in the cultured solution to 40%, the RPM of the impeller was automatically adjusted to 300 to 700 rpm during the incubation time. After induction, incubation was continued for another 14 hours and the incubation was terminated. The incubated solution was centrifuged at 7000 g for 30 minutes to obtain E. coli wet cells.

The remaining nutrients and optical density were measured after completion of incubation using Cedex BIO Analyzer (Roche). The results are shown in Table 5 below.

[Table 5]

| pH Meter | Acetate (mg/L) | Glucose (mg/L) | Glycerol (mg/L) | Mg$^{2+}$ (mg/L) | P (mg/L) | Optical density |
|---|---|---|---|---|---|---|
| 7.37 | 193.9 | 0.0 | 1190.7 | 56.2 | 6826.9 | 36.7 |

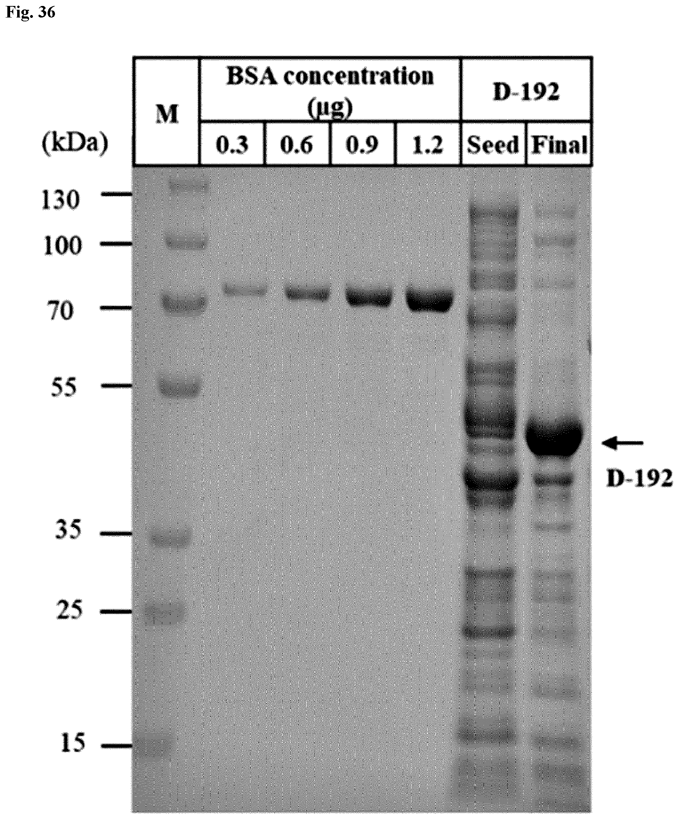

The ubiquitin-IL-1RA was identified through SDS-PAGE analysis method. The results are shown in Fig. 36. In addition, the concentration of the ubiquitin-IL-IRA protein was measured, and the result was analyzed to be 963.32 mg/L.

**Purification of ubiquitin-IL-1RA protein**

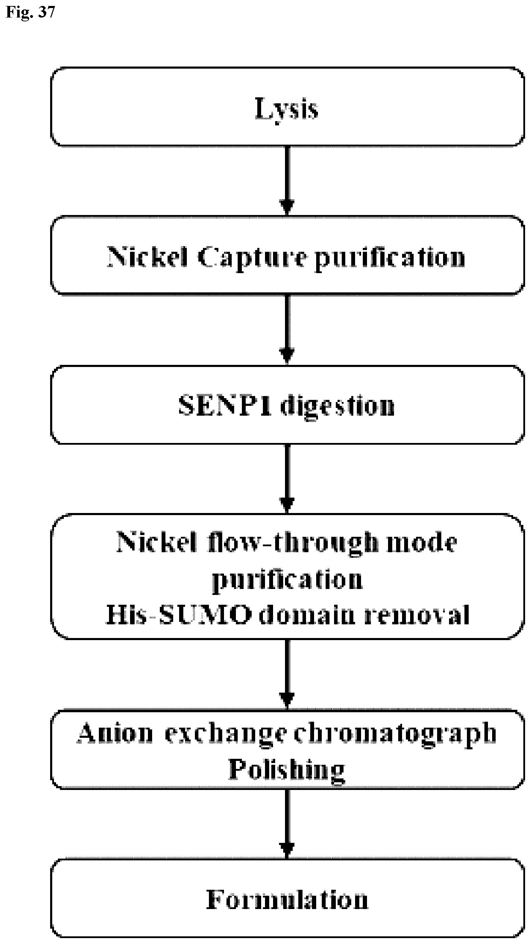

The ubiquitin-IL-IRA protein was purified by the following process (Fig. 37).

(A) Lysis/sonication

Resuspension of wet cells obtained by incubation was carried out using a lysis buffer (20 mM sodium phosphate, pH 7.0). It was carried out by adding 9 mL of a lysis buffer per 1 g of wet cells. Lysis samples were placed on ice, and sonication was carried out for 20 minutes under conditions of Pules on/off = 3 sec/5 sec, 45 % amplitude. The lysate was centrifuged at 14,000 rpm for 30 minutes to obtain only supernatant.

(B) Capture purification

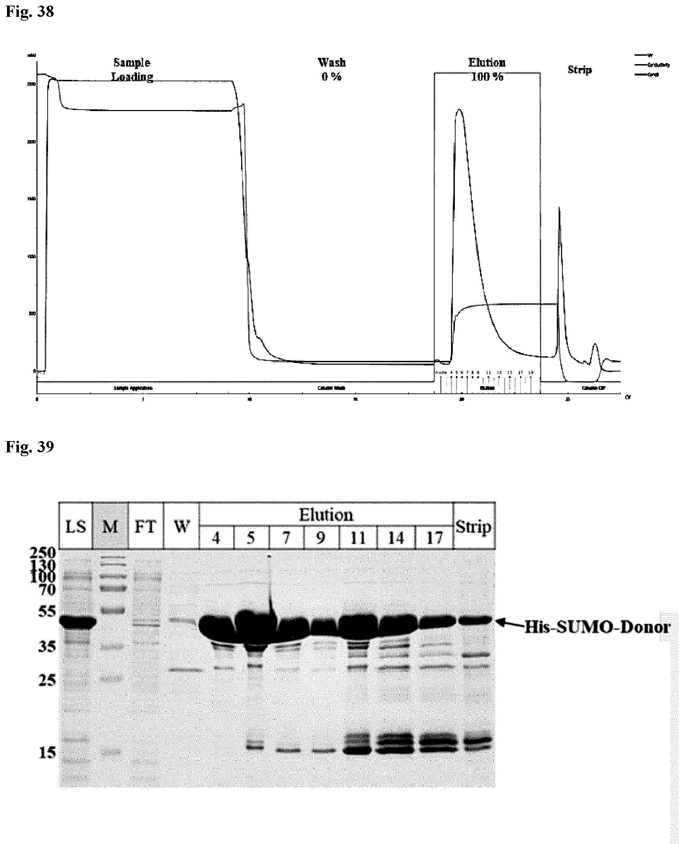

The lysate was loaded on a Ni-sepharose resin (Cytiva). After the sample loading was completed, it was sufficiently washed using a washing buffer (20 mM sodium phosphate, pH 7.0, 0.02 M imidazole) to remove the non-specific protein. Thereafter, the Hig-SUMO tagged ubiquitin-IL-IRA protein was recovered using an elution buffer (20 mM sodium phosphate, pH 7.0, 0.2 M imidazole). The recovered His-SUMO tagged ubiquitin-IL-IRA protein was subjected to dialysis with a 20 mM sodium phosphate, pH 7.0 buffer to remove imidazole. Purification of the donor protein through the Ni column was confirmed by SDS-PAGE. The results are shown in Figs. 38 and 39.

(C) SENP1 enzyme digestion

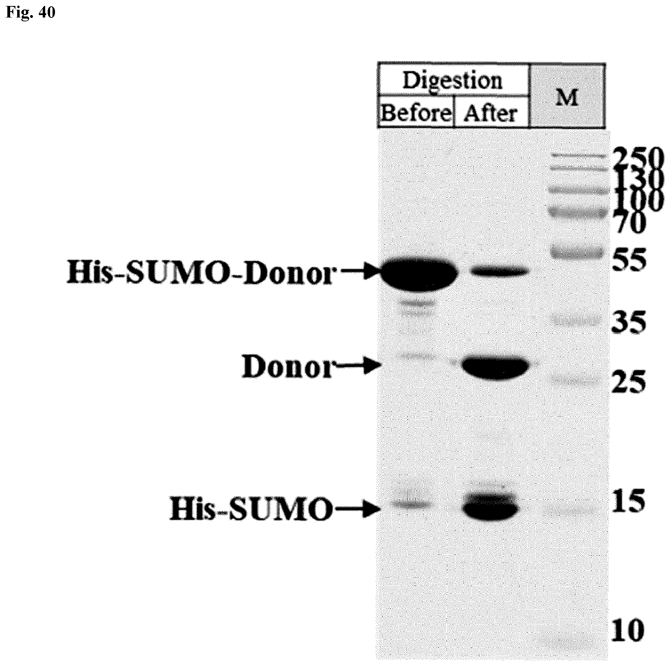

The His-SUMO tagged ubiquitin-IL-IRA protein and SENP1 were subjected to SENP1 enzyme digestion at a ratio of 100 mg of ubiquitin-IL-1RA protein : 1 mg of SENP1. The concentration of the recovered protein was quantified by Ni-purification, and the corresponding recombinant SNEP1 was mixed based on the amount of the His-SUMO tagged ubiquitin-IL-IRA protein. The reaction mixture was let stood at ambient temperature (15 to 25 °C) for 1 hour. The SENP1 enzyme digestion was confirmed by SDS-PAGE. The results are shown in Fig. 40.

(D) Removal of His-SUMO

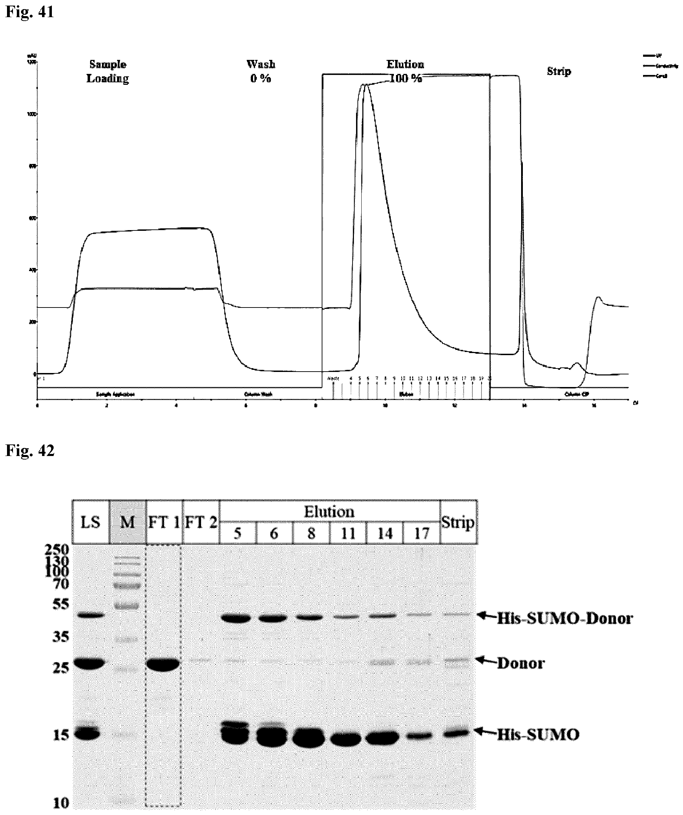

The reaction mixture was loaded on a Ni-sepharose resin (Cytiva) equilibrated with an equilibrium buffer (20 mM sodium phosphate, pH 7.0, 0.02 M imidazole). Sample loading was performed, and the ubiquitin-IL-1RA protein in which His-SUMO tag was cleaved was allowed to flow through. After the sample loading was completed, the remaining ubiquitin-IL-IRA protein was recovered using an equilibrium buffer (20 mM sodium phosphate, pH 7.0, 0.02 M imidazole), and the recovered ubiquitin-IL-IRA protein was subjected to dialysis with a 20 mM sodium phosphate, pH 7.0 buffer to remove imidazole. The process in which His-SUMO is removed was confirmed by SDS-PAGE. The results are shown in Figs. 41 and 42.

(E) Purification

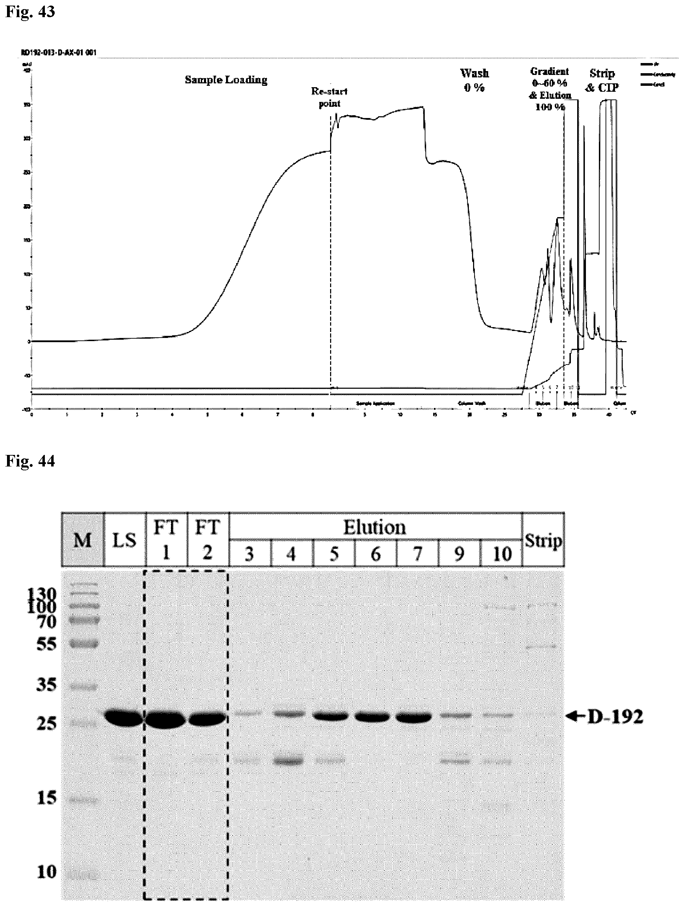

The ubiquitin-IL-IRA protein recovered in the previous step was loaded on an anion exchange resin column equilibrated with an equilibrium buffer (20 mM sodium phosphate, pH 7.0 buffer). The ubiquitin-IL-IRA protein was allowed to flow through. The recovered ubiquitin-IL-1RA protein was subjected to ultrafiltration to a final concentration of 10 mg/mL. The results of the anion exchange resin process for polishing are shown in Figs. 43 and 44.

**Example 17: Conjugation**

**Performance and yield of conjugation**

Conjugation was performed using the acceptor protein produced in Example 18 and the donor protein produced in Example 19. The molar ratio of the acceptor and donor proteins was 1 : 3. At this time, the acceptor protein can be set to 10 $\mu$M to 50 $\mu$M. In addition, E1, E2, E3, and ATP were added to the UniStac mixture to initiate the reaction. The reaction was carried out at 25 °C for 16 hours in a stationary state.

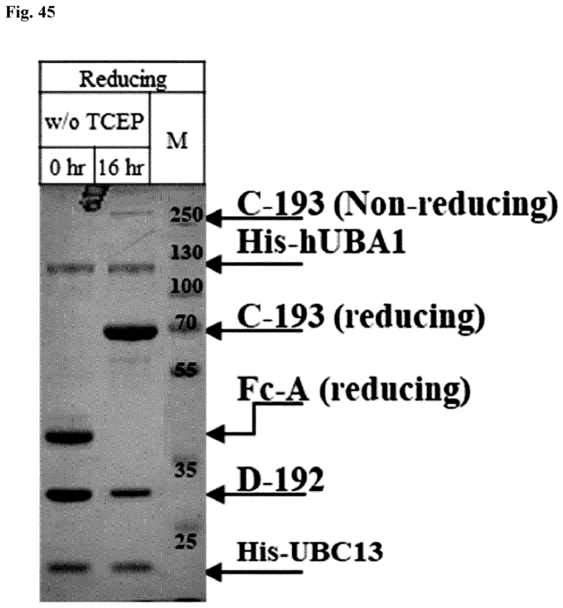

For the resulting product of conjugation reaction (C-193), the degree of conjugation was qualitatively analyzed by loading 1.12 $\mu$g of the acceptor on a 4 to 12 % gradient SDS-PAGE, and the results are shown in Fig. 45.

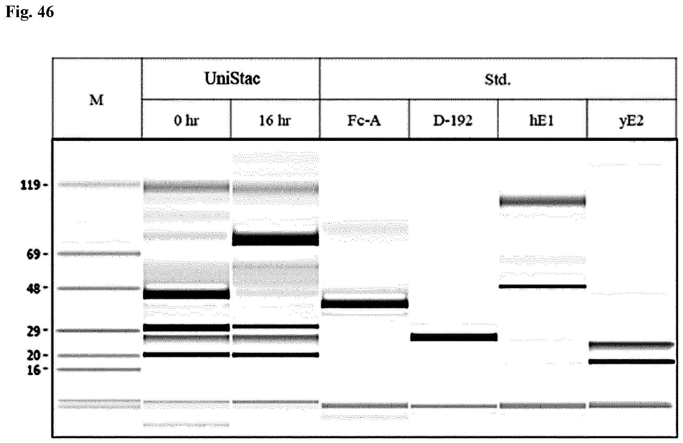

In addition, for the resulting product of the reaction, the degree of conjugation was quantitatively analyzed using $\mu$CE-SDS analysis method. Sample pretreatment was performed using the HT Protein Express Reagent Kit, and then analysis was performed using the Protein Express Assay Labchip, and the yield analysis results are shown in Table 6 below and Fig. 46.

[Table 6]

| Reaction step | Sample | Correlative Area | Conjugation yield (%) |
|---|---|---|---|
| Pre-reaction | acceptor protein | 6092.70 | 97.21 |
| | conjugate (C-193) | 0.00 | |
| Post-reaction | acceptor protein | 169.70 | |
| | conjugate (C-193) | 11150.76 | |

[Equation 1]

$$UniStac\ conjugaiton\ yield\ (\%) = 100 - \left( \frac{After\ Acceptor\ Corr.\ Area}{Before\ Acceptor\ Corr.\ Area} \times 100 \right)$$

The conjugation yield in Table 6 above was calculated using Equation 1 above, and the UniStac conjugation yield ws 97.21 %, indicating a very high acceptor specific value.

**Purification of UniStac conjugation**

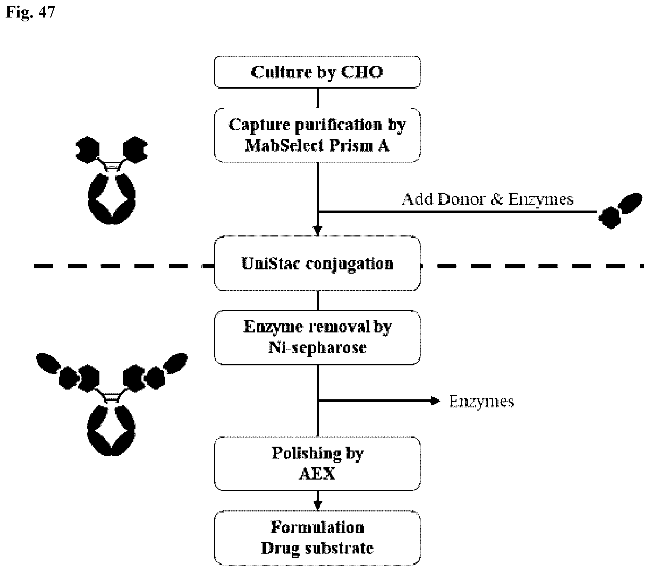

In order to recover only the conjugation (C-193) sample, purification was carried out using the following process (Fig. 47).

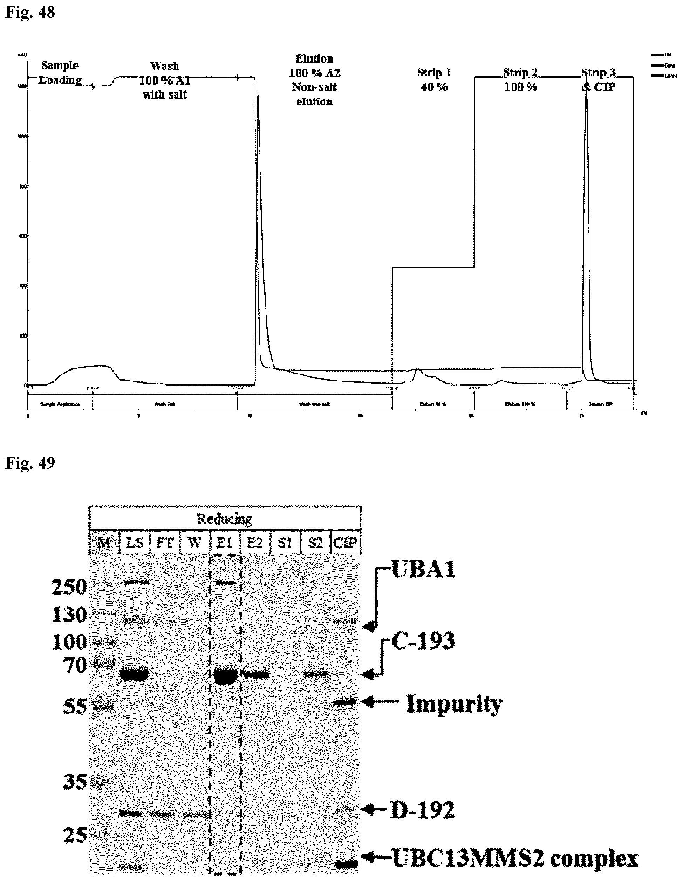

For the resulting product of the reaction, a Ni-sepharose resin equilibrated with an equilibrium buffer (25 mM Tris, pH 8.0, 0.5 M NaCl) was prepared. A small amount of 5 M NaCl was added to the loading sample to adjust the conductivity to 50 mS/cm. After loading the sample prepared with the corresponding conductivity into the prepared column was completed, an equilibrium buffer (25 mM Tris, pH 8.0, 0.5 M NaCl) was used to remove impurities. Thereafter, an elution buffer (25 mM Tris, pH 8.0) was used to recover the conjugation (C-193). The recovered conjugation (C-193) was subjected to dialysis with a pH 7.0 sodium phosphate buffer without salt to remove salt and imidazole. The Ni purification was confirmed through chromatography, and the results are shown in Figs. 48 and 49.

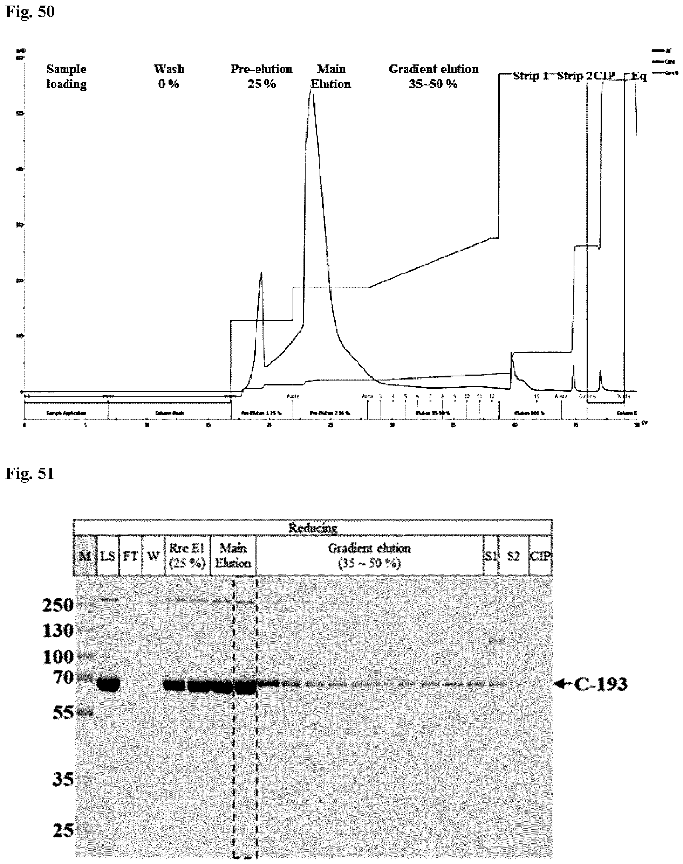

The conjugation was loaded on an anion exchange resin (anion exchange chromatography) column equilibrated with an equilibrium buffer (25 mM sodium phosphate, pH 7.0 buffer). An elution buffer (25 mM sodium phosphate, pH 7.0, 250 mM NaCl) was used to recover the conjugation, and the results are shown in Figs. 50 and 51.

**Final UniStac**

The conjugation (C-193) was subjected to dialysis with a formulation buffer (4.6 mM histidine, 5.7 mM Tris, pH 7.5, 10 mM arginine, 0.1 g/mL trehalose) to perform the formulation. The final UniStac product was prepared by dilution to 1.1 mg/mL and 0.5 mg/mL. The produced samples were stored in a -70 °C deep freezer.

**Example 18: Physicochemical analysis of final UniStac polymer**

In order to measure the purity of the final UniStac polymer (Drug Substance, C-193), SDS-PAGE (reducing and native conditions), μCE-SDS and SEC-HPLC analysis were carried out.

**SDS-PAGE analysis**

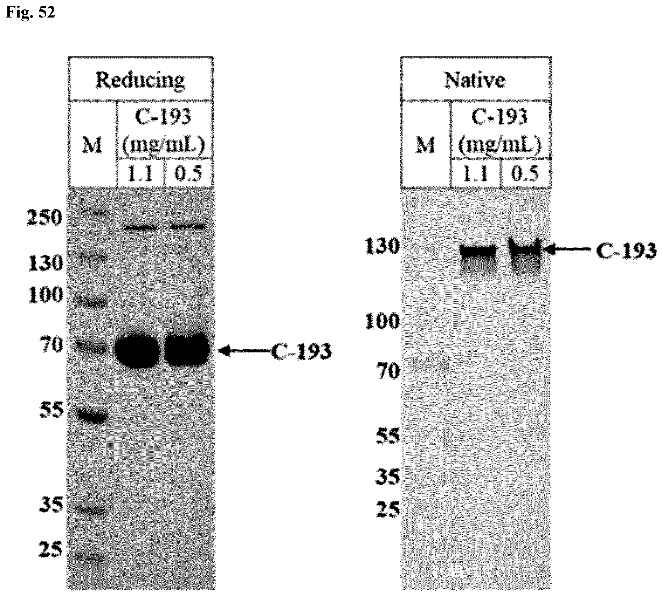

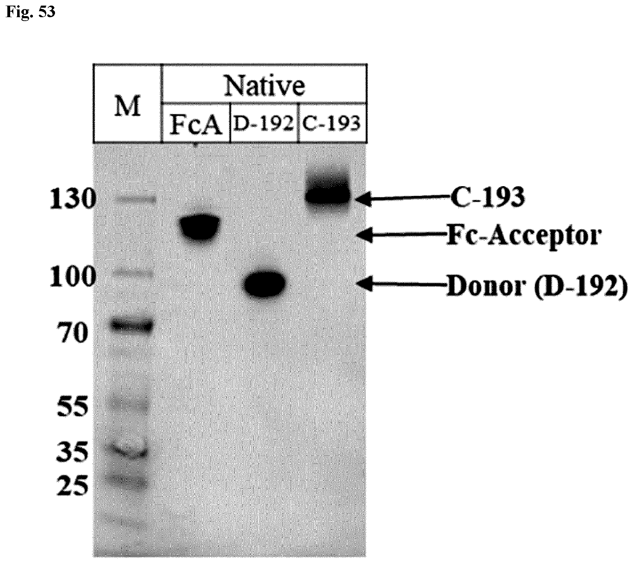

In order to analyze the final conjugate product (DS, C-193) under reducing conditions, reducing SDS-PAGE analysis was carried out using 4-12 % Bis-Tris Plus Gel and MES SDS Running Buffer. 3 μg of the sample was loaded into each of the prepared PAGE, and the results are shown in Fig. 52.
In order to analyze the final conjugate product (C-193 DS) under native conditions, native-PAGE analysis was carried out using 4-15 % T/G-PAG-BC non-SDS and Tris-glycine native Running Buffer. 4.5 μg of the sample was loaded into each of the prepared PAGE, and the results are shown in Fig. 53.

**μCE-SDS analysis**

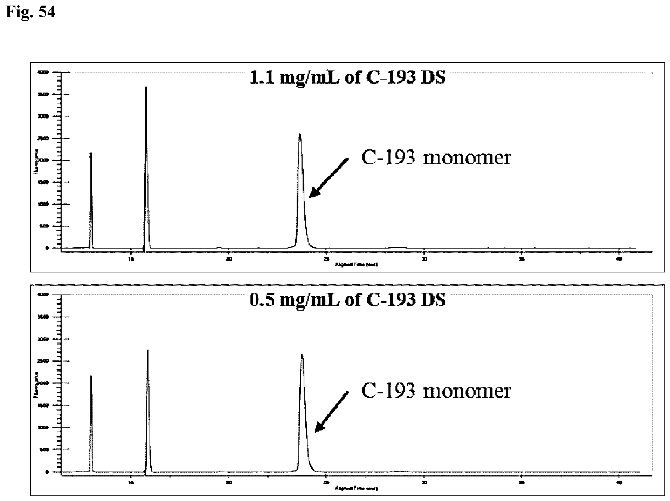

The degree of fragment inclusion in the C-193 DS sample was analyzed using μCE-SDS (Perkin Elmer Labchip GX II Touch.) analysis method. The sample pretreatment was carried out using a HT Protein Express Reagent Kit (Perkin Elmer), and then the analysis was carried out using a Protein Express Assay Labchip (Perkin Elmer). The results are shown in Table 7 below and Fig. 54.

[Table 7]

| Concentration of UniStac sample | Migration time (sec) | Corr. Area | Purity (%) |
|---|---|---|---|
| 1.1 mg/mL | 23.62 | 3527.21 | 98.12 |
| 0.5 mg/mL | 24.56 | 3616.85 | 98.69 |

[Equation 2]

$$\text{Purity (\%)} = \left( \frac{C-193 \; Corr.Area}{\text{Total} \; Corr.Area} \times 100 \right)$$

The purity of the monomer was calculated using Equation 2, and the results are shown in Table 7, confirming that the purity of the monomer was high as 98% or more.

**SEC-HPLC analysis**

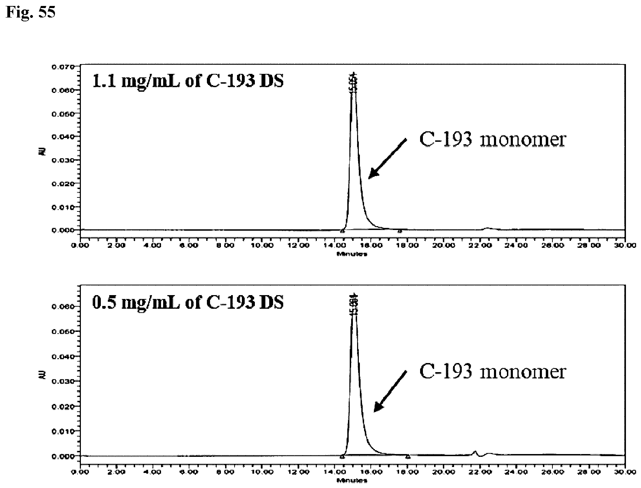

The degree of high molecular weight inclusion in the C-193 DS sample was analyzed using SEC-HPLC column and Alliance e2695 XC HPLC instrument. About 30 μg of the C-193 DS sample was injected into each of the prepared column to perform analysis, and the results are shown in Table 8 below and Fig. 55.

[Table 8]

| Concentration of sample | Retention Time (min) | Area | Purity % |
|---|---|---|---|
| 1.1 mg/mL | 15.051 | 2234084 | 100.00 |
| 0.5 mg/mL | 15.081 | 2264850 | 100.00 |

The purity of the monomer was calculated using Equation 2 above, and the results are shown in Table 8 above, confirming that the purity of the monomer was very high as 100 %.

**Example 19: Comparison of pharmacokinetics of fusion proteins (C-192, C-193 and D-192)**

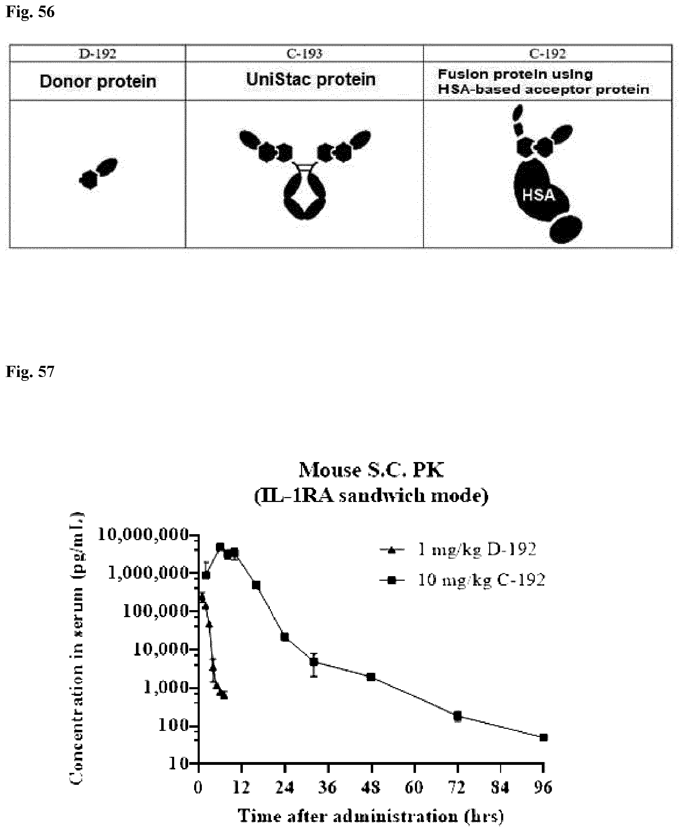

The donor protein (D-192), the UniStac protein (C-193), and the fusion protein (C-192) using an acceptor protein using a human-serum albumin as a carrier were prepared. The structures of the three samples are shown in Fig. 56 below.

9-week-old male ICR mice were subcutaneously administered once with the three samples, and then the blood was collected for each time period, and a pharmacokinetics measurement test was carried out to analyze the blood drug concentration and calculate the pharmacokinetic parameters.

8-week-old male ICR mice purchased from Orient BIO, Korea were quarantined and acclimatized for 7 days. After quarantine and acclimatization, body weights were ranked for all animals, and group separation (n=2 per blood collection time) was performed so that the average body weight of each group was uniformly distributed. Thereafter, the C-192 test substance at 10 mg/kg, the C-193 test substance at 1 mg/kg, and the D-192 test substance at 1 mg/kg dose were subcutaneously administered once to each mouse. Blood collection time points were before administration, 2, 6, 8, 10, 16, 32, 24, 48, 72 and 96 hours after administration of C-192, 0.5, 1, 2, 4, 6, 8, 10, 16, 24, 32, 40, 48, 56 and 72 hours after administration of C-193, and 1, 2, 3, 4, 5 and 6 hours after administration of D-192, respectively. The serum was separated from the collected blood by centrifugation and stored at -70 ± 10 °C for analysis of blood drug concentration.

In order to measure the blood drug concentration of the test substance, the human IL-1RA ELISA kit (Abcam, UK) having a specific reactivity to IL-1RA was used. First, 50 $\mu$L of standard material and serum for each time period were dispensed into a 96 well plate, and then 50 $\mu$L of the antibody cocktail provided by the human IL-1RA ELISA kit was dispensed into each well, and the reaction was carried out in a 25 °C mixing device (Thermo Micromixer) at 400 rpm for 1 hour. The solution in the plate well was discarded and shaken so as not to leave any residue. 300 $\mu$L of the washing solution was dispensed into each well, and then the procedure of discarding and shaking was repeated three times. 100 $\mu$L of the color former was dispensed into each well, and then the reaction was carried out in a 25 °C mixing device at 400 rpm 10 minutes.

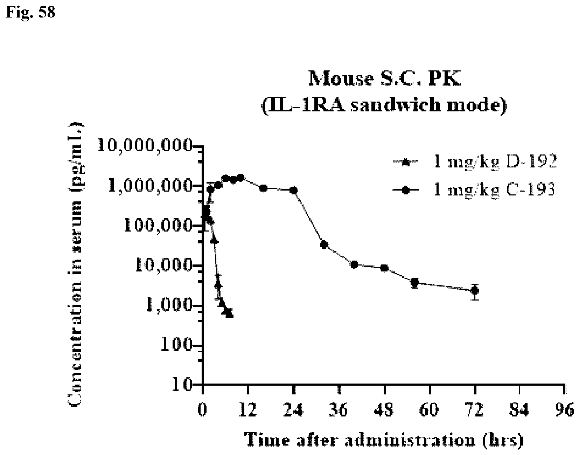

Finally, 100 $\mu$L of the stop solution was dispensed into each well, and then absorbance was measured at 450 nm using an absorbance measuring instrument (Multi-Mode Microplate Reader). The concentration of the drug in the serum for each time period calculated relative to the standard material was used to calculate the pharmacokinetic parameters. Figs. 57 and 58 show graphs of blood drug concentrations over time after subcutaneous administration of the fusion proteins (C-192, C-193 and D-192) in mice.

In addition, the pharmacokinetic parameters were calculated based on the experimental results, and the results are shown in Table 9 below.

[Table 9]

| PK parameter | C-192 | C-193 | D-192 |
|---|---|---|---|
|  | 10 mg/kg | 1 mg/kg | 1 mg/kg |
| $T_{max}$ (hr) | 6 | 10 | 1 |
| Cmax (pg/mL) | 4,853,634 | 1,696,762 | 250,240 |
| AUC (inf) (pg*hr/mL) | 37,378,687 | 28,185,225 | 426,747 |
| Half life (hr) | 9.3 | 13.7 | 0.4 |

As shown in Table 9 above, when the three fusion proteins were compared and evaluated based on the half-life indicating the in vivo stability of the drug, it was confirmed that the half-life of the fusion protein (C-192) using a human serum albumin as a carrier was increased by about 23 times from 0.4 hours to 9.3 hours compared to the acceptor protein (D-192).

In addition, it was confirmed that the half-life of the fusion protein (C-193) using Fc as a carrier was increased by about 33 times from 0.4 hours to 13.7 hours compared to the acceptor protein (D-192).

In the end, the fusion protein in which albumin and Fc carrier are fused has excellent pharmacokinetic properties by increasing half-life, AUC, Tmax, and Cmax, and can be utilized as an excellent drug that can be applied to a desired site with a small dose.

## Claims

1. A multifunctional multispecific multimeric biomolecule polymer, comprising a polyubiquitin scaffold which is formed by covalently bonding two or more ubiquitins, and 2 to 10 biomolecules comprising binding moieties, each specific for different binding sites, wherein the biomolecule comprises active sites that specifically bind to other biomolecules, small molecule chemical compounds or nanoparticles, and is directly bound to the N-terminus, the C-terminus, or both the N-terminus and the C-terminus of the ubiquitin or is bound by a linker, and a carrier that prolongs the in vivo stability and duration of the biomolecule is directly bound to the N-terminus, the C-terminus, or both the N-terminus and the C-terminus of the ubiquitin or is bound by a linker.

2. The multifunctional multispecific multimeric biomolecule polymer according to claim 1, wherein the linker is a combination of 1 to 30 repeats of GGGGS or EAAAK

3. The multifunctional multispecific multimeric biomolecule polymer according to claim 1 or 2, wherein the biomolecule bound to the N-terminus of the ubiquitin is the distal end of the multimeric biomolecule polymer.

4. The multifunctional multispecific multimeric biomolecule polymer according to claim 1 or 2, wherein the biomolecule bound to the C-terminus, the N-terminus, or both the C-terminus and the N-terminus of the ubiquitin is the proximal end of the multimeric biomolecule polymer.

5. The multifunctional multispecific multimeric biomolecule polymer according to claim 1 or 2, wherein the carrier is one or more selected from the group consisting of albumin, antibody fragment, Fc domain, transferrin, XTEN (genetic fusion of non-exact repeat peptide sequence), CTP (carboxy-terminal peptide), PAS (proline-alanine-serine polymer), ELK (elastin-like peptide), HAP (homo-amino acid polymer), GLK (gelatin-like protein), PEG (polyethylene glycol), and fatty acid.

6. The multifunctional multispecific multimeric biomolecule polymer according to claim 1 or 2, wherein the polyubiquitin scaffold is formed by covalently bonding a donor ubiquitin in which one or more lysines of the ubiquitin are substituted with other amino acids including arginine or alanine, and an acceptor ubiquitin in which the 6th, 11th, 27th, 29th, 33rd, 48th, or 63rd lysine from the N-terminus is substituted with other amino acids including arginine or alanine.

7. The multifunctional multispecific multimeric biomolecule polymer according to claim 1 or 2, wherein the 73rd leucine from the N-terminus of the ubiquitin is substituted with other amino acids including proline.

8. The multifunctional multispecific multimeric biomolecule polymer according to claim 1 or 2, wherein the biomolecule is selected from the group consisting of insulin, insulin analogue, glucagon, glucagon-like peptides, GLP-1 and glucagon dual agonist, GLP-1 and GIP dual agonist, GLP-1 and glucagon and GIP triple agonist, exendin-4, exendin-4 analogue, insulin secreting peptide and an analogue thereof, human growth hormone, growth hormone releasing hormone (GHRH), growth hormone releasing peptide, granulocyte colony stimulating factor (G-CSF), anti-obesity peptide, G-protein-coulped receptor, leptin, GIP (gastric inhibitory polypeptide), interleukins, interleukin receptors, interleukin binding proteins, interferons, interferon receptors, cytokine binding proteins, macrophage activator, macrophage peptide, B cell factor, T cell factor, suppressive factor of allergy, cell necrosis glycoprotein, immunotoxin, lymphotoxin, tumor necrosis factor (TNF), tumor inhibitory factor, metastasis growth factor, alpha-1 antitrypsin, albumin, $\alpha$-lactalbumin, apolipoprotein-E, erythropoietin (EPO), high glycosylated erythropoietin, angiopoietins, hemoglobin, thrombin, thrombin receptor activating peptide, thrombomodulin, blood factors VII, VIIa, VIII, IX, and XIII, plasminogen activator, fibrin-binding peptide, urokinase, streptokinase, hirudin, protein C, C-reactive protein, renin inhibitor, collagenase inhibitor, superoxide dismutase, platelet derived growth factor, epithelial growth factor, epidermal growth factor, angiostatin, angiotensin, bone morphogenetic growth factor, bone morphogenetic protein, calcitonin, atriopeptin, cartilage inducing factor, elcatonin, connective tissue activator, tissue factor pathway inhibitor, follicle stimulating hormone (FSH), luteinizing hormone (LH), luteinizing hormone releasing hormone (LHRH), nerve growth factors, parathyroid hormone (PTH), relaxin, secretin, somatomedin, adrenal cortical hormone, cholecystokinin, pancreatic polypeptide, gastrin releasing peptide, corticotropin releasing factor, thyroid stimulating hormone (TSH), autotaxin, lactoferrin, myostatin, receptor, receptor antagonist, fibroblast growth factor, adiponectin, interleukin receptor antagonist, cell surface antigen, virus derived vaccine antigen, monoclonal antibody, polyclonal antibody and antibody fragments.

9. A method for preparing a multifunctional multispecific multimeric biomolecule polymer, in which a polyubiquitin scaffold, two or more biomolecules comprising binding moieties, each specific for different binding sites, and a carrier that prolongs the in vivo duration are directly bound to the N-terminus or the C-terminus of the ubiquitin or are bound by a linker, wherein the method comprises

(i) recombinantly expressing a biomolecule to which a ubiquitin C-terminal tag is fused or bound by a linker from a host cell including a procaryotic cell or a eukaryotic cell, and
(ii) adding E1, E2 and E3 enzymes, or E1 and E2 enzymes for ubiquitination to the cell lysates or purified products of the host cell and reacting them,

wherein the polyubiquitin scaffold is formed by covalently bonding two or more ubiquitins, and
the biomolecule is composed of 2 to 10 biomolecules, has active sites that specifically bind to other biomolecules, small molecule chemical compounds or nanoparticles, and is bound to the N-terminus, the C-terminus, or both the N-terminus and the C-terminus of the ubiquitin by a linker.

10. The method according to claim 9, wherein the E2 enzyme binds to the 6th, 11th, 27th, 29th, 33rd, 48th or 63rd lysine from the N-terminus of the ubiquitin.

11. The method according to claim 9 or 10, wherein the E2 enzyme is an E2-25K ubiquitin conjugating enzyme.

12. The method according to claim 9 or 10, wherein the E2 enzyme is Ucb13-MMS2, a ubiquitin conjugating enzyme complex.

13. The method according to claim 9 or 10, wherein the ubiquitin C-terminal tag is one in which two or more ubiquitins are repeatedly linked in a head-to-tail form or in a branched form (branched type or iso-peptide branch type form).

14. The method according to claim 13, wherein the ubiquitin linked in a head-to-tail form or in a branched form is one in which the 75th and 76th glycines from the N-terminus are substituted with other amino acids including valine.

Fig. 1

Fig. 2

Fig. 3

Fig. 4

Fig. 5

Fig. 6

**(a)**

● Charge patches: 8 residues
✺ Topology: 7 residues

Linker: Charge, Stiffness

**(b)**

UniStac
Mix

UniStac Scaffold

**Fig. 7**

Fig. 8

Fig. 9

Fig. 10

Fig. 11

TIM
(Triose-phosphate isomerase)
Mw. 47.6kDa

Fig. 12

Fig. 13

Fig. 14

Fig. 15

| | XR | UniStaced XR |
|---|---|---|
| KFast | 0.002691 | 0.02680 |

Fig. 16

## XR pH stability

Fig. 17

| | OAC | UniStaced OAC |
|---|---|---|
| KFast | 0.005106 | 0.04471 |

Fig. 18

OAC pH stability

Fig. 19

XDH vs UniStaced XDH

| | XDH | UniStaced XDH |
|---|---|---|
| KFast | 3.413e-006 | 0.03844 |

Fig. 20

XDH pH stability

Fig. 21

**POPG vs Unistaced POPG**

|  | POPG | UniStaced POPG |
|---|---|---|
| KFast | 0.004217 | 0.008603 |

Fig. 22

**UniStac Polymer**

Fig. 23

**Enzyme complex synergistic effect (TIM + ALD +FBP)**

Legend:
- Monomer enzyme complex mixture
- UniStac enzyme complex polymer

**Fig. 24a**

Protein G-Ub
(41.7kDa)

Stacking gel

Linear Protein G Polymer

Linear Protein G Polymer

250k

E1

55k

E3

Protein G

35k

E2

| M | − | + |
|---|---|---|
| | UniStac Mix | |

**Fig. 24b**

Fig. 25

hGHK48_D77
(human growth hormone)
Mw: 43096.61

**Fig. 26a**

| UniStac Mix | + | + | - | M |
|---|---|---|---|---|
| Time (hr) | 20 | 1 | - | |

**Fig. 26b**

| M | - | + | + | + | + | + | + | UniStac Mix |
|---|---|---|---|---|---|---|---|---|
| | - | 0.5 | 1 | 2 | 3 | 4 | 20 | Time (hr) |

Fig. 27a

hGH Unistac

**Fig. 27b**

hGH E2-Unistac

**Fig. 28a**

Fig. 28b

| Sample | | Band intensity | Relative intensity |
|---|---|---|---|
| UniStac mix | + | 37479.0 | 1.16 |
| | - | 32150.1 | 1 |

Fig. 29a

**Fig. 29b**

Unistac type-2

Fig. 29c

Unistac type-3

| M | A>D | A>D | A=D | A<D | A: Acceptor<br>D: Donor |
|---|---|---|---|---|---|
| | − | + | + | + | UniStac Mix |

**Fig. 30**

Plasma Concentration-time Profiles of Test Substance in SD Rats (N=3).

- OGB1 : Di-Ub-Albumin
- OGB3 : Human serum albumin

Summary of Pharmacokinetics Parameter

| Group / Dose (mg/kg) | | $AUC_{all}$ (ng·hr/mL) | $C_{max}$ (ng/mL) | $T_{max}$ (hr) | $t_{1/2}$ (hr) |
|---|---|---|---|---|---|
| G2 | Mean | 282,470.33 * | 7,769.81 * | 16 | 23.73 |
| OGB1: 0.833 | S.D | 35,191.94 | 1,597.53 | 6.9 | 3.35 |
| | N | 3 | 3 | 3 | 3 |
| G3 | Mean | 155,737.74 | 3,855.00 | 20 | 23.01 |
| OGB3: 1 | S.D | 1,909.85 | 189.59 | 6.9 | 6.59 |
| | N | 3 | 3 | 3 | 3 |

Significantly different from Group 3 by Aspin-Welch t-test: * $p<0.05$.

**Fig. 31**

[IgGk(SP)-Ub(A)-Hinge(IgG1)-Fc(IgG1)]

**Fig. 32**

**Fig. 33**

- SDS-PAGE (Non-Reducing)
- Westernblot (Human IgG Fc(HRP)
  Cat. No : Ab99823, Lot. No : GR3286554-1)
- In-house gel (10%)
- Sample Buffer (CUREBIO)

Fig. 34

**Fig. 35**

Fig. 36

Fig. 37

```
┌─────────────────────────────────────────┐
│                  Lysis                   │
└─────────────────────────────────────────┘
                    │
                    ▼
┌─────────────────────────────────────────┐
│        Nickel Capture purification       │
└─────────────────────────────────────────┘
                    │
                    ▼
┌─────────────────────────────────────────┐
│             SENP1 digestion              │
└─────────────────────────────────────────┘
                    │
                    ▼
┌─────────────────────────────────────────┐
│       Nickel flow-through mode           │
│              purification                │
│       His-SUMO domain removal            │
└─────────────────────────────────────────┘
                    │
                    ▼
┌─────────────────────────────────────────┐
│    Anion exchange chromatograph          │
│              Polishing                   │
└─────────────────────────────────────────┘
                    │
                    ▼
┌─────────────────────────────────────────┐
│              Formulation                 │
└─────────────────────────────────────────┘
```

Fig. 38

Fig. 39

Fig. 40

Fig. 41

Fig. 42

Fig. 43

Fig. 44

Fig. 45

Fig. 46

Fig. 47

Culture by CHO

Capture purification by
MabSelect Prism A

Add Donor & Enzymes

UniStac conjugation

Enzyme removal by
Ni-sepharose

Enzymes

Polishing by
AEX

Formulation
Drug substrate

Fig. 48

Fig. 49

Fig. 50

Fig. 51

Fig. 52

Fig. 53

Fig. 54

Fig. 55

Fig. 56

| D-192 | C-193 | C-192 |
|---|---|---|
| **Donor protein** | **UniStac protein** | **Fusion protein using HSA-based acceptor protein** |
| | | |

Fig. 57

**Mouse S.C. PK (IL-1RA sandwich mode)**

Fig. 58

Mouse S.C. PK
(IL-1RA sandwich mode)

## INTERNATIONAL SEARCH REPORT

| International application No. |
| --- |
| **PCT/KR2020/017029** |

### A. CLASSIFICATION OF SUBJECT MATTER

**C07K 14/47**(2006.01)i; **C07K 1/14**(2006.01)i; **C12P 21/06**(2006.01)i; **C12N 9/10**(2006.01)i

According to International Patent Classification (IPC) or to both national classification and IPC

### B. FIELDS SEARCHED

Minimum documentation searched (classification system followed by classification symbols)

C07K 14/47(2006.01); C07K 1/04(2006.01); C07K 14/00(2006.01); C07K 14/725(2006.01); C07K 16/00(2006.01); C07K 16/18(2006.01); C07K 17/00(2006.01); C12N 9/64(2006.01); C12Q 1/37(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 유비퀴틴(ubiquitin), 폴리유비퀴틴(polyubiquitin), 결합 모이어티(binding moieties), 생체분자(biomolecule), 캐리어(carrier), 생체 내 지속시간(in vivo half-life)

### C. DOCUMENTS CONSIDERED TO BE RELEVANT

| Category* | Citation of document, with indication, where appropriate, of the relevant passages | Relevant to claim No. |
| --- | --- | --- |
| X | KR 10-2014-0142293 A (GENENTECH, INC.) 11 December 2014 (2014-12-11)<br>See claims 1-10, 22-25 and 34-50; paragraphs [0006]-[0008], [0026], [0067], [0116], [0118], [0136], [0138], [0139], [0370] and [0453]; and figures 13 and 32. | 1-14 |
| A | WO 2019-007869 A1 (GLAXOSMITHKLINE INTELLECTUAL PROPERTY DEVELOPMENT LIMITED) 10 January 2019 (2019-01-10)<br>See entire document. | 1-14 |
| A | WO 2009-009773 A1 (THE JOHNS HOPKINS UNIVERSITY) 15 January 2009 (2009-01-15)<br>See entire document. | 1-14 |
| A | US 2013-0267680 A1 (OVAA, H. et al.) 10 October 2013 (2013-10-10)<br>See entire document. | 1-14 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **15 March 2021** | **15 March 2021** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
|---|
| **PCT/KR2020/017029** |

**C.    DOCUMENTS CONSIDERED TO BE RELEVANT**

| Category* | Citation of document, with indication, where appropriate, of the relevant passages | Relevant to claim No. |
|---|---|---|
| A | RENZ, C. et al. The ubiquitin-conjugating enzyme Ubc13-Mms2 cooperates with a family of FYVE-type -RING ubiquitin protein ligases in K63-polyubiquitylation at internal membranes. bioRxiv. electronic publication 02 March 2019, inner pp. 1-43.<br>   See entire document. | 1-14 |
| A | KR 10-2008-0080635 A (GENENTECH, INC.) 04 September 2008 (2008-09-04)<br>   See entire document. | 1-14 |

Form PCT/ISA/210 (second sheet) (July 2019)

**INTERNATIONAL SEARCH REPORT**

International application No.

**PCT/KR2020/017029**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a.  ☑  forming part of the international application as filed:

        ☑  in the form of an Annex C/ST.25 text file.

        ☐  on paper or in the form of an image file.

    b.  ☐  furnished together with the international application under PCT Rule 13*ter*.1(a) for the purposes of international search only in the form of an Annex C/ST.25 text file.

    c.  ☐  furnished subsequent to the international filing date for the purposes of international search only:

        ☐  in the form of an Annex C/ST.25 text file (Rule 13*ter*.1(a)).

        ☐  on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (July 2019)

| INTERNATIONAL SEARCH REPORT | | | International application No. | | |
|---|---|---|---|---|---|
| Information on patent family members | | | **PCT/KR2020/017029** | | |

| Patent document cited in search report | | | Publication date (day/month/year) | Patent family member(s) | | | Publication date (day/month/year) |
|---|---|---|---|---|---|---|---|
| KR | 10-2014-0142293 | A | 11 December 2014 | CA | 2866835 | A1 | 19 September 2013 |
| | | | | CN | 104254541 | A | 31 December 2014 |
| | | | | EP | 2825549 | A1 | 21 January 2015 |
| | | | | EP | 2825549 | B1 | 10 October 2018 |
| | | | | HK | 1205524 | A1 | 18 December 2015 |
| | | | | JP | 2015-520730 | A | 23 July 2015 |
| | | | | JP | 6198807 | B2 | 20 September 2017 |
| | | | | KR | 10-2012-0014154 | A | 16 February 2012 |
| | | | | MX | 2014010943 | A | 26 November 2014 |
| | | | | RU | 2014141617 | A | 10 May 2016 |
| | | | | US | 2013-0281314 | A1 | 24 October 2013 |
| | | | | US | 9139863 | B2 | 22 September 2015 |
| | | | | WO | 2013-137920 | A1 | 19 September 2013 |
| WO | 2019-007869 | A1 | 10 January 2019 | BR | 112019028024 | A2 | 07 July 2020 |
| | | | | CA | 3068365 | A1 | 10 January 2019 |
| | | | | CN | 110997710 | A | 10 April 2020 |
| | | | | EP | 3649149 | A1 | 13 May 2020 |
| | | | | GB | 2568987 | A | 05 June 2019 |
| | | | | JP | 2020-529970 | A | 15 October 2020 |
| | | | | US | 10683360 | B2 | 16 June 2020 |
| | | | | US | 2019-0002578 | A1 | 03 January 2019 |
| | | | | US | 2020-0283538 | A1 | 10 September 2020 |
| WO | 2009-009773 | A1 | 15 January 2009 | None | | | |
| US | 2013-0267680 | A1 | 10 October 2013 | EP | 2616479 | A1 | 24 July 2013 |
| | | | | WO | 2012-036551 | A1 | 22 March 2012 |
| KR | 10-2008-0080635 | A | 04 September 2008 | AR | 057237 | A1 | 21 November 2007 |
| | | | | AU | 2006-342025 | A1 | 25 October 2007 |
| | | | | AU | 2006-342025 | A2 | 26 June 2008 |
| | | | | AU | 2006-342025 | B2 | 31 January 2013 |
| | | | | AU | 2006-342025 | C1 | 08 August 2013 |
| | | | | BR | PI0620697 | A2 | 22 November 2011 |
| | | | | CA | 2633887 | A1 | 25 October 2007 |
| | | | | CA | 2633887 | C | 22 December 2015 |
| | | | | CL | 2013002738 | A1 | 21 February 2014 |
| | | | | CN | 101374862 | A | 25 February 2009 |
| | | | | CN | 101374862 | B | 16 December 2015 |
| | | | | CN | 103396485 | A | 20 November 2013 |
| | | | | CN | 103396485 | B | 10 August 2016 |
| | | | | EP | 1973948 | A2 | 01 October 2008 |
| | | | | EP | 1973948 | B1 | 11 February 2015 |
| | | | | EP | 2325208 | A1 | 25 May 2011 |
| | | | | EP | 2325208 | B1 | 20 September 2017 |
| | | | | EP | 3309170 | A1 | 18 April 2018 |
| | | | | EP | 3309170 | B1 | 22 May 2019 |
| | | | | ES | 2535856 | T3 | 18 May 2015 |
| | | | | HK | 1121471 | A1 | 24 April 2009 |
| | | | | IL | 191617 | A | 11 February 2009 |
| | | | | IL | 191617 | B | 30 April 2017 |
| | | | | IL | 251562 | A | 29 May 2017 |
| | | | | JP | 2009-519716 | A | 21 May 2009 |

Form PCT/ISA/210 (patent family annex) (July 2019)

**INTERNATIONAL SEARCH REPORT**
Information on patent family members

International application No.

**PCT/KR2020/017029**

| Patent document cited in search report | Publication date (day/month/year) | Patent family member(s) | | | Publication date (day/month/year) |
|---|---|---|---|---|---|
| | | JP | 2013-220104 | A | 28 October 2013 |
| | | JP | 5358187 | B2 | 04 December 2013 |
| | | JP | 6143538 | B2 | 07 June 2017 |
| | | KR | 10-1538521 | B1 | 22 July 2015 |
| | | NO | 20083127 | A | 15 September 2008 |
| | | NO | 20083127 | L | 15 September 2008 |
| | | RU | 2008128134 | A | 20 January 2010 |
| | | TW | 200808823 | A | 16 February 2008 |
| | | TW | I395753 | B | 11 May 2013 |
| | | US | 2007-0218069 | A1 | 20 September 2007 |
| | | US | 2010-0267050 | A1 | 21 October 2010 |
| | | US | 2014-0179905 | A1 | 26 June 2014 |
| | | US | 7763245 | B2 | 27 July 2010 |
| | | US | 8603475 | B2 | 10 December 2013 |
| | | US | 9487578 | B2 | 08 November 2016 |
| | | WO | 2007-120334 | A2 | 25 October 2007 |
| | | WO | 2007-120334 | A3 | 29 May 2008 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 504785
- KR 1020050050824
- KR 1020150120852
- US 249334

**Non-patent literature cited in the description**

- **LI C ; WEN A ; SHEN B ; LU J ; HUANG Y ; CHANG Y.** Fast cloning: a highly simplified, purification-free, sequence- and ligation-independent PCR cloning method. *BMC Biotechnol,* 2011, vol. 11, 92

<110>     ONEGENE BIOTECHNOLOGY INC.

<120>     A multivalent multispecific multimeric biomolecule having
          prolonged in vivo half-life

<130>     OGB20P-0008-WO

<150>     KR 10-2019-0154945
<151>     2019-11-27

<160>     29

<170>     KoPatentIn 3.0

<210>     1
<211>     76
<212>     PRT
<213>     Artificial Sequence

<220>
<223>     Ubiquitin C-terminal Tag


<400>     1
Met Gln Ile Phe Val Lys Thr Leu Thr Gly Lys Thr Ile Thr Leu Glu
  1               5                  10                  15

Val Glu Pro Ser Asp Thr Ile Glu Asn Val Lys Ala Lys Ile Gln Asp
              20                  25                  30

Lys Glu Gly Ile Pro Pro Asp Gln Gln Arg Leu Ile Phe Ala Gly Lys
              35                  40                  45

Gln Leu Glu Asp Gly Arg Thr Leu Ser Asp Tyr Asn Ile Gln Lys Glu
          50                  55                  60

Ser Thr Leu His Leu Val Leu Arg Leu Arg Gly Gly
 65                  70                  75


<210>     2
<211>     506
<212>     PRT
<213>     Artificial Sequence

<220>
<223>     Xylose Reductase


<400>     2
Met Gly His His His His His His Ser Asp Gln Glu Ala Lys Pro Ser
  1               5                  10                  15

Thr Glu Asp Leu Gly Asp Lys Lys Glu Gly Glu Tyr Ile Lys Leu Lys
              20                  25                  30

Val Ile Gly Gln Asp Ser Ser Glu Ile His Phe Lys Val Lys Met Thr
              35                  40                  45

Thr His Leu Lys Lys Leu Lys Glu Ser Tyr Cys Gln Arg Gln Gly Val
          50                  55                  60

Pro Met Asn Ser Leu Arg Phe Leu Phe Glu Gly Gln Arg Ile Ala Asp

21

|     | 65  |     |     |     | 70  |     |     |     | 75  |     |     |     | 80  |
|-----|-----|-----|-----|-----|-----|-----|-----|-----|-----|-----|-----|-----|-----|

Asn His Thr Pro Lys Glu Leu Gly Met Glu Glu Glu Asp Val Ile Glu
                85                    90                    95

Val Tyr Gln Glu Gln Thr Gly Gly Met Pro Ser Ile Lys Leu Asn Ser
                100                   105                   110

Gly Tyr Asp Met Pro Ala Val Gly Phe Gly Cys Trp Lys Val Asp Val
                115                   120                   125

Asp Thr Cys Ser Glu Gln Ile Tyr Arg Ala Ile Lys Thr Gly Tyr Arg
        130                   135                   140

Leu Phe Asp Gly Ala Glu Asp Tyr Ala Asn Glu Lys Leu Val Gly Ala
145                   150                   155                   160

Gly Val Lys Lys Ala Ile Asp Glu Gly Ile Val Lys Arg Glu Asp Leu
                165                   170                   175

Phe Leu Thr Ser Lys Leu Trp Asn Asn Tyr His His Pro Asp Asn Val
                180                   185                   190

Glu Lys Ala Leu Asn Arg Thr Leu Ser Asp Leu Gln Val Asp Tyr Val
                195                   200                   205

Asp Leu Phe Leu Ile His Phe Pro Val Thr Phe Lys Phe Val Pro Leu
        210                   215                   220

Glu Glu Lys Tyr Pro Pro Gly Phe Tyr Cys Gly Lys Gly Asp Asn Phe
225                   230                   235                   240

Asp Tyr Glu Asp Val Pro Ile Leu Glu Thr Trp Lys Ala Leu Glu Lys
                245                   250                   255

Leu Val Lys Ala Gly Lys Ile Arg Ser Ile Gly Val Ser Asn Phe Pro
                260                   265                   270

Gly Ala Leu Leu Leu Asp Leu Leu Arg Gly Ala Thr Ile Lys Pro Ser
        275                   280                   285

Val Leu Gln Val Glu His His Pro Tyr Leu Gln Gln Pro Arg Leu Ile
        290                   295                   300

Glu Phe Ala Gln Ser Arg Gly Ile Ala Val Thr Ala Tyr Ser Ser Phe
305                   310                   315                   320

Gly Pro Gln Ser Phe Val Glu Leu Asn Gln Gly Arg Ala Leu Asn Thr
                325                   330                   335

Ser Pro Leu Phe Glu Asn Glu Thr Ile Lys Ala Ile Ala Ala Lys His
        340                   345                   350

Gly Lys Ser Pro Ala Gln Val Leu Leu Arg Trp Ser Ser Gln Arg Gly
        355                   360                   365

Ile Ala Ile Ile Pro Lys Ser Asn Thr Val Pro His Leu Leu Glu Asn
        370                   375                   380

Lys Asp Val Asn Ser Phe Asp Leu Asp Glu Gln Asp Phe Ala Asp Ile
385                   390                   395                   400

Ala Lys Leu Asp Ile Asn Leu Arg Phe Asn Asp Pro Trp Asp Trp Asp

```
                    405                      410                      415

Lys Ile Pro Ile Phe Val Gly Gly His His His His His Met Gln
            420                  425                  430

Ile Phe Val Lys Thr Leu Thr Gly Lys Thr Ile Thr Leu Glu Val Glu
            435                  440                  445

Pro Ser Asp Thr Ile Glu Asn Val Lys Ala Lys Ile Gln Asp Lys Glu
            450                  455                  460

Gly Ile Pro Pro Asp Gln Gln Arg Leu Ile Phe Ala Gly Lys Gln Leu
465                  470                  475                  480

Glu Asp Gly Arg Thr Leu Ser Asp Tyr Asn Ile Gln Lys Glu Ser Thr
                    485                  490                  495

Leu His Leu Val Leu Arg Leu Arg Gly Gly
            500                  505


<210>    3
<211>    542
<212>    PRT
<213>    Artificial Sequence

<220>
<223>    Xylitol dehydrogenase


<400>    3
Met Gly His His His His His His Ser Asp Gln Glu Ala Lys Pro Ser
    1                   5                   10                  15

Thr Glu Asp Leu Gly Asp Lys Lys Glu Gly Glu Tyr Ile Lys Leu Lys
            20                  25                  30

Val Ile Gly Gln Asp Ser Ser Glu Ile His Phe Lys Val Lys Met Thr
            35                  40                  45

Thr His Leu Lys Lys Leu Lys Glu Ser Tyr Cys Gln Arg Gln Gly Val
        50                  55                  60

Pro Met Asn Ser Leu Arg Phe Leu Phe Glu Gly Gln Arg Ile Ala Asp
65                  70                  75                  80

Asn His Thr Pro Lys Glu Leu Gly Met Glu Glu Glu Asp Val Ile Glu
                85                  90                  95

Val Tyr Gln Glu Gln Thr Gly Gly Met Thr Ala Asn Pro Ser Leu Val
                100                 105                 110

Leu Asn Lys Ile Asp Asp Ile Ser Phe Glu Thr Tyr Asp Ala Pro Glu
            115                 120                 125

Ile Ser Glu Pro Thr Asp Val Leu Val Gln Val Lys Lys Thr Gly Ile
        130                 135                 140

Cys Gly Ser Asp Ile His Phe Tyr Ala His Gly Arg Ile Gly Asn Phe
145                 150                 155                 160

Val Leu Thr Lys Pro Met Val Leu Gly His Glu Ser Ala Gly Thr Val
                165                 170                 175
```

```
Val Gln Val Gly Lys Gly Val Thr Ser Leu Lys Val Gly Asp Asn Val
            180                 185                 190

Ala Ile Glu Pro Gly Ile Pro Ser Arg Phe Ser Asp Glu Tyr Lys Ser
            195                 200                 205

Gly His Tyr Asn Leu Cys Pro His Met Ala Phe Ala Ala Thr Pro Asn
210                 215                 220

Ser Lys Glu Gly Glu Pro Asn Pro Pro Gly Thr Leu Cys Lys Tyr Phe
225                 230                 235                 240

Lys Ser Pro Glu Asp Phe Leu Val Lys Leu Pro Asp His Val Ser Leu
            245                 250                 255

Glu Leu Gly Ala Leu Val Glu Pro Leu Ser Val Gly Val His Ala Ser
            260                 265                 270

Lys Leu Gly Ser Val Ala Phe Gly Asp Tyr Val Ala Val Phe Gly Ala
            275                 280                 285

Gly Pro Val Gly Leu Leu Ala Ala Ala Val Ala Lys Thr Phe Gly Ala
290                 295                 300

Lys Gly Val Ile Val Val Asp Ile Phe Asp Asn Lys Leu Lys Met Ala
305                 310                 315                 320

Lys Asp Ile Gly Ala Ala Thr His Thr Phe Asn Ser Lys Thr Gly Gly
            325                 330                 335

Ser Glu Glu Leu Ile Lys Ala Phe Gly Gly Asn Val Pro Asn Val Val
            340                 345                 350

Leu Glu Cys Thr Gly Ala Glu Pro Cys Ile Lys Leu Gly Val Asp Ala
            355                 360                 365

Ile Ala Pro Gly Gly Arg Phe Val Gln Val Gly Asn Ala Ala Gly Pro
            370                 375                 380

Val Ser Phe Pro Ile Thr Val Phe Ala Met Lys Glu Leu Thr Leu Phe
385                 390                 395                 400

Gly Ser Phe Arg Tyr Gly Phe Asn Asp Tyr Lys Thr Ala Val Gly Ile
            405                 410                 415

Phe Asp Thr Asn Tyr Gln Asn Gly Arg Glu Asn Ala Pro Ile Asp Phe
            420                 425                 430

Glu Gln Leu Ile Thr His Arg Tyr Lys Phe Lys Asp Ala Ile Glu Ala
            435                 440                 445

Tyr Asp Leu Val Arg Ala Gly Lys Gly Ala Val Lys Cys Leu Ile Asp
            450                 455                 460

Gly Pro Glu Met Gln Ile Phe Val Lys Thr Leu Thr Gly Lys Thr Ile
465                 470                 475                 480

Thr Leu Glu Val Glu Pro Ser Asp Thr Ile Glu Asn Val Lys Ala Lys
            485                 490                 495

Ile Gln Asp Lys Glu Gly Ile Pro Pro Asp Gln Gln Arg Leu Ile Phe
            500                 505                 510
```

24

```
            Ala Gly Lys Gln Leu Glu Asp Gly Arg Thr Leu Ser Asp Tyr Asn Ile
                    515                 520                 525

            Gln Lys Glu Ser Thr Leu His Leu Val Leu Arg Leu Arg Gly
                    530                 535                 540


<210>    4
<211>    407
<212>    PRT
<213>    Artificial Sequence

<220>
<223>    Oxaloacetate decarboxylase


<400>    4
Met Gly His His His His His His Ser Asp Gln Glu Ala Lys Pro Ser
 1               5                  10                  15

Thr Glu Asp Leu Gly Asp Lys Lys Glu Gly Glu Tyr Ile Lys Leu Lys
                20                  25                  30

Val Ile Gly Gln Asp Ser Ser Glu Ile His Phe Lys Val Lys Met Thr
                35                  40                  45

Thr His Leu Lys Lys Leu Lys Glu Ser Tyr Cys Gln Arg Gln Gly Val
        50                  55                  60

Pro Met Asn Ser Leu Arg Phe Leu Phe Glu Gly Gln Arg Ile Ala Asp
 65                  70                  75                  80

Asn His Thr Pro Lys Glu Leu Gly Met Glu Glu Glu Asp Val Ile Glu
                85                  90                  95

Val Tyr Gln Glu Gln Thr Gly Gly Met Tyr Glu Leu Gly Val Val Tyr
                100                 105                 110

Arg Asn Ile Gln Arg Ala Asp Arg Ala Ala Ala Asp Gly Leu Ala Ala
                115                 120                 125

Leu Gly Ser Ala Thr Val His Glu Ala Met Gly Arg Val Gly Leu Leu
        130                 135                 140

Lys Pro Tyr Met Arg Pro Ile Tyr Ala Gly Lys Gln Val Ser Gly Thr
145                 150                 155                 160

Ala Val Thr Val Leu Leu Gln Pro Gly Asp Asn Trp Met Met His Val
                165                 170                 175

Ala Ala Glu Gln Ile Gln Pro Gly Asp Ile Val Val Ala Ala Val Thr
                180                 185                 190

Ala Glu Cys Thr Asp Gly Tyr Phe Gly Asp Leu Leu Ala Thr Ser Phe
        195                 200                 205

Gln Ala Arg Gly Ala Arg Ala Leu Ile Ile Asp Ala Gly Val Arg Asp
        210                 215                 220

Val Lys Thr Leu Gln Glu Met Asp Phe Pro Val Trp Ser Lys Ala Ile
```

225 230 235 240

Ser Ser Lys Gly Thr Ile Lys Ala Thr Leu Gly Ser Val Asn Ile Pro
245 250 255

Ile Val Cys Ala Gly Met Leu Val Thr Pro Gly Asp Val Ile Val Ala
260 265 270

Asp Asp Asp Gly Val Val Cys Val Pro Ala Ala Arg Ala Val Glu Val
275 280 285

Leu Ala Ala Ala Gln Lys Arg Glu Ser Phe Glu Gly Glu Lys Arg Ala
290 295 300

Lys Leu Ala Ser Gly Val Leu Gly Leu Asp Met Tyr Lys Met Arg Glu
305 310 315 320

Pro Leu Glu Lys Ala Gly Leu Lys Tyr Ile Asp Met Gln Ile Phe Val
325 330 335

Lys Thr Leu Thr Gly Lys Thr Ile Thr Leu Glu Val Glu Pro Ser Asp
340 345 350

Thr Ile Glu Asn Val Lys Ala Lys Ile Gln Asp Lys Glu Gly Ile Pro
355 360 365

Pro Asp Gln Gln Arg Leu Ile Phe Ala Gly Lys Gln Leu Glu Asp Gly
370 375 380

Arg Thr Leu Ser Asp Tyr Asn Ile Gln Lys Glu Ser Thr Leu His Leu
385 390 395 400

Val Leu Arg Leu Arg Gly Gly
405


<210> 5
<211> 430
<212> PRT
<213> Artificial Sequence

<220>
<223> Triose-phosphate isomerase


<400> 5
Met Gly His His His His His His Ser Asp Gln Glu Ala Lys Pro Ser
1 5 10 15

Thr Glu Asp Leu Gly Asp Lys Lys Glu Gly Glu Tyr Ile Lys Leu Lys
20 25 30

Val Ile Gly Gln Asp Ser Ser Glu Ile His Phe Lys Val Lys Met Thr
35 40 45

Thr His Leu Lys Lys Leu Lys Glu Ser Tyr Cys Gln Arg Gln Gly Val
50 55 60

Pro Met Asn Ser Leu Arg Phe Leu Phe Glu Gly Gln Arg Ile Ala Asp
65 70 75 80

Asn His Thr Pro Lys Glu Leu Gly Met Glu Glu Glu Asp Val Ile Glu
85 90 95

Val Tyr Gln Glu Gln Thr Gly Gly Met Arg Arg Val Leu Val Ala Gly
        100             105             110

Asn Trp Lys Met His Lys Thr Pro Ser Glu Ala Arg Val Trp Phe Ala
        115             120             125

Glu Leu Lys Arg Leu Leu Pro Pro Leu Gln Ser Glu Ala Ala Val Leu
        130             135             140

Pro Ala Phe Pro Ile Leu Pro Val Ala Lys Glu Val Leu Ala Glu Thr
145             150             155             160

Gln Val Gly Tyr Gly Ala Gln Asp Val Ser Ala His Lys Glu Gly Ala
            165             170             175

Tyr Thr Gly Glu Val Ser Ala Arg Met Leu Ser Asp Leu Gly Cys Arg
        180             185             190

Tyr Ala Ile Val Gly His Ser Glu Arg Arg Arg Tyr His Gly Glu Thr
        195             200             205

Asp Ala Leu Val Ala Glu Lys Ala Lys Arg Leu Leu Glu Glu Gly Ile
    210             215             220

Thr Pro Ile Leu Cys Val Gly Glu Pro Leu Glu Val Arg Glu Lys Gly
225             230             235             240

Glu Ala Val Pro Tyr Thr Leu Arg Gln Leu Arg Gly Ser Leu Glu Gly
            245             250             255

Val Glu Pro Pro Gly Pro Glu Ala Leu Val Ile Ala Tyr Glu Pro Val
            260             265             270

Trp Ala Ile Gly Thr Gly Lys Asn Ala Thr Pro Glu Asp Ala Glu Ala
        275             280             285

Met His Gln Glu Ile Arg Lys Ala Leu Ser Glu Arg Tyr Gly Glu Ala
        290             295             300

Phe Ala Ser Arg Val Arg Ile Leu Tyr Gly Gly Ser Val Asn Pro Lys
305             310             315             320

Asn Phe Ala Asp Leu Leu Ser Met Pro Asn Val Asp Gly Gly Leu Val
            325             330             335

Gly Gly Ala Ser Leu Glu Leu Glu Ser Phe Leu Ala Leu Leu Arg Ile
            340             345             350

Ala Gly Met Gln Ile Phe Val Lys Thr Leu Thr Gly Lys Thr Ile Thr
            355             360             365

Leu Glu Val Glu Pro Ser Asp Thr Ile Glu Asn Val Lys Ala Lys Ile
        370             375             380

Gln Asp Lys Glu Gly Ile Pro Pro Asp Gln Gln Arg Leu Ile Phe Ala
385             390             395             400

Gly Lys Gln Leu Glu Asp Gly Arg Thr Leu Ser Asp Tyr Asn Ile Gln
            405             410             415

Lys Glu Ser Thr Leu His Leu Val Leu Arg Leu Arg Gly Gly
            420             425             430

```
<210>    6
<211>    496
<212>    PRT
<213>    Artificial Sequence

<220>
<223>    Aldolase


<400>    6
Met Gly His His His His His His Ser Asp Gln Glu Ala Lys Pro Ser
 1               5                   10                  15

Thr Glu Asp Leu Gly Asp Lys Lys Glu Gly Glu Tyr Ile Lys Leu Lys
            20                  25                  30

Val Ile Gly Gln Asp Ser Ser Glu Ile His Phe Lys Val Lys Met Thr
            35                  40                  45

Thr His Leu Lys Lys Leu Lys Glu Ser Tyr Cys Gln Arg Gln Gly Val
        50                  55                  60

Pro Met Asn Ser Leu Arg Phe Leu Phe Glu Gly Gln Arg Ile Ala Asp
65                  70                  75                  80

Asn His Thr Pro Lys Glu Leu Gly Met Glu Glu Glu Asp Val Ile Glu
                85                  90                  95

Val Tyr Gln Glu Gln Thr Gly Gly Met Val Thr Met Pro Tyr Val Lys
            100                 105                 110

Asn Thr Lys Glu Ile Leu Glu Lys Ala Ser Lys Glu Arg Tyr Ala Ile
        115                 120                 125

Gly Ala Phe Asn Phe Asn Asn Met Glu Phe Leu Gln Ala Ile Leu Glu
    130                 135                 140

Ala Ala Glu Glu Glu Lys Ala Pro Val Ile Val Ala Thr Ser Glu Gly
145                 150                 155                 160

Ala Ile Lys Tyr Ile Gly Lys Gly Asp Ile Glu Thr Gly Ala Lys Leu
                165                 170                 175

Ala Val Glu Met Val Arg Thr Tyr Ala Glu Lys Leu Ser Val Pro Val
            180                 185                 190

Ala Leu His Leu Asp His Gly Arg Asp Phe Lys Val Ile Met Ala Ala
        195                 200                 205

Ile Lys Ala Gly Tyr Ser Ser Val Met Ile Asp Ala Ser His Leu Pro
    210                 215                 220

Phe Glu Glu Asn Leu Arg Glu Thr Lys Arg Ile Val Glu Ile Ala His
225                 230                 235                 240

Ala Val Gly Ile Ser Val Glu Ala Glu Leu Gly Lys Leu Lys Gly Ile
            245                 250                 255

Glu Asp Asn Val Val Glu Lys Glu Ser Val Leu Val Asp Pro Glu Glu
            260                 265                 270
```

28

```
Ala Lys Val Phe Val Lys Glu Thr Glu Val Asp Phe Leu Ala Pro Ala
        275                 280             285

Ile Gly Thr Ser His Gly Ala Phe Lys Phe Lys Gly Glu Ala Gln Leu
        290                 295             300

Asp Phe Glu Arg Leu Lys Lys Val Lys Glu Tyr Thr Gln Ile Pro Leu
305                 310                 315                 320

Val Leu His Gly Ala Ser Met Val Pro Gln Asp Ile Val Lys Leu Ala
        325                 330                 335

Asn Glu Tyr Gly Ala Glu Leu Ser Gly Ala Lys Gly Val Pro Glu Asp
        340                 345                 350

Met Leu Lys Lys Ala Ile Glu Leu Gly Ile Asn Lys Ile Asn Thr Asp
        355                 360                 365

Thr Asp Leu Arg Ile Thr Phe Val Ala Tyr Leu Arg Lys Val Leu Ser
        370                 375                 380

Glu Asp Lys Ser Gln Ile Asp Pro Arg Lys Ile Phe Lys Pro Val Phe
385                 390                 395                 400

Glu Gln Val Lys Glu Ile Val Lys Glu Arg Ile Arg Ile Phe Gly Ser
                405                 410                 415

Ser Gly Lys Ala Met Gln Ile Phe Val Lys Thr Leu Thr Gly Lys Thr
            420                 425                 430

Ile Thr Leu Glu Val Glu Pro Ser Asp Thr Ile Glu Asn Val Lys Ala
        435                 440                 445

Lys Ile Gln Asp Lys Glu Gly Ile Pro Pro Asp Gln Gln Arg Leu Ile
    450                 455                 460

Phe Ala Gly Lys Gln Leu Glu Asp Gly Arg Thr Leu Ser Asp Tyr Asn
465                 470                 475                 480

Ile Gln Lys Glu Ser Thr Leu His Leu Val Leu Arg Leu Arg Gly Gly
            485                 490                 495
```

<210>    7
<211>    437
<212>    PRT
<213>    Artificial Sequence

<220>
<223>    Fructose 1,6-bisphosphatase

<400>    7

```
Met Gly His His His His His Ser Asp Gln Glu Ala Lys Pro Ser
  1                 5                 10                15

Thr Glu Asp Leu Gly Asp Lys Lys Glu Gly Glu Tyr Ile Lys Leu Lys
            20                  25                  30

Val Ile Gly Gln Asp Ser Ser Glu Ile His Phe Lys Val Lys Met Thr
        35                  40                  45
```

```
Thr His Leu Lys Lys Leu Lys Glu Ser Tyr Cys Gln Arg Gln Gly Val
    50                      55              60

Pro Met Asn Ser Leu Arg Phe Leu Phe Glu Gly Gln Arg Ile Ala Asp
65              70              75                      80

Asn His Thr Pro Lys Glu Leu Gly Met Glu Glu Glu Asp Val Ile Glu
            85              90                      95

Val Tyr Gln Glu Gln Thr Gly Gly Met Leu Asp Arg Leu Asp Phe Ser
            100             105             110

Ile Lys Leu Leu Arg Lys Val Gly His Leu Leu Met Ile His Trp Gly
        115             120             125

Arg Val Asp Asn Val Glu Lys Lys Thr Gly Phe Lys Asp Ile Val Thr
    130             135             140

Glu Ile Asp Arg Glu Ala Gln Arg Met Ile Val Asp Glu Ile Arg Lys
145             150             155             160

Phe Phe Pro Asp Glu Asn Ile Met Ala Glu Glu Gly Ile Phe Glu Lys
            165             170             175

Gly Asp Arg Leu Trp Ile Ile Asp Pro Ile Asp Gly Thr Ile Asn Phe
        180             185             190

Val His Gly Leu Pro Asn Phe Ser Ile Ser Leu Ala Tyr Val Glu Asn
    195             200             205

Gly Glu Val Lys Leu Gly Val Val His Ala Pro Ala Leu Asn Glu Thr
210             215             220

Leu Tyr Ala Glu Glu Gly Ser Gly Ala Phe Phe Asn Gly Glu Arg Ile
225             230             235             240

Arg Val Ser Glu Asn Ala Ser Leu Glu Glu Cys Val Gly Ser Thr Gly
            245             250             255

Ser Tyr Val Asp Phe Thr Gly Lys Phe Ile Glu Arg Met Glu Lys Arg
            260             265             270

Thr Arg Arg Ile Arg Ile Leu Gly Ser Ala Ala Leu Asn Ala Ala Tyr
        275             280             285

Val Gly Ala Gly Arg Val Asp Phe Phe Val Thr Trp Arg Ile Asn Pro
    290             295             300

Trp Asp Ile Ala Ala Gly Leu Ile Ile Val Lys Glu Ala Gly Gly Met
305             310             315             320

Val Thr Asp Phe Ser Gly Lys Glu Ala Asn Ala Phe Ser Lys Asn Phe
            325             330             335

Ile Phe Ser Asn Gly Leu Ile His Asp Glu Val Val Lys Val Val Asn
        340             345             350

Glu Val Val Glu Glu Ile Gly Gly Lys Met Gln Ile Phe Val Lys Thr
        355             360             365

Leu Thr Gly Lys Thr Ile Thr Leu Glu Val Glu Pro Ser Asp Thr Ile
    370             375             380
```

```
Glu Asn Val Lys Ala Lys Ile Gln Asp Lys Glu Gly Ile Pro Pro Asp
385             390             395             400

Gln Gln Arg Leu Ile Phe Ala Gly Lys Gln Leu Glu Asp Gly Arg Thr
            405             410             415

Leu Ser Asp Tyr Asn Ile Gln Lys Glu Ser Thr Leu His Leu Val Leu
            420             425             430

Arg Leu Arg Gly Gly
            435


<210>   8
<211>   772
<212>   PRT
<213>   Artificial Sequence

<220>
<223>   Pyruvate oxidase


<400>   8
Met Gly His His His His His His Ser Asp Gln Glu Ala Lys Pro Ser
  1               5               10              15

Thr Glu Asp Leu Gly Asp Lys Lys Glu Gly Glu Tyr Ile Lys Leu Lys
            20              25              30

Val Ile Gly Gln Asp Ser Ser Glu Ile His Phe Lys Val Lys Met Thr
            35              40              45

Thr His Leu Lys Lys Leu Lys Glu Ser Tyr Cys Gln Arg Gln Gly Val
        50              55              60

Pro Met Asn Ser Leu Arg Phe Leu Phe Glu Gly Gln Arg Ile Ala Asp
 65             70              75              80

Asn His Thr Pro Lys Glu Leu Gly Met Glu Glu Glu Asp Val Ile Glu
            85              90              95

Val Tyr Gln Glu Gln Thr Gly Gly Met Ser Asp Asn Lys Ile Asn Ile
            100             105             110

Gly Leu Ala Val Met Lys Ile Leu Glu Ser Trp Gly Ala Asp Thr Ile
            115             120             125

Tyr Gly Ile Pro Ser Gly Thr Leu Ser Ser Leu Met Asp Ala Met Gly
            130             135             140

Glu Glu Glu Asn Asn Val Lys Phe Leu Gln Val Lys His Glu Glu Val
145             150             155             160

Gly Ala Met Ala Ala Val Met Gln Ser Lys Phe Gly Gly Asn Leu Gly
            165             170             175

Val Thr Val Gly Ser Gly Gly Pro Gly Ala Ser His Leu Ile Asn Gly
            180             185             190

Leu Tyr Asp Ala Ala Met Asp Asn Ile Pro Val Val Ala Ile Leu Gly
            195             200             205

Ser Arg Pro Gln Arg Glu Leu Asn Met Asp Ala Phe Gln Glu Leu Asn
```

31

```
          210                      215                         220

Gln Asn Pro Met Tyr Asp His Ile Ala Val Tyr Asn Arg Arg Val Ala
225                 230                 235                 240

Tyr Ala Glu Gln Leu Pro Lys Leu Val Asp Glu Ala Ala Arg Met Ala
                245                 250                 255

Ile Ala Lys Arg Gly Val Ala Val Leu Glu Val Pro Gly Asp Phe Ala
                260                 265                 270

Lys Val Glu Ile Asp Asn Asp Gln Trp Tyr Ser Ser Ala Asn Ser Leu
                275                 280                 285

Arg Lys Tyr Ala Pro Ile Ala Pro Ala Ala Gln Asp Ile Asp Ala Ala
                290                 295                 300

Val Glu Leu Leu Asn Asn Ser Lys Arg Pro Val Ile Tyr Ala Gly Ile
305                 310                 315                 320

Gly Thr Met Gly His Gly Pro Ala Val Gln Glu Leu Ala Arg Lys Ile
                325                 330                 335

Lys Ala Pro Val Ile Thr Thr Gly Lys Asn Phe Glu Thr Phe Glu Trp
                340                 345                 350

Asp Phe Glu Ala Leu Thr Gly Ser Thr Tyr Arg Val Gly Trp Lys Pro
                355                 360                 365

Ala Asn Glu Thr Ile Leu Glu Ala Asp Thr Val Leu Phe Ala Gly Ser
                370                 375                 380

Asn Phe Pro Phe Ser Glu Val Glu Gly Thr Phe Arg Asn Val Asp Asn
385                 390                 395                 400

Phe Ile Gln Ile Asp Ile Asp Pro Ala Met Leu Gly Lys Arg His His
                405                 410                 415

Ala Asp Val Ala Ile Leu Gly Asp Ala Gly Leu Ala Ile Asp Glu Ile
                420                 425                 430

Leu Asn Lys Val Asp Ala Val Glu Glu Ser Ala Trp Trp Thr Ala Asn
                435                 440                 445

Leu Lys Asn Ile Ala Asn Trp Arg Glu Tyr Ile Asn Met Leu Glu Thr
                450                 455                 460

Lys Glu Glu Gly Asp Leu Gln Phe Tyr Gln Val Tyr Asn Ala Ile Asn
465                 470                 475                 480

Asn His Ala Asp Glu Asp Ala Ile Tyr Ser Ile Asp Val Gly Asn Ser
                485                 490                 495

Thr Gln Thr Ser Ile Arg His Leu His Met Thr Pro Lys Asn Met Trp
                500                 505                 510

Arg Thr Ser Pro Leu Phe Ala Thr Met Gly Ile Ala Ile Pro Gly Gly
                515                 520                 525

Leu Gly Ala Lys Asn Thr Tyr Pro Asp Arg Gln Val Trp Asn Ile Ile
                530                 535                 540

Gly Asp Gly Ala Phe Ser Met Thr Tyr Pro Asp Val Val Thr Asn Val
```

32

545 550 555 560

Arg Tyr Asn Met Pro Val Ile Asn Val Val Phe Ser Asn Thr Glu Tyr
565 570 575

Ala Phe Ile Lys Asn Lys Tyr Glu Asp Thr Asn Lys Asn Leu Phe Gly
580 585 590

Val Asp Phe Thr Asp Val Asp Tyr Ala Lys Ile Ala Glu Ala Gln Gly
595 600 605

Ala Lys Gly Phe Thr Val Ser Arg Ile Glu Asp Met Asp Arg Val Met
610 615 620

Ala Glu Ala Val Ala Ala Asn Lys Ala Gly His Thr Val Val Ile Asp
625 630 635 640

Cys Lys Ile Thr Gln Asp Arg Pro Ile Pro Val Glu Thr Leu Lys Leu
645 650 655

Asp Ser Lys Leu Tyr Ser Glu Asp Glu Ile Lys Ala Tyr Lys Glu Arg
660 665 670

Tyr Glu Ala Ala Asn Leu Val Pro Phe Arg Glu Tyr Leu Glu Ala Glu
675 680 685

Gly Leu Glu Ser Lys Tyr Ile Lys Met Gln Ile Phe Val Lys Thr Leu
690 695 700

Thr Gly Lys Thr Ile Thr Leu Glu Val Glu Pro Ser Asp Thr Ile Glu
705 710 715 720

Asn Val Lys Ala Lys Ile Gln Asp Lys Glu Gly Ile Pro Pro Asp Gln
725 730 735

Gln Arg Leu Ile Phe Ala Gly Lys Gln Leu Glu Asp Gly Arg Thr Leu
740 745 750

Ser Asp Tyr Asn Ile Gln Lys Glu Ser Thr Leu His Leu Val Leu Arg
755 760 765

Leu Arg Gly Gly
770

<210> 9
<211> 1024
<212> PRT
<213> Artificial Sequence

<220>
<223> Yeast UBE1

<400> 9
Met Ser Ser Asn Asn Ser Gly Leu Ser Ala Ala Gly Glu Ile Asp Glu
1 5 10 15

Ser Leu Tyr Ser Arg Gln Leu Tyr Val Leu Gly Lys Glu Ala Met Leu
20 25 30

Lys Met Gln Thr Ser Asn Val Leu Ile Leu Gly Leu Lys Gly Leu Gly
35 40 45

33

```
Val Glu Ile Ala Lys Asn Val Val Leu Ala Gly Val Lys Ser Met Thr
    50                  55                  60

Val Phe Asp Pro Glu Pro Val Gln Leu Ala Asp Leu Ser Thr Gln Phe
    65                  70                  75                  80

Phe Leu Thr Glu Lys Asp Ile Gly Gln Lys Arg Gly Asp Val Thr Arg
                85                  90                  95

Ala Lys Leu Ala Glu Leu Asn Ala Tyr Val Pro Val Asn Val Leu Asp
            100                 105                 110

Ser Leu Asp Asp Val Thr Gln Leu Ser Gln Phe Gln Val Val Val Ala
            115                 120                 125

Thr Asp Thr Val Ser Leu Glu Asp Lys Val Lys Ile Asn Glu Phe Cys
    130                 135                 140

His Ser Ser Gly Ile Arg Phe Ile Ser Ser Glu Thr Arg Gly Leu Phe
145                 150                 155                 160

Gly Asn Thr Phe Val Asp Leu Gly Asp Glu Phe Thr Val Leu Asp Pro
                165                 170                 175

Thr Gly Glu Glu Pro Arg Thr Gly Met Val Ser Asp Ile Glu Pro Asp
            180                 185                 190

Gly Thr Val Thr Met Leu Asp Asp Asn Arg His Gly Leu Glu Asp Gly
    195                 200                 205

Asn Phe Val Arg Phe Ser Glu Val Glu Gly Leu Asp Lys Leu Asn Asp
    210                 215                 220

Gly Thr Leu Phe Lys Val Glu Val Leu Gly Pro Phe Ala Phe Arg Ile
225                 230                 235                 240

Gly Ser Val Lys Glu Tyr Gly Glu Tyr Lys Lys Gly Gly Ile Phe Thr
                245                 250                 255

Glu Val Lys Val Pro Arg Lys Ile Ser Phe Lys Ser Leu Lys Gln Gln
            260                 265                 270

Leu Ser Asn Pro Glu Phe Val Phe Ser Asp Phe Ala Lys Phe Asp Arg
    275                 280                 285

Ala Ala Gln Leu His Leu Gly Phe Gln Ala Leu His Gln Phe Ala Val
    290                 295                 300

Arg His Asn Gly Glu Leu Pro Arg Thr Met Asn Asp Glu Asp Ala Asn
305                 310                 315                 320

Glu Leu Ile Lys Leu Val Thr Asp Leu Ser Val Gln Gln Pro Glu Val
            325                 330                 335

Leu Gly Glu Gly Val Asp Val Asn Glu Asp Leu Ile Lys Glu Leu Ser
            340                 345                 350

Tyr Gln Ala Arg Gly Asp Ile Pro Gly Val Val Ala Phe Phe Gly Gly
            355                 360                 365

Leu Val Ala Gln Glu Val Leu Lys Ala Cys Ser Gly Lys Phe Thr Pro
    370                 375                 380
```

34

```
Leu Lys Gln Phe Met Tyr Phe Asp Ser Leu Glu Ser Leu Pro Asp Pro
385             390             395             400

Lys Asn Phe Pro Arg Asn Glu Lys Thr Thr Gln Pro Val Asn Ser Arg
            405             410             415

Tyr Asp Asn Gln Ile Ala Val Phe Gly Leu Asp Phe Gln Lys Lys Ile
            420             425             430

Ala Asn Ser Lys Val Phe Leu Val Gly Ser Gly Ala Ile Gly Cys Glu
            435             440             445

Met Leu Lys Asn Trp Ala Leu Leu Gly Leu Gly Ser Gly Ser Asp Gly
    450             455             460

Tyr Ile Val Val Thr Asp Asn Asp Ser Ile Glu Lys Ser Asn Leu Asn
465             470             475             480

Arg Gln Phe Leu Phe Arg Pro Lys Asp Val Gly Lys Asn Lys Ser Glu
            485             490             495

Val Ala Ala Glu Ala Val Cys Ala Met Asn Pro Asp Leu Lys Gly Lys
            500             505             510

Ile Asn Ala Lys Ile Asp Lys Val Gly Pro Glu Thr Glu Glu Ile Phe
            515             520             525

Asn Asp Ser Phe Trp Glu Ser Leu Asp Phe Val Thr Asn Ala Leu Asp
    530             535             540

Asn Val Asp Ala Arg Thr Tyr Val Asp Arg Arg Cys Val Phe Tyr Arg
545             550             555             560

Lys Pro Leu Leu Glu Ser Gly Thr Leu Gly Thr Lys Gly Asn Thr Gln
            565             570             575

Val Ile Ile Pro Arg Leu Thr Glu Ser Tyr Ser Ser Ser Arg Asp Pro
            580             585             590

Pro Glu Lys Ser Ile Pro Leu Cys Thr Leu Arg Ser Phe Pro Asn Lys
            595             600             605

Ile Asp His Thr Ile Ala Trp Ala Lys Ser Leu Phe Gln Gly Tyr Phe
    610             615             620

Thr Asp Ser Ala Glu Asn Val Asn Met Tyr Leu Thr Gln Pro Asn Phe
625             630             635             640

Val Glu Gln Thr Leu Lys Gln Ser Gly Asp Val Lys Gly Val Leu Glu
            645             650             655

Ser Ile Ser Asp Ser Leu Ser Ser Lys Pro His Asn Phe Glu Asp Cys
            660             665             670

Ile Lys Trp Ala Arg Leu Glu Phe Glu Lys Lys Phe Asn His Asp Ile
    675             680             685

Lys Gln Leu Leu Phe Asn Phe Pro Lys Asp Ala Lys Thr Ser Asn Gly
    690             695             700

Glu Pro Phe Trp Ser Gly Ala Lys Arg Ala Pro Thr Pro Leu Glu Phe
705             710             715             720
```

```
Asp Ile Tyr Asn Asn Asp His Phe His Phe Val Val Ala Gly Ala Ser
            725             730                     735

Leu Arg Ala Tyr Asn Tyr Gly Ile Lys Ser Asp Asp Ser Asn Ser Lys
            740             745                 750

Pro Asn Val Asp Glu Tyr Lys Ser Val Ile Asp His Met Ile Ile Pro
            755             760             765

Glu Phe Thr Pro Asn Ala Asn Leu Lys Ile Gln Val Asn Asp Asp Asp
    770             775             780

Pro Asp Pro Asn Ala Asn Ala Ala Asn Gly Ser Asp Glu Ile Asp Gln
785             790             795                     800

Leu Val Ser Ser Leu Pro Asp Pro Ser Thr Leu Ala Gly Phe Lys Leu
            805             810                 815

Glu Pro Val Asp Phe Glu Lys Asp Asp Asp Thr Asn His His Ile Glu
            820             825             830

Phe Ile Thr Ala Cys Ser Asn Cys Arg Ala Gln Asn Tyr Phe Ile Glu
            835             840             845

Thr Ala Asp Arg Gln Lys Thr Lys Phe Ile Ala Gly Arg Ile Ile Pro
    850             855             860

Ala Ile Ala Thr Thr Thr Ser Leu Val Thr Gly Leu Val Asn Leu Glu
865             870             875                     880

Leu Tyr Lys Leu Ile Asp Asn Lys Thr Asp Ile Glu Gln Tyr Lys Asn
            885             890                 895

Gly Phe Val Asn Leu Ala Leu Pro Phe Phe Gly Phe Ser Glu Pro Ile
            900             905             910

Ala Ser Pro Lys Gly Glu Tyr Asn Asn Lys Lys Tyr Asp Lys Ile Trp
            915             920             925

Asp Arg Phe Asp Ile Lys Gly Asp Ile Lys Leu Ser Asp Leu Ile Glu
    930             935             940

His Phe Glu Lys Asp Glu Gly Leu Glu Ile Thr Met Leu Ser Tyr Gly
945             950             955                     960

Val Ser Leu Leu Tyr Ala Ser Phe Phe Pro Pro Lys Lys Leu Lys Glu
            965             970                 975

Arg Leu Asn Leu Pro Ile Thr Gln Leu Val Lys Leu Val Thr Lys Lys
            980             985                 990

Asp Ile Pro Ala His Val Ser Thr Met Ile Leu Glu Ile Cys Ala Asp
            995             1000            1005

Asp Lys Glu Gly Glu Asp Val Glu Val Pro Phe Ile Thr Ile His Leu
    1010            1015            1020
```

<210> 10
<211> 147

```
<212>    PRT
<213>    Artificial Sequence

<220>
<223>    Ubch5a (Homo sapiens)


<400>    10
Met Ala Leu Lys Arg Ile Gln Lys Glu Leu Ser Asp Leu Gln Arg Asp
 1               5                  10                  15

Pro Pro Ala His Cys Ser Ala Gly Pro Val Gly Asp Asp Leu Phe His
             20                  25                  30

Trp Gln Ala Thr Ile Met Gly Pro Pro Asp Ser Ala Tyr Gln Gly Gly
             35                  40                  45

Val Phe Phe Leu Thr Val His Phe Pro Thr Asp Tyr Pro Phe Lys Pro
         50                  55                  60

Pro Lys Ile Ala Phe Thr Thr Lys Ile Tyr His Pro Asn Ile Asn Ser
 65                  70                  75                  80

Asn Gly Ser Ile Cys Leu Asp Ile Leu Arg Ser Gln Trp Ser Pro Ala
                 85                  90                  95

Leu Thr Val Ser Lys Val Leu Leu Ser Ile Cys Ser Leu Leu Cys Asp
                100                 105                 110

Pro Asn Pro Asp Asp Pro Leu Val Pro Asp Ile Ala Gln Ile Tyr Lys
             115                 120                 125

Ser Asp Lys Glu Lys Tyr Asn Arg His Ala Arg Glu Trp Thr Gln Lys
             130                 135                 140

Tyr Ala Met
145


<210>    11
<211>    154
<212>    PRT
<213>    Artificial Sequence

<220>
<223>    Ubch7 (Homo sapiens)


<400>    11
Met Ala Ala Ser Arg Arg Leu Met Lys Glu Leu Glu Glu Ile Arg Lys
 1               5                  10                  15

Cys Gly Met Lys Asn Phe Arg Asn Ile Gln Val Asp Glu Ala Asn Leu
             20                  25                  30

Leu Thr Trp Gln Gly Leu Ile Val Pro Asp Asn Pro Pro Tyr Asp Lys
             35                  40                  45

Gly Ala Phe Arg Ile Glu Ile Asn Phe Pro Ala Glu Tyr Pro Phe Lys
         50                  55                  60

Pro Pro Lys Ile Thr Phe Lys Thr Lys Ile Tyr His Pro Asn Ile Asp
 65                  70                  75                  80
```

37

```
Glu Lys Gly Gln Val Cys Leu Pro Val Ile Ser Ala Glu Asn Trp Lys
                85                  90                  95


Pro Ala Thr Lys Thr Asp Gln Val Ile Gln Ser Leu Ile Ala Leu Val
            100                 105                 110


Asn Asp Pro Gln Pro Glu His Pro Leu Arg Ala Asp Leu Ala Glu Glu
        115                 120                 125


Tyr Ser Lys Asp Arg Lys Lys Phe Cys Lys Asn Ala Glu Glu Phe Thr
    130                 135                 140


Lys Lys Tyr Gly Glu Lys Arg Pro Val Asp
145                 150



<210>    12
<211>    200
<212>    PRT
<213>    Artificial Sequence


<220>
<223>    E2-25K (Homo sapiens)



<400>    12
Met Ala Asn Ile Ala Val Gln Arg Ile Lys Arg Glu Phe Lys Glu Val
    1               5                   10                  15


Leu Lys Ser Glu Glu Thr Ser Lys Asn Gln Ile Lys Val Asp Leu Val
            20                  25                  30


Asp Glu Asn Phe Thr Glu Leu Arg Gly Glu Ile Ala Gly Pro Pro Asp
        35                  40                  45


Thr Pro Tyr Glu Gly Gly Arg Tyr Gln Leu Glu Ile Lys Ile Pro Glu
    50                  55                  60


Thr Tyr Pro Phe Asn Pro Pro Lys Val Arg Phe Ile Thr Lys Ile Trp
65                  70                  75                  80


His Pro Asn Ile Ser Ser Val Thr Gly Ala Ile Cys Leu Asp Ile Leu
                85                  90                  95


Lys Asp Gln Trp Ala Ala Ala Met Thr Leu Arg Thr Val Leu Leu Ser
            100                 105                 110


Leu Gln Ala Leu Leu Ala Ala Ala Glu Pro Asp Asp Pro Gln Asp Ala
        115                 120                 125


Val Val Ala Asn Gln Tyr Lys Gln Asn Pro Glu Met Phe Lys Gln Thr
    130                 135                 140


Ala Arg Leu Trp Ala His Val Tyr Ala Gly Ala Pro Val Ser Ser Pro
145                 150                 155                 160


Glu Tyr Thr Lys Lys Ile Glu Asn Leu Cys Ala Met Gly Phe Asp Arg
                165                 170                 175


Asn Ala Val Ile Val Ala Leu Ser Ser Lys Ser Trp Asp Val Glu Thr
                180                 185                 190
```

38

```
Ala Thr Glu Leu Leu Leu Ser Asn
        195                 200
```

```
<210>   13
<211>   153
<212>   PRT
<213>   Artificial Sequence

<220>
<223>   Ubc13 (Saccharomyces cerevisiae)


<400>   13
Met Ala Ser Leu Pro Lys Arg Ile Ile Lys Glu Thr Glu Lys Leu Val
  1               5                  10                  15

Ser Asp Pro Val Pro Gly Ile Thr Ala Glu Pro His Asp Asp Asn Leu
            20                  25                  30

Arg Tyr Phe Gln Val Thr Ile Glu Gly Pro Glu Gln Ser Pro Tyr Glu
        35                  40                  45

Asp Gly Ile Phe Glu Leu Glu Leu Tyr Leu Pro Asp Asp Tyr Pro Met
        50                  55                  60

Glu Ala Pro Lys Val Arg Phe Leu Thr Lys Ile Tyr His Pro Asn Ile
 65                  70                  75                  80

Asp Arg Leu Gly Arg Ile Cys Leu Asp Val Leu Lys Thr Asn Trp Ser
                85                  90                  95

Pro Ala Leu Gln Ile Arg Thr Val Leu Leu Ser Ile Gln Ala Leu Leu
            100                 105                 110

Ala Ser Pro Asn Pro Asn Asp Pro Leu Ala Asn Asp Val Ala Glu Asp
            115                 120                 125

Trp Ile Lys Asn Glu Gln Gly Ala Lys Ala Lys Ala Arg Glu Trp Thr
        130                 135                 140

Lys Leu Tyr Ala Lys Lys Lys Pro Glu
145                 150
```

```
<210>   14
<211>   137
<212>   PRT
<213>   Artificial Sequence

<220>
<223>   MMS2 (UEV - Ubiquitin-conjugating enzyme variant) (Saccharomyces
        cerevisiae)


<400>   14
Met Ser Lys Val Pro Arg Asn Phe Arg Leu Leu Glu Glu Leu Glu Lys
  1               5                  10                  15

Gly Glu Lys Gly Phe Gly Pro Glu Ser Cys Ser Tyr Gly Leu Ala Asp
            20                  25                  30

Ser Asp Asp Ile Thr Met Thr Lys Trp Asn Gly Thr Ile Leu Gly Pro
```

```
                35                      40                        45

        Pro His Ser Asn His Glu Asn Arg Ile Tyr Ser Leu Ser Ile Asp Cys
            50                      55                      60

        Gly Pro Asn Tyr Pro Asp Ser Pro Pro Lys Val Thr Phe Ile Ser Lys
            65                      70                      75              80

        Ile Asn Leu Pro Cys Val Asn Pro Thr Thr Gly Glu Val Gln Thr Asp
                        85                      90                      95

        Phe His Thr Leu Arg Asp Trp Lys Arg Ala Tyr Thr Met Glu Thr Leu
                100                     105                     110

        Leu Leu Asp Leu Arg Lys Glu Met Ala Thr Pro Ala Asn Lys Lys Leu
                115                     120                     125

        Arg Gln Pro Lys Glu Gly Glu Thr Phe
            130                     135
```

<210> 15
<211> 807
<212> PRT
<213> Artificial Sequence

<220>
<223> E3 RSP5

<400> 15
```
        Met Pro Ser Ser Ile Ser Val Lys Leu Val Ala Ala Glu Ser Leu Tyr
            1               5                   10                      15

        Lys Arg Asp Val Phe Arg Ser Pro Asp Pro Phe Ala Val Leu Thr Ile
                        20                      25                      30

        Asp Gly Tyr Gln Thr Lys Ser Thr Ser Ala Ala Lys Lys Thr Leu Asn
                35                      40                      45

        Pro Tyr Trp Asn Glu Thr Phe Lys Phe Asp Asp Ile Asn Glu Asn Ser
            50                      55                      60

        Ile Leu Thr Ile Gln Val Phe Asp Gln Lys Lys Phe Lys Lys Lys Asp
            65                      70                      75              80

        Gln Gly Phe Leu Gly Val Val Asn Val Arg Val Gly Asp Val Leu Gly
                        85                      90                      95

        His Leu Asp Glu Asp Thr Ala Thr Ser Ser Gly Arg Pro Arg Glu Glu
                100                     105                     110

        Thr Ile Thr Arg Asp Leu Lys Lys Ser Asn Asp Gly Met Ala Val Ser
                115                     120                     125

        Gly Arg Leu Ile Val Val Leu Ser Lys Leu Pro Ser Ser Ser Pro His
            130                     135                     140

        Ser Gln Ala Pro Ser Gly His Thr Ala Ser Ser Ser Thr Asn Thr Ser
        145                     150                     155             160

        Ser Thr Thr Arg Thr Asn Gly His Ser Thr Ser Ser Thr Arg Asn His
                        165                     170                     175
```

40

```
Ser Thr Ser His Pro Ser Arg Gly Thr Ala Gln Ala Val Glu Ser Thr
            180                 185                 190

Leu Gln Ser Gly Thr Thr Ala Ala Thr Asn Thr Ala Thr Thr Ser His
            195                 200                 205

Arg Ser Thr Asn Ser Thr Ser Ser Ala Thr Arg Gln Tyr Ser Ser Phe
    210                 215                 220

Glu Asp Gln Tyr Gly Arg Leu Pro Pro Gly Trp Glu Arg Arg Thr Asp
225                 230                 235                 240

Asn Phe Gly Arg Thr Tyr Tyr Val Asp His Asn Thr Arg Thr Thr Thr
            245                 250                 255

Trp Lys Arg Pro Thr Leu Asp Gln Thr Glu Ala Glu Arg Gly Gln Leu
            260                 265                 270

Asn Ala Asn Thr Glu Leu Glu Arg Arg Gln His Arg Gly Arg Thr Leu
            275                 280                 285

Pro Gly Gly Ser Ser Asp Asn Ser Ser Val Thr Val Gln Val Gly Gly
    290                 295                 300

Gly Ser Asn Ile Pro Pro Val Asn Gly Ala Ala Ala Ala Ala Phe Ala
305                 310                 315                 320

Ala Thr Gly Gly Thr Thr Ser Gly Leu Gly Glu Leu Pro Ser Gly Trp
            325                 330                 335

Glu Gln Arg Phe Thr Pro Glu Gly Arg Ala Tyr Phe Val Asp His Asn
            340                 345                 350

Thr Arg Thr Thr Thr Trp Val Asp Pro Arg Arg Gln Gln Tyr Ile Arg
            355                 360                 365

Thr Tyr Gly Pro Thr Asn Thr Thr Ile Gln Gln Gln Pro Val Ser Gln
    370                 375                 380

Leu Gly Pro Leu Pro Ser Gly Trp Glu Met Arg Leu Thr Asn Thr Ala
385                 390                 395                 400

Arg Val Tyr Phe Val Asp His Asn Thr Lys Thr Thr Thr Trp Asp Asp
            405                 410                 415

Pro Arg Leu Pro Ser Ser Leu Asp Gln Asn Val Pro Gln Tyr Lys Arg
            420                 425                 430

Asp Phe Arg Arg Lys Val Ile Tyr Phe Arg Ser Gln Pro Ala Leu Arg
            435                 440                 445

Ile Leu Pro Gly Gln Cys His Ile Lys Val Arg Arg Lys Asn Ile Phe
    450                 455                 460

Glu Asp Ala Tyr Gln Glu Ile Met Arg Gln Thr Pro Glu Asp Leu Lys
465                 470                 475                 480

Lys Arg Leu Met Ile Lys Phe Asp Gly Glu Glu Gly Leu Asp Tyr Gly
            485                 490                 495

Gly Val Ser Arg Glu Phe Phe Phe Leu Leu Ser His Glu Met Phe Asn
            500                 505                 510
```

```
Pro Phe Tyr Cys Leu Phe Glu Tyr Ser Ala Tyr Asp Asn Tyr Thr Ile
        515                 520                 525

Gln Ile Asn Pro Asn Ser Gly Ile Asn Pro Glu His Leu Asn Tyr Phe
        530                 535                 540

Lys Phe Ile Gly Arg Val Val Gly Leu Gly Val Phe His Arg Arg Phe
545                 550                 555                 560

Leu Asp Ala Phe Phe Val Gly Ala Leu Tyr Lys Met Met Leu Arg Lys
                565                 570                 575

Lys Val Val Leu Gln Asp Met Glu Gly Val Asp Ala Glu Val Tyr Asn
        580                 585                 590

Ser Leu Asn Trp Met Leu Glu Asn Ser Ile Asp Gly Val Leu Asp Leu
        595                 600                 605

Thr Phe Ser Ala Asp Asp Glu Arg Phe Gly Glu Val Val Thr Val Asp
        610                 615                 620

Leu Lys Pro Asp Gly Arg Asn Ile Glu Val Thr Asp Gly Asn Lys Lys
625                 630                 635                 640

Glu Tyr Val Glu Leu Tyr Thr Gln Trp Arg Ile Val Asp Arg Val Gln
                645                 650                 655

Glu Gln Phe Lys Ala Phe Met Asp Gly Phe Asn Glu Ile Pro Glu Asp
                660                 665                 670

Leu Val Thr Val Phe Asp Glu Arg Glu Leu Glu Leu Leu Ile Gly Gly
                675                 680                 685

Ile Ala Glu Ile Asp Ile Glu Asp Trp Lys Lys His Thr Asp Tyr Arg
        690                 695                 700

Gly Tyr Gln Glu Ser Asp Glu Val Ile Gln Trp Phe Trp Lys Cys Val
705                 710                 715                 720

Ser Glu Trp Asp Asn Glu Gln Arg Ala Arg Leu Leu Gln Phe Thr Thr
                725                 730                 735

Gly Thr Ser Arg Ile Pro Val Asn Gly Phe Lys Asp Leu Gln Gly Ser
                740                 745                 750

Asp Gly Pro Arg Arg Phe Thr Ile Glu Lys Ala Gly Glu Val Gln Gln
                755                 760                 765

Leu Pro Lys Ser His Thr Cys Phe Asn Arg Val Asp Leu Pro Gln Tyr
        770                 775                 780

Val Asp Tyr Asp Ser Met Lys Gln Lys Leu Thr Leu Ala Val Glu Glu
785                 790                 795                 800

Thr Ile Gly Phe Gly Gln Glu
                805
```

```
<210>    16
<211>    1319
<212>    PRT
<213>    Artificial Sequence
```

```
<220>
<223>    E3 DOA10


<400>    16
Met Asp Val Asp Ser Asp Val Asn Val Ser Arg Leu Arg Asp Glu Leu
 1               5                  10                  15

His Lys Val Ala Asn Glu Glu Thr Asp Thr Ala Thr Phe Asn Asp Asp
            20                  25                  30

Ala Pro Ser Gly Ala Thr Cys Arg Ile Cys Arg Gly Glu Ala Thr Glu
        35                  40                  45

Asp Asn Pro Leu Phe His Pro Cys Lys Cys Arg Gly Ser Ile Lys Tyr
    50                  55                  60

Met His Glu Ser Cys Leu Leu Glu Trp Val Ala Ser Lys Asn Ile Asp
65                  70                  75                  80

Ile Ser Lys Pro Gly Ala Asp Val Lys Cys Asp Ile Cys His Tyr Pro
                85                  90                  95

Ile Gln Phe Lys Thr Ile Tyr Ala Glu Asn Met Pro Glu Lys Ile Pro
            100                 105                 110

Phe Ser Leu Leu Leu Ser Lys Ser Ile Leu Thr Phe Phe Glu Lys Ala
        115                 120                 125

Arg Leu Ala Leu Thr Ile Gly Leu Ala Ala Val Leu Tyr Ile Ile Gly
    130                 135                 140

Val Pro Leu Val Trp Asn Met Phe Gly Lys Leu Tyr Thr Met Met Leu
145                 150                 155                 160

Asp Gly Ser Ser Pro Tyr Pro Gly Asp Phe Leu Lys Ser Leu Ile Tyr
                165                 170                 175

Gly Tyr Asp Gln Ser Ala Thr Pro Glu Leu Thr Thr Arg Ala Ile Phe
            180                 185                 190

Tyr Gln Leu Leu Gln Asn His Ser Phe Thr Ser Leu Gln Phe Ile Met
    195                 200                 205

Ile Val Ile Leu His Ile Ala Leu Tyr Phe Gln Tyr Asp Met Ile Val
    210                 215                 220

Arg Glu Asp Val Phe Ser Lys Met Val Phe His Lys Ile Gly Pro Arg
225                 230                 235                 240

Leu Ser Pro Lys Asp Leu Lys Ser Arg Leu Lys Glu Arg Phe Pro Met
                245                 250                 255

Met Asp Asp Arg Met Val Glu Tyr Leu Ala Arg Glu Met Arg Ala His
                260                 265                 270

Asp Glu Asn Arg Gln Glu Gln Gly His Asp Arg Leu Asn Met Pro Ala
            275                 280                 285

Ala Ala Ala Asp Asn Asn Asn Asn Val Ile Asn Pro Arg Asn Asp Asn
        290                 295                 300
```

Val Pro Pro Gln Asp Pro Asn Asp His Arg Asn Phe Glu Asn Leu Arg
305                 310             315             320

His Val Asp Glu Leu Asp His Asp Glu Ala Thr Glu Glu His Glu Asn
                325             330             335

Asn Asp Ser Asp Asn Ser Leu Pro Ser Gly Asp Asp Ser Ser Arg Ile
            340             345             350

Leu Pro Gly Ser Ser Ser Asp Asn Glu Glu Asp Glu Glu Ala Glu Gly
        355             360             365

Gln Gln Gln Gln Gln Gln Pro Glu Glu Glu Ala Asp Tyr Arg Asp His
    370             375             380

Ile Glu Pro Asn Pro Ile Asp Met Trp Ala Asn Arg Arg Ala Gln Asn
385             390             395             400

Glu Phe Asp Asp Leu Ile Ala Ala Gln Gln Asn Ala Ile Asn Arg Pro
            405             410             415

Asn Ala Pro Val Phe Ile Pro Pro Ala Gln Asn Arg Ala Gly Asn
        420             425             430

Val Asp Gln Asp Glu Gln Asp Phe Gly Ala Ala Val Gly Val Pro Pro
    435             440             445

Ala Gln Ala Asn Pro Asp Asp Gln Gly Gln Gly Pro Leu Val Ile Asn
    450             455             460

Leu Lys Leu Lys Leu Leu Asn Val Ile Ala Tyr Phe Ile Ile Ala Val
465             470             475             480

Val Phe Thr Ala Ile Tyr Leu Ala Ile Ser Tyr Leu Phe Pro Thr Phe
            485             490             495

Ile Gly Phe Gly Leu Leu Lys Ile Tyr Phe Gly Ile Phe Lys Val Ile
        500             505             510

Leu Arg Gly Leu Cys His Leu Tyr Tyr Leu Ser Gly Ala His Ile Ala
        515             520             525

Tyr Asn Gly Leu Thr Lys Leu Val Pro Lys Val Asp Val Ala Met Ser
    530             535             540

Trp Ile Ser Asp His Leu Ile His Asp Ile Ile Tyr Leu Tyr Asn Gly
545             550             555             560

Tyr Thr Glu Asn Thr Met Lys His Ser Ile Phe Ile Arg Ala Leu Pro
            565             570             575

Ala Leu Thr Thr Tyr Leu Thr Ser Val Ser Ile Val Cys Ala Ser Ser
        580             585             590

Asn Leu Val Ser Arg Gly Tyr Gly Arg Glu Asn Gly Met Ser Asn Pro
    595             600             605

Thr Arg Arg Leu Ile Phe Gln Ile Leu Phe Ala Leu Lys Cys Thr Phe
    610             615             620

Lys Val Phe Thr Leu Phe Phe Ile Glu Leu Ala Gly Phe Pro Ile Leu
625             630             635             640

```
Ala Gly Val Met Leu Asp Phe Ser Leu Phe Cys Pro Ile Leu Ala Ser
            645         650             655

Asn Ser Arg Met Leu Trp Val Pro Ser Ile Cys Ala Ile Trp Pro Pro
            660         665             670

Phe Ser Leu Phe Val Tyr Trp Thr Ile Gly Thr Leu Tyr Met Tyr Trp
            675         680             685

Phe Ala Lys Tyr Ile Gly Met Ile Arg Lys Asn Ile Ile Arg Pro Gly
    690             695             700

Val Leu Phe Phe Ile Arg Ser Pro Glu Asp Pro Asn Ile Lys Ile Leu
705             710             715             720

His Asp Ser Leu Ile His Pro Met Ser Ile Gln Leu Ser Arg Leu Cys
            725         730             735

Leu Ser Met Phe Ile Tyr Ala Ile Phe Ile Val Leu Gly Phe Gly Phe
            740         745             750

His Thr Arg Ile Phe Phe Pro Phe Met Leu Lys Ser Asn Leu Leu Ser
            755         760             765

Val Pro Glu Ala Tyr Lys Pro Thr Ser Ile Ile Ser Trp Lys Phe Asn
    770             775             780

Thr Ile Leu Leu Thr Leu Tyr Phe Thr Lys Arg Ile Leu Glu Ser Ser
785             790             795             800

Ser Tyr Val Lys Pro Leu Leu Glu Arg Tyr Trp Lys Thr Ile Phe Lys
            805             810             815

Leu Cys Ser Arg Lys Leu Arg Leu Ser Ser Phe Ile Leu Gly Lys Asp
            820             825             830

Thr Pro Thr Glu Arg Gly His Ile Val Tyr Arg Asn Leu Phe Tyr Lys
            835             840             845

Tyr Ile Ala Ala Lys Asn Ala Glu Trp Ser Asn Gln Glu Leu Phe Thr
    850             855             860

Lys Pro Lys Thr Leu Glu Gln Ala Glu Glu Leu Phe Gly Gln Val Arg
865             870             875             880

Asp Val His Ala Tyr Phe Val Pro Asp Gly Val Leu Met Arg Val Pro
            885             890             895

Ser Ser Asp Ile Val Ser Arg Asn Tyr Val Gln Thr Met Phe Val Pro
            900             905             910

Val Thr Lys Asp Asp Lys Leu Leu Lys Pro Leu Asp Leu Glu Arg Ile
    915             920             925

Lys Glu Arg Asn Lys Arg Ala Ala Gly Glu Phe Gly Tyr Leu Asp Glu
    930             935             940

Gln Asn Thr Glu Tyr Asp Gln Tyr Tyr Ile Val Tyr Val Pro Pro Asp
945             950             955             960

Phe Arg Leu Arg Tyr Met Thr Leu Leu Gly Leu Val Trp Leu Phe Ala
            965             970             975
```

45

```
Ser Ile Leu Met Leu Gly Val Thr Phe Ile Ser Gln Ala Leu Ile Asn
        980                     985                 990

Phe Val Cys Ser Phe Gly Phe Leu Pro Val Val Lys Leu Leu Leu Gly
        995                     1000                1005

Glu Arg Asn Lys Val Tyr Val Ala Trp Lys Glu Leu Ser Asp Ile Ser
    1010                    1015                1020

Tyr Ser Tyr Leu Asn Ile Tyr Tyr Val Cys Val Gly Ser Val Cys Leu
1025                1030                1035                1040

Ser Lys Ile Ala Lys Asp Ile Leu His Phe Thr Glu Gly Gln Asn Thr
        1045                    1050                1055

Leu Asp Glu His Ala Val Asp Glu Asn Glu Val Glu Glu Val Glu His
        1060                    1065                1070

Asp Ile Pro Glu Arg Asp Ile Asn Asn Ala Pro Val Asn Asn Ile Asn
        1075                    1080                1085

Asn Val Glu Glu Gly Gln Gly Ile Phe Met Ala Ile Phe Asn Ser Ile
        1090                    1095                1100

Phe Asp Ser Met Leu Val Lys Tyr Asn Leu Met Val Phe Ile Ala Ile
1105                1110                1115                1120

Met Ile Ala Val Ile Arg Thr Met Val Ser Trp Val Val Leu Thr Asp
        1125                    1130                1135

Gly Ile Leu Ala Cys Tyr Asn Tyr Leu Thr Ile Arg Val Phe Gly Asn
        1140                    1145                1150

Ser Ser Tyr Thr Ile Gly Asn Ser Lys Trp Phe Lys Tyr Asp Glu Ser
        1155                    1160                1165

Leu Leu Phe Val Val Trp Ile Ile Ser Ser Met Val Asn Phe Gly Thr
    1170                    1175                1180

Gly Tyr Lys Ser Leu Lys Leu Phe Phe Arg Asn Arg Asn Thr Ser Lys
1185                1190                1195                1200

Leu Asn Phe Leu Lys Thr Met Ala Leu Glu Leu Phe Lys Gln Gly Phe
        1205                    1210                1215

Leu His Met Val Ile Tyr Val Leu Pro Ile Ile Ile Leu Ser Leu Val
        1220                    1225                1230

Phe Leu Arg Asp Val Ser Thr Lys Gln Ile Ile Asp Ile Ser His Gly
        1235                    1240                1245

Ser Arg Ser Phe Thr Leu Ser Leu Asn Glu Ser Phe Pro Thr Trp Thr
    1250                    1255                1260

Arg Met Gln Asp Ile Tyr Phe Gly Leu Leu Ile Ala Leu Glu Ser Phe
1265                1270                1275                1280

Thr Phe Phe Phe Gln Ala Thr Val Leu Phe Ile Gln Trp Phe Lys Ser
        1285                    1290                1295

Thr Val Gln Asn Val Lys Asp Glu Val Tyr Thr Lys Gly Arg Ala Leu
        1300                    1305                1310
```

46

```
Glu Asn Leu Pro Asp Glu Ser
        1315
```

```
<210>    17
<211>    278
<212>    PRT
<213>    Artificial Sequence

<220>
<223>    E3 MARCH5


<400>    17
Met Pro Asp Gln Ala Leu Gln Gln Met Leu Asp Arg Ser Cys Trp Val
  1               5                  10                  15

Cys Phe Ala Thr Asp Glu Asp Asp Arg Thr Ala Glu Trp Val Arg Pro
            20                  25                  30

Cys Arg Cys Arg Gly Ser Thr Lys Trp Val His Gln Ala Cys Leu Gln
        35                  40                  45

Arg Trp Val Asp Glu Lys Gln Arg Gly Asn Ser Thr Ala Arg Val Ala
        50                  55                  60

Cys Pro Gln Cys Asn Ala Glu Tyr Leu Ile Val Phe Pro Lys Leu Gly
65                  70                  75                  80

Pro Val Val Tyr Val Leu Asp Leu Ala Asp Arg Leu Ile Ser Lys Ala
                85                  90                  95

Cys Pro Phe Ala Ala Ala Gly Ile Met Val Gly Ser Ile Tyr Trp Thr
            100                 105                 110

Ala Val Thr Tyr Gly Ala Val Thr Val Met Gln Val Val Gly His Lys
            115                 120                 125

Glu Gly Leu Asp Val Met Glu Arg Ala Asp Pro Leu Phe Leu Leu Ile
    130                 135                 140

Gly Leu Pro Thr Ile Pro Val Met Leu Ile Leu Gly Lys Met Ile Arg
145                 150                 155                 160

Trp Glu Asp Tyr Val Leu Arg Leu Trp Arg Lys Tyr Ser Asn Lys Leu
                165                 170                 175

Gln Ile Leu Asn Ser Ile Phe Pro Gly Ile Gly Cys Pro Val Pro Arg
            180                 185                 190

Ile Pro Ala Glu Ala Asn Pro Leu Ala Asp His Val Ser Ala Thr Arg
            195                 200                 205

Ile Leu Cys Gly Ala Leu Val Phe Pro Thr Ile Ala Thr Ile Val Gly
    210                 215                 220

Lys Leu Met Phe Ser Ser Val Asn Ser Asn Leu Gln Arg Thr Ile Leu
225                 230                 235                 240

Gly Gly Ile Ala Phe Val Ala Ile Lys Gly Ala Phe Lys Val Tyr Phe
                245                 250                 255

Lys Gln Gln Gln Tyr Leu Arg Gln Ala His Arg Lys Ile Leu Asn Tyr
```

```
                        260                   265                   270

        Pro Glu Gln Glu Glu Ala
                275


        <210>   18
        <211>   1116
        <212>   PRT
        <213>   Artificial Sequence

        <220>
        <223>   His-SUMO-hGH-Ub(D)


        <400>   18
        Met Glu Thr Gly Leu Tyr His Ile Ser His Ile Ser His Ile Ser His
          1               5                  10                  15

        Ile Ser His Ile Ser His Ile Ser Ser Glu Arg Ala Ser Pro Gly Leu
                    20                  25                  30

        Asn Gly Leu Ala Ala Leu Ala Leu Tyr Ser Pro Arg Ala Ser Glu Arg
                35                  40                  45

        Thr His Arg Gly Leu Ala Ala Ser Pro Leu Glu Ala Gly Leu Tyr Ala
                50                  55                  60

        Ser Pro Leu Tyr Ser Leu Tyr Ser Gly Leu Ala Gly Leu Tyr Gly Leu
         65                  70                  75                  80

        Ala Thr Tyr Arg Ile Leu Glu Leu Tyr Ser Leu Glu Ala Leu Tyr Ser
                    85                  90                  95

        Val Ala Leu Ile Leu Glu Gly Leu Tyr Gly Leu Asn Ala Ser Pro Ser
                    100                 105                 110

        Glu Arg Ser Glu Arg Gly Leu Ala Ile Leu Glu His Ile Ser Pro His
                    115                 120                 125

        Glu Leu Tyr Ser Val Ala Leu Leu Tyr Ser Met Glu Thr Thr His Arg
                130                 135                 140

        Thr His Arg His Ile Ser Leu Glu Ala Leu Tyr Ser Leu Tyr Ser Leu
        145                 150                 155                 160

        Glu Ala Leu Tyr Ser Gly Leu Ala Ser Glu Arg Thr Tyr Arg Cys Tyr
                    165                 170                 175

        Ser Gly Leu Asn Ala Arg Gly Gly Leu Asn Gly Leu Tyr Val Ala Leu
                    180                 185                 190

        Pro Arg Ala Met Glu Thr Ala Ser Asn Ser Glu Arg Leu Glu Ala Ala
                    195                 200                 205

        Arg Gly Pro His Glu Leu Glu Ala Pro His Glu Gly Leu Ala Gly Leu
                    210                 215                 220

        Tyr Gly Leu Asn Ala Arg Gly Ile Leu Glu Ala Leu Ala Ala Ser Pro
        225                 230                 235                 240

        Ala Ser Asn His Ile Ser Thr His Arg Pro Arg Ala Leu Tyr Ser Gly
                    245                 250                 255
```

48

```
Leu Ala Leu Glu Ala Gly Leu Tyr Met Glu Thr Gly Leu Ala Gly Leu
        260                 265                 270

Ala Gly Leu Ala Ala Ser Pro Val Ala Leu Ile Leu Glu Gly Leu Ala
        275                 280                 285

Val Ala Leu Thr Tyr Arg Gly Leu Asn Gly Leu Ala Gly Leu Asn Thr
290                 295                 300

His Arg Gly Leu Tyr Gly Leu Tyr Pro His Glu Pro Arg Ala Thr His
305                 310                 315                 320

Arg Ile Leu Glu Pro Arg Ala Leu Glu Ala Ser Glu Arg Ala Arg Gly
            325                 330                 335

Leu Glu Ala Pro His Glu Ala Ser Pro Ala Ser Asn Ala Leu Ala Met
        340                 345                 350

Glu Thr Leu Glu Ala Ala Arg Gly Ala Leu Ala His Ile Ser Ala Arg
        355                 360                 365

Gly Leu Glu Ala His Ile Ser Gly Leu Asn Leu Glu Ala Ala Leu Ala
370                 375                 380

Pro His Glu Ala Ser Pro Thr His Arg Thr Tyr Arg Gly Leu Asn Gly
385                 390                 395                 400

Leu Ala Pro His Glu Gly Leu Ala Gly Leu Ala Ala Leu Ala Thr Tyr
            405                 410                 415

Arg Ile Leu Glu Pro Arg Ala Leu Tyr Ser Gly Leu Ala Gly Leu Asn
        420                 425                 430

Leu Tyr Ser Thr Tyr Arg Ser Glu Arg Pro His Glu Leu Glu Ala Gly
        435                 440                 445

Leu Asn Ala Ser Asn Pro Arg Ala Gly Leu Asn Thr His Arg Ser Glu
        450                 455                 460

Arg Leu Glu Ala Cys Tyr Ser Pro His Glu Ser Glu Arg Gly Leu Ala
465                 470                 475                 480

Ser Glu Arg Ile Leu Glu Pro Arg Ala Thr His Arg Pro Arg Ala Ser
            485                 490                 495

Glu Arg Ala Ser Asn Ala Arg Gly Gly Leu Ala Gly Leu Ala Thr His
        500                 505                 510

Arg Gly Leu Asn Gly Leu Asn Leu Tyr Ser Ser Glu Arg Ala Ser Asn
        515                 520                 525

Leu Glu Ala Gly Leu Ala Leu Glu Ala Leu Glu Ala Ala Arg Gly Ile
        530                 535                 540

Leu Glu Ser Glu Arg Leu Glu Ala Leu Glu Ala Leu Glu Ala Ile Leu
545                 550                 555                 560

Glu Gly Leu Asn Ser Glu Arg Thr Arg Pro Leu Glu Ala Gly Leu Ala
        565                 570                 575

Pro Arg Ala Val Ala Leu Gly Leu Asn Pro His Glu Leu Glu Ala Ala
        580                 585                 590
```

```
Arg Gly Ser Glu Arg Val Ala Leu Pro His Glu Ala Leu Ala Ala Ser
        595             600             605

Asn Ser Glu Arg Leu Glu Ala Val Ala Leu Thr Tyr Arg Gly Leu Tyr
    610             615             620

Ala Leu Ala Ser Glu Arg Ala Ser Pro Ser Glu Arg Ala Ser Asn Val
625             630             635             640

Ala Leu Thr Tyr Arg Ala Ser Pro Leu Glu Ala Leu Glu Ala Leu Tyr
            645             650             655

Ser Ala Ser Pro Leu Glu Ala Gly Leu Ala Gly Leu Ala Gly Leu Tyr
            660             665             670

Ile Leu Glu Gly Leu Asn Thr His Arg Leu Glu Ala Met Glu Thr Gly
        675             680             685

Leu Tyr Ala Arg Gly Leu Glu Ala Gly Leu Ala Ala Ser Pro Gly Leu
    690             695             700

Tyr Ser Glu Arg Pro Arg Ala Ala Arg Gly Thr His Arg Gly Leu Tyr
705             710             715             720

Gly Leu Asn Ile Leu Glu Pro His Glu Leu Tyr Ser Gly Leu Asn Thr
            725             730             735

His Arg Thr Tyr Arg Ser Glu Arg Leu Tyr Ser Pro His Glu Ala Ser
        740             745             750

Pro Thr His Arg Ala Ser Asn Ser Glu Arg His Ile Ser Ala Ser Asn
        755             760             765

Ala Ser Pro Ala Ser Pro Ala Leu Ala Leu Glu Ala Leu Glu Ala Leu
    770             775             780

Tyr Ser Ala Ser Asn Thr Tyr Arg Gly Leu Tyr Leu Glu Ala Leu Glu
785             790             795             800

Ala Thr Tyr Arg Cys Tyr Ser Pro His Glu Ala Arg Gly Leu Tyr Ser
            805             810             815

Ala Ser Pro Met Glu Thr Ala Ser Pro Leu Tyr Ser Val Ala Leu Gly
            820             825             830

Leu Ala Thr His Arg Pro His Glu Leu Glu Ala Ala Arg Gly Ile Leu
        835             840             845

Glu Val Ala Leu Gly Leu Asn Cys Tyr Ser Ala Arg Gly Ser Glu Arg
    850             855             860

Val Ala Leu Gly Leu Ala Gly Leu Tyr Ser Glu Arg Cys Tyr Ser Gly
865             870             875             880

Leu Tyr Pro His Glu Met Glu Thr Gly Leu Asn Ile Leu Glu Pro His
            885             890             895

Glu Val Ala Leu Ala Arg Gly Thr His Arg Leu Glu Ala Thr His Arg
        900             905             910

Gly Leu Tyr Ala Arg Gly Thr His Arg Ile Leu Glu Thr His Arg Leu
    915             920             925
```

```
Glu Ala Gly Leu Ala Val Ala Leu Gly Leu Ala Pro Arg Ala Ser Glu
    930             935             940

Arg Ala Ser Pro Thr His Arg Ile Leu Glu Gly Leu Ala Ala Ser Asn
945             950             955             960

Val Ala Leu Ala Arg Gly Ala Leu Ala Ala Arg Gly Ile Leu Glu Gly
            965             970             975

Leu Asn Ala Ser Pro Ala Arg Gly Gly Leu Ala Gly Leu Tyr Ile Leu
            980             985             990

Glu Pro Arg Ala Pro Arg Ala Ala Ser Pro Gly Leu Asn Gly Leu Asn
    995             1000            1005

Ala Arg Gly Leu Glu Ala Ile Leu Glu Pro His Glu Ala Leu Ala Gly
    1010            1015            1020

Leu Tyr Ala Arg Gly Gly Leu Asn Leu Glu Ala Gly Leu Ala Ala Ser
1025            1030            1035            1040

Pro Gly Leu Tyr Ala Arg Gly Thr His Arg Leu Glu Ala Ser Glu Arg
            1045            1050            1055

Ala Ser Pro Thr Tyr Arg Ala Ser Asn Ile Leu Glu Gly Leu Asn Ala
            1060            1065            1070

Arg Gly Gly Leu Ala Ser Glu Arg Thr His Arg Leu Glu Ala His Ile
        1075            1080            1085

Ser Leu Glu Ala Val Ala Leu Leu Glu Ala Ala Arg Gly Leu Glu Ala
    1090            1095            1100

Ala Arg Gly Gly Leu Tyr Gly Leu Tyr Ala Ser Pro
1105            1110            1115
```

```
<210>    19
<211>    1116
<212>    PRT
<213>    Artificial Sequence

<220>
<223>    His-SUMO- hGH-Ub(A)


<400>    19
Met Glu Thr Gly Leu Tyr His Ile Ser His Ile Ser His Ile Ser His
1             5               10              15

Ile Ser His Ile Ser His Ile Ser Ser Glu Arg Ala Ser Pro Gly Leu
            20              25              30

Asn Gly Leu Ala Ala Leu Ala Leu Tyr Ser Pro Arg Ala Ser Glu Arg
        35              40              45

Thr His Arg Gly Leu Ala Ala Ser Pro Leu Glu Ala Gly Leu Tyr Ala
    50              55              60

Ser Pro Leu Tyr Ser Leu Tyr Ser Gly Leu Ala Gly Leu Tyr Gly Leu
65              70              75              80
```

```
Ala Thr Tyr Arg Ile Leu Glu Leu Tyr Ser Leu Glu Ala Leu Tyr Ser
                85                  90              95

Val Ala Leu Ile Leu Glu Gly Leu Tyr Gly Leu Asn Ala Ser Pro Ser
            100              105              110

Glu Arg Ser Glu Arg Gly Leu Ala Ile Leu Glu His Ile Ser Pro His
        115              120              125

Glu Leu Tyr Ser Val Ala Leu Leu Tyr Ser Met Glu Thr Thr His Arg
    130              135              140

Thr His Arg His Ile Ser Leu Glu Ala Leu Tyr Ser Leu Tyr Ser Leu
145              150              155              160

Glu Ala Leu Tyr Ser Gly Leu Ala Ser Glu Arg Thr Tyr Arg Cys Tyr
            165              170              175

Ser Gly Leu Asn Ala Arg Gly Gly Leu Asn Gly Leu Tyr Val Ala Leu
            180              185              190

Pro Arg Ala Met Glu Thr Ala Ser Asn Ser Glu Arg Leu Glu Ala Ala
        195              200              205

Arg Gly Pro His Glu Leu Glu Ala Pro His Glu Gly Leu Ala Gly Leu
    210              215              220

Tyr Gly Leu Asn Ala Arg Gly Ile Leu Glu Ala Leu Ala Ala Ser Pro
225              230              235              240

Ala Ser Asn His Ile Ser Thr His Arg Pro Arg Ala Leu Tyr Ser Gly
            245              250              255

Leu Ala Leu Glu Ala Gly Leu Tyr Met Glu Thr Gly Leu Ala Gly Leu
            260              265              270

Ala Gly Leu Ala Ala Ser Pro Val Ala Leu Ile Leu Glu Gly Leu Ala
        275              280              285

Val Ala Leu Thr Tyr Arg Gly Leu Asn Gly Leu Ala Gly Leu Asn Thr
    290              295              300

His Arg Gly Leu Tyr Gly Leu Tyr Pro His Glu Pro Arg Ala Thr His
305              310              315              320

Arg Ile Leu Glu Pro Arg Ala Leu Glu Ala Ser Glu Arg Ala Arg Gly
            325              330              335

Leu Glu Ala Pro His Glu Ala Ser Pro Ala Ser Asn Ala Leu Ala Met
        340              345              350

Glu Thr Leu Glu Ala Ala Arg Gly Ala Leu Ala His Ile Ser Ala Arg
    355              360              365

Gly Leu Glu Ala His Ile Ser Gly Leu Asn Leu Glu Ala Ala Leu Ala
    370              375              380

Pro His Glu Ala Ser Pro Thr His Arg Thr Tyr Arg Gly Leu Asn Gly
385              390              395              400

Leu Ala Pro His Glu Gly Leu Ala Gly Leu Ala Ala Leu Ala Thr Tyr
            405              410              415
```

52

```
Arg Ile Leu Glu Pro Arg Ala Leu Tyr Ser Gly Leu Ala Gly Leu Asn
        420                 425                 430

Leu Tyr Ser Thr Tyr Arg Ser Glu Arg Pro His Glu Leu Glu Ala Gly
        435                 440                 445

Leu Asn Ala Ser Asn Pro Arg Ala Gly Leu Asn Thr His Arg Ser Glu
        450                 455                 460

Arg Leu Glu Ala Cys Tyr Ser Pro His Glu Ser Glu Arg Gly Leu Ala
465                 470                 475                 480

Ser Glu Arg Ile Leu Glu Pro Arg Ala Thr His Arg Pro Arg Ala Ser
            485                 490                 495

Glu Arg Ala Ser Asn Ala Arg Gly Gly Leu Ala Gly Leu Ala Thr His
        500                 505                 510

Arg Gly Leu Asn Gly Leu Asn Leu Tyr Ser Ser Glu Arg Ala Ser Asn
        515                 520                 525

Leu Glu Ala Gly Leu Ala Leu Glu Ala Leu Glu Ala Ala Arg Gly Ile
    530                 535                 540

Leu Glu Ser Glu Arg Leu Glu Ala Leu Glu Ala Leu Glu Ala Ile Leu
545                 550                 555                 560

Glu Gly Leu Asn Ser Glu Arg Thr Arg Pro Leu Glu Ala Gly Leu Ala
            565                 570                 575

Pro Arg Ala Val Ala Leu Gly Leu Asn Pro His Glu Leu Glu Ala Ala
            580                 585                 590

Arg Gly Ser Glu Arg Val Ala Leu Pro His Glu Ala Leu Ala Ala Ser
        595                 600                 605

Asn Ser Glu Arg Leu Glu Ala Val Ala Leu Thr Tyr Arg Gly Leu Tyr
    610                 615                 620

Ala Leu Ala Ser Glu Arg Ala Ser Pro Ser Glu Arg Ala Ser Asn Val
625                 630                 635                 640

Ala Leu Thr Tyr Arg Ala Ser Pro Leu Glu Ala Leu Glu Ala Leu Tyr
            645                 650                 655

Ser Ala Ser Pro Leu Glu Ala Gly Leu Ala Gly Leu Ala Gly Leu Tyr
        660                 665                 670

Ile Leu Glu Gly Leu Asn Thr His Arg Leu Glu Ala Met Glu Thr Gly
        675                 680                 685

Leu Tyr Ala Arg Gly Leu Glu Ala Gly Leu Ala Ala Ser Pro Gly Leu
    690                 695                 700

Tyr Ser Glu Arg Pro Arg Ala Ala Arg Gly Thr His Arg Gly Leu Tyr
705                 710                 715                 720

Gly Leu Asn Ile Leu Glu Pro His Glu Leu Tyr Ser Gly Leu Asn Thr
            725                 730                 735

His Arg Thr Tyr Arg Ser Glu Arg Leu Tyr Ser Pro His Glu Ala Ser
        740                 745                 750
```

53

```
Pro Thr His Arg Ala Ser Asn Ser Glu Arg His Ile Ser Ala Ser Asn
        755                 760             765

Ala Ser Pro Ala Ser Pro Ala Leu Ala Leu Glu Ala Leu Glu Ala Leu
        770             775             780

Tyr Ser Ala Ser Asn Thr Tyr Arg Gly Leu Tyr Leu Glu Ala Leu Glu
785                 790             795                 800

Ala Thr Tyr Arg Cys Tyr Ser Pro His Glu Ala Arg Gly Leu Tyr Ser
            805             810                 815

Ala Ser Pro Met Glu Thr Ala Ser Pro Leu Tyr Ser Val Ala Leu Gly
            820             825             830

Leu Ala Thr His Arg Pro His Glu Leu Glu Ala Ala Arg Gly Ile Leu
            835             840             845

Glu Val Ala Leu Gly Leu Asn Cys Tyr Ser Ala Arg Gly Ser Glu Arg
        850             855             860

Val Ala Leu Gly Leu Ala Gly Leu Tyr Ser Glu Arg Cys Tyr Ser Gly
865             870             875                 880

Leu Tyr Pro His Glu Met Glu Thr Gly Leu Asn Ile Leu Glu Pro His
            885             890                 895

Glu Val Ala Leu Ala Arg Gly Thr His Arg Leu Glu Ala Thr His Arg
        900             905             910

Gly Leu Tyr Ala Arg Gly Thr His Arg Ile Leu Glu Thr His Arg Leu
        915             920             925

Glu Ala Gly Leu Ala Val Ala Leu Gly Leu Ala Pro Arg Ala Ser Glu
    930             935             940

Arg Ala Ser Pro Thr His Arg Ile Leu Glu Gly Leu Ala Ala Ser Asn
945             950             955             960

Val Ala Leu Ala Arg Gly Ala Leu Ala Ala Arg Gly Ile Leu Glu Gly
            965             970             975

Leu Asn Ala Ser Pro Ala Arg Gly Gly Leu Ala Gly Leu Tyr Ile Leu
            980             985             990

Glu Pro Arg Ala Pro Arg Ala Ala Ser Pro Gly Leu Asn Gly Leu Asn
        995             1000            1005

Ala Arg Gly Leu Glu Ala Ile Leu Glu Pro His Glu Ala Leu Ala Gly
    1010            1015            1020

Leu Tyr Ala Arg Gly Gly Leu Asn Leu Glu Ala Gly Leu Ala Ala Ser
1025            1030            1035            1040

Pro Gly Leu Tyr Ala Arg Gly Thr His Arg Leu Glu Ala Ser Glu Arg
            1045            1050            1055

Ala Ser Pro Thr Tyr Arg Ala Ser Asn Ile Leu Glu Gly Leu Asn Leu
        1060            1065            1070

Tyr Ser Gly Leu Ala Ser Glu Arg Thr His Arg Leu Glu Ala His Ile
        1075            1080            1085
```

54

```
Ser Leu Glu Ala Val Ala Leu Leu Glu Ala Ala Arg Gly Pro Arg Ala
    1090            1095            1100

Ala Arg Gly Gly Leu Tyr Gly Leu Tyr Ala Ser Pro
1105            1110            1115


<210>    20
<211>    804
<212>    PRT
<213>    Artificial Sequence

<220>
<223>    Ub(A)-hGH


<400>    20
Met Glu Thr Gly Leu Asn Ile Leu Glu Pro His Glu Val Ala Leu Ala
    1            5            10            15

Arg Gly Thr His Arg Leu Glu Ala Thr His Arg Gly Leu Tyr Ala Arg
            20            25            30

Gly Thr His Arg Ile Leu Glu Thr His Arg Leu Glu Ala Gly Leu Ala
        35            40            45

Val Ala Leu Gly Leu Ala Pro Arg Ala Ser Glu Arg Ala Ser Pro Thr
    50            55            60

His Arg Ile Leu Glu Gly Leu Ala Ala Ser Asn Val Ala Leu Ala Arg
65            70            75            80

Gly Ala Leu Ala Ala Arg Gly Ile Leu Glu Gly Leu Asn Ala Ser Pro
        85            90            95

Ala Arg Gly Gly Leu Ala Gly Leu Tyr Ile Leu Glu Pro Arg Ala Pro
        100            105            110

Arg Ala Ala Ser Pro Gly Leu Asn Gly Leu Asn Ala Arg Gly Leu Glu
    115            120            125

Ala Ile Leu Glu Pro His Glu Ala Leu Ala Gly Leu Tyr Ala Arg Gly
    130            135            140

Gly Leu Asn Leu Glu Ala Gly Leu Ala Ala Ser Pro Gly Leu Tyr Ala
145            150            155            160

Arg Gly Thr His Arg Leu Glu Ala Ser Glu Arg Ala Ser Pro Thr Tyr
            165            170            175

Arg Ala Ser Asn Ile Leu Glu Gly Leu Asn Leu Tyr Ser Gly Leu Ala
        180            185            190

Ser Glu Arg Thr His Arg Leu Glu Ala His Ile Ser Leu Glu Ala Val
    195            200            205

Ala Leu Leu Glu Ala Ala Arg Gly Pro Arg Ala Ala Arg Gly Gly Leu
    210            215            220

Tyr Gly Leu Tyr Ala Ser Pro Pro His Glu Pro Arg Ala Thr His Arg
225            230            235            240

Ile Leu Glu Pro Arg Ala Leu Glu Ala Ser Glu Arg Ala Arg Gly Leu
```

```
                    245                     250                     255

Glu Ala Pro His Glu Ala Ser Pro Ala Ser Asn Ala Leu Ala Met Glu
            260                 265                 270

Thr Leu Glu Ala Ala Arg Gly Ala Leu Ala His Ile Ser Ala Arg Gly
            275                 280                 285

Leu Glu Ala His Ile Ser Gly Leu Asn Leu Glu Ala Ala Leu Ala Pro
            290                 295                 300

His Glu Ala Ser Pro Thr His Arg Thr Tyr Arg Gly Leu Asn Gly Leu
305                 310                 315                 320

Ala Pro His Glu Gly Leu Ala Gly Leu Ala Ala Leu Ala Thr Tyr Arg
                325                 330                 335

Ile Leu Glu Pro Arg Ala Leu Tyr Ser Gly Leu Ala Gly Leu Asn Leu
            340                 345                 350

Tyr Ser Thr Tyr Arg Ser Glu Arg Pro His Glu Leu Glu Ala Gly Leu
            355                 360                 365

Asn Ala Ser Asn Pro Arg Ala Gly Leu Asn Thr His Arg Ser Glu Arg
    370                 375                 380

Leu Glu Ala Cys Tyr Ser Pro His Glu Ser Glu Arg Gly Leu Ala Ser
385                 390                 395                 400

Glu Arg Ile Leu Glu Pro Arg Ala Thr His Arg Pro Arg Ala Ser Glu
            405                 410                 415

Arg Ala Ser Asn Ala Arg Gly Gly Leu Ala Gly Leu Ala Thr His Arg
            420                 425                 430

Gly Leu Asn Gly Leu Asn Leu Tyr Ser Ser Glu Arg Ala Ser Asn Leu
            435                 440                 445

Glu Ala Gly Leu Ala Leu Glu Ala Leu Glu Ala Ala Arg Gly Ile Leu
    450                 455                 460

Glu Ser Glu Arg Leu Glu Ala Leu Glu Ala Leu Glu Ala Ile Leu Glu
465                 470                 475                 480

Gly Leu Asn Ser Glu Arg Thr Arg Pro Leu Glu Ala Gly Leu Ala Pro
            485                 490                 495

Arg Ala Val Ala Leu Gly Leu Asn Pro His Glu Leu Glu Ala Ala Arg
            500                 505                 510

Gly Ser Glu Arg Val Ala Leu Pro His Glu Ala Leu Ala Ala Ser Asn
            515                 520                 525

Ser Glu Arg Leu Glu Ala Val Ala Leu Thr Tyr Arg Gly Leu Tyr Ala
    530                 535                 540

Leu Ala Ser Glu Arg Ala Ser Pro Ser Glu Arg Ala Ser Asn Val Ala
545                 550                 555                 560

Leu Thr Tyr Arg Ala Ser Pro Leu Glu Ala Leu Glu Ala Leu Tyr Ser
            565                 570                 575

Ala Ser Pro Leu Glu Ala Gly Leu Ala Gly Leu Ala Gly Leu Tyr Ile
```

56

580 585 590

Leu Glu Gly Leu Asn Thr His Arg Leu Glu Ala Met Glu Thr Gly Leu
595 600 605

Tyr Ala Arg Gly Leu Glu Ala Gly Leu Ala Ala Ser Pro Gly Leu Tyr
610 615 620

Ser Glu Arg Pro Arg Ala Ala Arg Gly Thr His Arg Gly Leu Tyr Gly
625 630 635 640

Leu Asn Ile Leu Glu Pro His Glu Leu Tyr Ser Gly Leu Asn Thr His
645 650 655

Arg Thr Tyr Arg Ser Glu Arg Leu Tyr Ser Pro His Glu Ala Ser Pro
660 665 670

Thr His Arg Ala Ser Asn Ser Glu Arg His Ile Ser Ala Ser Asn Ala
675 680 685

Ser Pro Ala Ser Pro Ala Leu Ala Leu Glu Ala Leu Glu Ala Leu Tyr
690 695 700

Ser Ala Ser Asn Thr Tyr Arg Gly Leu Tyr Leu Glu Ala Leu Glu Ala
705 710 715 720

Thr Tyr Arg Cys Tyr Ser Pro His Glu Ala Arg Gly Leu Tyr Ser Ala
725 730 735

Ser Pro Met Glu Thr Ala Ser Pro Leu Tyr Ser Val Ala Leu Gly Leu
740 745 750

Ala Thr His Arg Pro His Glu Leu Glu Ala Ala Arg Gly Ile Leu Glu
755 760 765

Val Ala Leu Gly Leu Asn Cys Tyr Ser Ala Arg Gly Ser Glu Arg Val
770 775 780

Ala Leu Gly Leu Ala Gly Leu Tyr Ser Glu Arg Cys Tyr Ser Gly Leu
785 790 795 800

Tyr Pro His Glu

<210> 21
<211> 1116
<212> PRT
<213> Artificial Sequence

<220>
<223> SUMO-Ub(A)-hGH

<400> 21
Met Glu Thr Gly Leu Tyr His Ile Ser His Ile Ser His Ile Ser His
1 5 10 15

Ile Ser His Ile Ser His Ile Ser Ser Glu Arg Ala Ser Pro Gly Leu
20 25 30

Asn Gly Leu Ala Ala Leu Ala Leu Tyr Ser Pro Arg Ala Ser Glu Arg
35 40 45

```
        Thr His Arg Gly Leu Ala Ala Ser Pro Leu Glu Ala Gly Leu Tyr Ala
            50                      55                  60

        Ser Pro Leu Tyr Ser Leu Tyr Ser Gly Leu Ala Gly Leu Tyr Gly Leu
            65                      70                  75                  80

        Ala Thr Tyr Arg Ile Leu Glu Leu Tyr Ser Leu Glu Ala Leu Tyr Ser
                    85                      90                      95

        Val Ala Leu Ile Leu Glu Gly Leu Tyr Gly Leu Asn Ala Ser Pro Ser
                    100                     105                 110

        Glu Arg Ser Glu Arg Gly Leu Ala Ile Leu Glu His Ile Ser Pro His
                    115                     120                 125

        Glu Leu Tyr Ser Val Ala Leu Leu Tyr Ser Met Glu Thr Thr His Arg
            130                     135                 140

        Thr His Arg His Ile Ser Leu Glu Ala Leu Tyr Ser Leu Tyr Ser Leu
        145                     150                 155                 160

        Glu Ala Leu Tyr Ser Gly Leu Ala Ser Glu Arg Thr Tyr Arg Cys Tyr
                        165                 170                 175

        Ser Gly Leu Asn Ala Arg Gly Gly Leu Asn Gly Leu Tyr Val Ala Leu
                    180                     185                 190

        Pro Arg Ala Met Glu Thr Ala Ser Asn Ser Glu Arg Leu Glu Ala Ala
                195                     200                 205

        Arg Gly Pro His Glu Leu Glu Ala Pro His Glu Gly Leu Ala Gly Leu
            210                     215                 220

        Tyr Gly Leu Asn Ala Arg Gly Ile Leu Glu Ala Leu Ala Ala Ser Pro
        225                     230                 235                 240

        Ala Ser Asn His Ile Ser Thr His Arg Pro Arg Ala Leu Tyr Ser Gly
                        245                 250                 255

        Leu Ala Leu Glu Ala Gly Leu Tyr Met Glu Thr Gly Leu Ala Gly Leu
                    260                     265                 270

        Ala Gly Leu Ala Ala Ser Pro Val Ala Leu Ile Leu Glu Gly Leu Ala
                    275                     280                 285

        Val Ala Leu Thr Tyr Arg Gly Leu Asn Gly Leu Ala Gly Leu Asn Thr
            290                     295                 300

        His Arg Gly Leu Tyr Gly Leu Tyr Met Glu Thr Gly Leu Asn Ile Leu
        305                     310                 315                 320

        Glu Pro His Glu Val Ala Leu Ala Arg Gly Thr His Arg Leu Glu Ala
                        325                     330                 335

        Thr His Arg Gly Leu Tyr Ala Arg Gly Thr His Arg Ile Leu Glu Thr
                    340                     345                 350

        His Arg Leu Glu Ala Gly Leu Ala Val Ala Leu Gly Leu Ala Pro Arg
                    355                     360                 365

        Ala Ser Glu Arg Ala Ser Pro Thr His Arg Ile Leu Glu Gly Leu Ala
            370                     375                 380
```

58

```
Ala Ser Asn Val Ala Leu Ala Arg Gly Ala Leu Ala Ala Arg Gly Ile
385                 390                 395                 400

Leu Glu Gly Leu Asn Ala Ser Pro Ala Arg Gly Gly Leu Ala Gly Leu
                405             410             415

Tyr Ile Leu Glu Pro Arg Ala Pro Arg Ala Ala Ser Pro Gly Leu Asn
            420             425             430

Gly Leu Asn Ala Arg Gly Leu Glu Ala Ile Leu Glu Pro His Glu Ala
        435             440             445

Leu Ala Gly Leu Tyr Leu Tyr Ser Gly Leu Asn Leu Glu Ala Gly Leu
    450             455             460

Ala Ala Ser Pro Gly Leu Tyr Ala Arg Gly Thr His Arg Leu Glu Ala
465             470             475             480

Ser Glu Arg Ala Ser Pro Thr Tyr Arg Ala Ser Asn Ile Leu Glu Gly
            485             490             495

Leu Asn Ala Arg Gly Gly Leu Ala Ser Glu Arg Thr His Arg Leu Glu
        500             505             510

Ala His Ile Ser Leu Glu Ala Val Ala Leu Leu Glu Ala Ala Arg Gly
    515             520             525

Pro Arg Ala Ala Arg Gly Gly Leu Tyr Gly Leu Tyr Ala Ser Pro Pro
    530             535             540

His Glu Pro Arg Ala Thr His Arg Ile Leu Glu Pro Arg Ala Leu Glu
545             550             555             560

Ala Ser Glu Arg Ala Arg Gly Leu Glu Ala Pro His Glu Ala Ser Pro
            565             570             575

Ala Ser Asn Ala Leu Ala Met Glu Thr Leu Glu Ala Ala Arg Gly Ala
        580             585             590

Leu Ala His Ile Ser Ala Arg Gly Leu Glu Ala His Ile Ser Gly Leu
    595             600             605

Asn Leu Glu Ala Ala Leu Ala Pro His Glu Ala Ser Pro Thr His Arg
    610             615             620

Thr Tyr Arg Gly Leu Asn Gly Leu Ala Pro His Glu Gly Leu Ala Gly
625             630             635             640

Leu Ala Ala Leu Ala Thr Tyr Arg Ile Leu Glu Pro Arg Ala Leu Tyr
            645             650             655

Ser Gly Leu Ala Gly Leu Asn Leu Tyr Ser Thr Tyr Arg Ser Glu Arg
        660             665             670

Pro His Glu Leu Glu Ala Gly Leu Asn Ala Ser Asn Pro Arg Ala Gly
    675             680             685

Leu Asn Thr His Arg Ser Glu Arg Leu Glu Ala Cys Tyr Ser Pro His
    690             695             700

Glu Ser Glu Arg Gly Leu Ala Ser Glu Arg Ile Leu Glu Pro Arg Ala
705             710             715             720
```

59

```
Thr His Arg Pro Arg Ala Ser Glu Arg Ala Ser Asn Ala Arg Gly Gly
                725                 730                 735

Leu Ala Gly Leu Ala Thr His Arg Gly Leu Asn Gly Leu Asn Leu Tyr
            740                 745                 750

Ser Ser Glu Arg Ala Ser Asn Leu Glu Ala Gly Leu Ala Leu Glu Ala
        755                 760                 765

Leu Glu Ala Ala Arg Gly Ile Leu Glu Ser Glu Arg Leu Glu Ala Leu
    770                 775                 780

Glu Ala Leu Glu Ala Ile Leu Glu Gly Leu Asn Ser Glu Arg Thr Arg
785                 790                 795                 800

Pro Leu Glu Ala Gly Leu Ala Pro Arg Ala Val Ala Leu Gly Leu Asn
            805                 810                 815

Pro His Glu Leu Glu Ala Ala Arg Gly Ser Glu Arg Val Ala Leu Pro
        820                 825                 830

His Glu Ala Leu Ala Ala Ser Asn Ser Glu Arg Leu Glu Ala Val Ala
    835                 840                 845

Leu Thr Tyr Arg Gly Leu Tyr Ala Leu Ala Ser Glu Arg Ala Ser Pro
    850                 855                 860

Ser Glu Arg Ala Ser Asn Val Ala Leu Thr Tyr Arg Ala Ser Pro Leu
865                 870                 875                 880

Glu Ala Leu Glu Ala Leu Tyr Ser Ala Ser Pro Leu Glu Ala Gly Leu
            885                 890                 895

Ala Gly Leu Ala Gly Leu Tyr Ile Leu Glu Gly Leu Asn Thr His Arg
            900                 905                 910

Leu Glu Ala Met Glu Thr Gly Leu Tyr Ala Arg Gly Leu Glu Ala Gly
        915                 920                 925

Leu Ala Ala Ser Pro Gly Leu Tyr Ser Glu Arg Pro Arg Ala Ala Arg
    930                 935                 940

Gly Thr His Arg Gly Leu Tyr Gly Leu Asn Ile Leu Glu Pro His Glu
945                 950                 955                 960

Leu Tyr Ser Gly Leu Asn Thr His Arg Thr Tyr Arg Ser Glu Arg Leu
            965                 970                 975

Tyr Ser Pro His Glu Ala Ser Pro Thr His Arg Ala Ser Asn Ser Glu
        980                 985                 990

Arg His Ile Ser Ala Ser Asn Ala Ser Pro Ala Ser Pro Ala Leu Ala
        995                 1000                1005

Leu Glu Ala Leu Glu Ala Leu Tyr Ser Ala Ser Asn Thr Tyr Arg Gly
    1010                1015                1020

Leu Tyr Leu Glu Ala Leu Glu Ala Thr Tyr Arg Cys Tyr Ser Pro His
1025                1030                1035                1040

Glu Ala Arg Gly Leu Tyr Ser Ala Ser Pro Met Glu Thr Ala Ser Pro
                1045                1050                1055
```

60

Leu Tyr Ser Val Ala Leu Gly Leu Ala Thr His Arg Pro His Glu Leu
       1060             1065          1070

Glu Ala Ala Arg Gly Ile Leu Glu Val Ala Leu Gly Leu Asn Cys Tyr
      1075          1080          1085

Ser Ala Arg Gly Ser Glu Arg Val Ala Leu Gly Leu Ala Gly Leu Tyr
   1090          1095          1100

Ser Glu Arg Cys Tyr Ser Gly Leu Tyr Pro His Glu
1105          1110          1115


<210>	22
<211>	60
<212>	DNA
<213>	Artificial Sequence

<220>
<223>	Signal peptide (IgGk)


<400>	22
atggaaactg atactctgct gctgtgggtg ctgctgctgt gggtgcccgg ctcaactggt	60

                                                              60


<210>	23
<211>	231
<212>	DNA
<213>	Artificial Sequence

<220>
<223>	Ub (A)


<400>	23
atgcagatct tcgtgaggac cctgacagat cggaccatca cactggaggt ggagccaagc	60

gacaccatcg agaacgtgag ggccagaatc caggaccggg agggcatccc ccctgatcag	120

cagagactga tcttcgctgg ccgccagctg gaggacggaa ggaccctgag cgattacaat	180

atccagaaag agtctacact gcacctggtg ctgagaccgc gcgtcgtgga t	231


<210>	24
<211>	36
<212>	DNA
<213>	Artificial Sequence

<220>
<223>	Hinge (IgG1)


<400>	24
gagccaaaat cttgtgacaa aactcataca tgtccc	36


<210>	25
<211>	660

<212>      DNA
<213>      Artificial Sequence

<220>
<223>      Fc (IgG1)


<400>      25
ccatgtcccg cacctgaact gctgggcgga cctagcgtgt ttctgttccc acctaagcca          60

aaggacaccc tgatgatctc caggaccccc gaggtgacat gcgtggtggt ggacgtgagc          120

cacgaggacc ccgaggtgaa gttcaactgg tacgtggatg gcgtggaggt gcataatgcc          180

aagacaaagc caagggagga gcagtacaac agcacctatc gggtggtgtc tgtgctgaca          240

gtgctgcacc aggactggct gaacggcaag gagtataagt gcaaggtgtc taataaggcc          300

ctgcccgctc ctatcgagaa gaccatctcc aaggccaagg gccagccaag agagccccag          360

gtgtacacac tgccccctag ccgcgacgag ctgaccaaga accaggtgtc tctgacatgt          420

ctggtgaagg gcttctatcc ttctgatatc gctgtggagt gggagtccaa tggccagcca          480

gagaacaatt acaagaccac accacccgtg ctggactctg atggctcctt ctttctgtat          540

tccaagctga ccgtggataa gagcagatgg cagcagggca acgtgttctc ctgtagcgtg          600

atgcatgaag cactgcataa tcactatacc cagaagtcac tgtcactgag tcccggtaaa          660

660


<210>      26
<211>      20
<212>      PRT
<213>      Artificial Sequence

<220>
<223>      Signal peptide (IgGk)


<400>      26
Met Glu Thr Asp Thr Leu Leu Leu Trp Val Leu Leu Leu Trp Val Pro
1               5                   10                  15

Gly Ser Thr Gly
            20


<210>      27
<211>      77
<212>      PRT
<213>      Artificial Sequence

<220>
<223>      Ub(A)


<400>      27
Met Gln Ile Phe Val Arg Thr Leu Thr Asp Arg Thr Ile Thr Leu Glu
1               5                   10                  15

Val Glu Pro Ser Asp Thr Ile Glu Asn Val Arg Ala Arg Ile Gln Asp
20          25          30

Arg Glu Gly Ile Pro Pro Asp Gln Gln Arg Leu Ile Phe Ala Gly Arg
35          40          45

Gln Leu Glu Asp Gly Arg Thr Leu Ser Asp Tyr Asn Ile Gln Lys Glu
50          55          60

Ser Thr Leu His Leu Val Leu Arg Pro Arg Val Val Asp
65          70          75

<210>    28
<211>    12
<212>    PRT
<213>    Artificial Sequence

<220>
<223>    Hinge (IgG1)

<400>    28
Glu Pro Lys Ser Cys Asp Lys Thr His Thr Cys Pro
1          5          10

<210>    29
<211>    660
<212>    PRT
<213>    Artificial Sequence

<220>
<223>    Fc (IgG1)

<400>    29
Pro Arg Ala Cys Tyr Ser Pro Arg Ala Ala Leu Ala Pro Arg Ala Gly
1          5          10          15

Leu Ala Leu Glu Ala Leu Glu Ala Gly Leu Tyr Gly Leu Tyr Pro Arg
20          25          30

Ala Ser Glu Arg Val Ala Leu Pro His Glu Leu Glu Ala Pro His Glu
35          40          45

Pro Arg Ala Pro Arg Ala Leu Tyr Ser Pro Arg Ala Leu Tyr Ser Ala
50          55          60

Ser Pro Thr His Arg Leu Glu Ala Met Glu Thr Ile Leu Glu Ser Glu
65          70          75          80

Arg Ala Arg Gly Thr His Arg Pro Arg Ala Gly Leu Ala Val Ala Leu
85          90          95

Thr His Arg Cys Tyr Ser Val Ala Leu Val Ala Leu Val Ala Leu Ala
100          105          110

Ser Pro Val Ala Leu Ser Glu Arg His Ile Ser Gly Leu Ala Ala Ser
115          120          125

Pro Pro Arg Ala Gly Leu Ala Val Ala Leu Leu Tyr Ser Pro His Glu
130          135          140

```
Ala Ser Asn Thr Arg Pro Thr Tyr Arg Val Ala Leu Ala Ser Pro Gly
145             150             155             160

Leu Tyr Val Ala Leu Gly Leu Ala Val Ala Leu His Ile Ser Ala Ser
                165             170             175

Asn Ala Leu Ala Leu Tyr Ser Thr His Arg Leu Tyr Ser Pro Arg Ala
            180             185             190

Ala Arg Gly Gly Leu Ala Gly Leu Ala Gly Leu Asn Thr Tyr Arg Ala
            195             200             205

Ser Asn Ser Glu Arg Thr His Arg Thr Tyr Arg Ala Arg Gly Val Ala
            210             215             220

Leu Val Ala Leu Ser Glu Arg Val Ala Leu Leu Glu Ala Thr His Arg
225             230             235             240

Val Ala Leu Leu Glu Ala His Ile Ser Gly Leu Asn Ala Ser Pro Thr
            245             250             255

Arg Pro Leu Glu Ala Ala Ser Asn Gly Leu Tyr Leu Tyr Ser Gly Leu
            260             265             270

Ala Thr Tyr Arg Leu Tyr Ser Cys Tyr Ser Leu Tyr Ser Val Ala Leu
            275             280             285

Ser Glu Arg Ala Ser Asn Leu Tyr Ser Ala Leu Ala Leu Glu Ala Pro
            290             295             300

Arg Ala Ala Leu Ala Pro Arg Ala Ile Leu Glu Gly Leu Ala Leu Tyr
305             310             315             320

Ser Thr His Arg Ile Leu Glu Ser Glu Arg Leu Tyr Ser Ala Leu Ala
            325             330             335

Leu Tyr Ser Gly Leu Tyr Gly Leu Asn Pro Arg Ala Ala Arg Gly Gly
            340             345             350

Leu Ala Pro Arg Ala Gly Leu Asn Val Ala Leu Thr Tyr Arg Thr His
            355             360             365

Arg Leu Glu Ala Pro Arg Ala Pro Arg Ala Ser Glu Arg Ala Arg Gly
    370             375             380

Ala Ser Pro Gly Leu Ala Leu Glu Ala Thr His Arg Leu Tyr Ser Ala
385             390             395             400

Ser Asn Gly Leu Asn Val Ala Leu Ser Glu Arg Leu Glu Ala Thr His
            405             410             415

Arg Cys Tyr Ser Leu Glu Ala Val Ala Leu Leu Tyr Ser Gly Leu Tyr
            420             425             430

Pro His Glu Thr Tyr Arg Pro Arg Ala Ser Glu Arg Ala Ser Pro Ile
    435             440             445

Leu Glu Ala Leu Ala Val Ala Leu Gly Leu Ala Thr Arg Pro Gly Leu
    450             455             460

Ala Ser Glu Arg Ala Ser Asn Gly Leu Tyr Gly Leu Asn Pro Arg Ala
465             470             475             480
```

64

```
Gly Leu Ala Ala Ser Asn Ala Ser Asn Thr Tyr Arg Leu Tyr Ser Thr
            485             490             495

His Arg Thr His Arg Pro Arg Ala Pro Arg Ala Val Ala Leu Leu Glu
            500             505             510

Ala Ala Ser Pro Ser Glu Arg Ala Ser Pro Gly Leu Tyr Ser Glu Arg
        515             520             525

Pro His Glu Pro His Glu Leu Glu Ala Thr Tyr Arg Ser Glu Arg Leu
    530             535             540

Tyr Ser Leu Glu Ala Thr His Arg Val Ala Leu Ala Ser Pro Leu Tyr
545             550             555             560

Ser Ser Glu Arg Ala Arg Gly Thr Arg Pro Gly Leu Asn Gly Leu Asn
            565             570             575

Gly Leu Tyr Ala Ser Asn Val Ala Leu Pro His Glu Ser Glu Arg Cys
        580             585             590

Tyr Ser Ser Glu Arg Val Ala Leu Met Glu Thr His Ile Ser Gly Leu
        595             600             605

Ala Ala Leu Ala Leu Glu Ala His Ile Ser Ala Ser Asn His Ile Ser
    610             615             620

Thr Tyr Arg Thr His Arg Gly Leu Asn Leu Tyr Ser Ser Glu Arg Leu
625             630             635             640

Glu Ala Ser Glu Arg Leu Glu Ala Ser Glu Arg Pro Arg Ala Gly Leu
            645             650             655

Tyr Leu Tyr Ser
            660
```